(19) Europäisches Patentamt
European Patent Office
Office européen des brevets

(11) EP 1 207 391 A1

(12) **EUROPEAN PATENT APPLICATION**
published in accordance with Art. 158(3) EPC

(43) Date of publication:
**22.05.2002 Bulletin 2002/21**

(21) Application number: **00940910.3**

(22) Date of filing: **30.06.2000**

(51) Int Cl.[7]: **G01N 33/15**, G01N 33/566, G01N 33/50, A61K 47/48, A61K 38/20, A61K 38/21, A61K 31/7028, C07H 15/04

(86) International application number:
**PCT/JP00/04362**

(87) International publication number:
**WO 01/02851 (11.01.2001 Gazette 2001/02)**

(84) Designated Contracting States:
**AT BE CH CY DE DK ES FI FR GB GR IE IT LI LU MC NL PT SE**
Designated Extension States:
**AL LT LV MK RO SI**

(30) Priority: **30.06.1999 JP 18676199**

(71) Applicant: **Ajinomoto Co., Inc.**
**Tokyo 104-8315 (JP)**

(72) Inventors:
- **SATO, Haruya Ajinomoto Co., Inc.**
  **Kawasaki-shi, Kanagawa 210-8681 (JP)**
- **TAKAHARA, Yoshiyuki Ajinomoto Co., Inc.**
  **Kawasaki-shi, Kanagawa 210-8681 (JP)**
- **HAYASHI, Eiko Ajinomoto Co., Inc.**
  **Kawasaki-shi, Kanagawa 210-8681 (JP)**
- **TABATA, Tomoyuki Ajinomoto Co., Inc.**
  **Kawasaki-shi, Kanagawa 210-8681 (JP)**
- **SUZUKI, Manabu Ajinomoto Co., Inc.**
  **Kawasaki-shi, Kanagawa 210-8681 (JP)**
- **TANAKA, Yasuhiro Ajinomoto Co., Inc.**
  **Kawasaki-shi, Kanagawa 210-8681 (JP)**
- **YUGIYAMA, Yuichi**
  **Sodegaura-shi, Chiba 299-0232 (JP)**
- **KATO, Masao**
  **Kiyose-shi, Tokyo 204-0012 (JP)**

(74) Representative: **Silverman, Warren et al**
**Haseltine Lake & Co.**
**Imperial House,**
**15-19 Kingsway**
**London WC2B 6UD (GB)**

## (54) METHOD FOR SCREENING TARGETING DDS PREPARATION

(57) A method for screening an optimized ligand, which can avoid the internalization thereof in cell, with the guidance of a characteristic of the ligand of being capable of accumulating specifically in a target tissue by, after systemic administration, collecting the ligand around a specific receptor (ASGP-R, etc.) on the cell membrane due to the binding affinity to the receptor so as to minimize the internalization mediated by the receptor; and tissue-targeting DDS preparations which can be accumulated and retained around a receptor by binding an optimum ligand to a physiologically active protein via a covalent bond. Namely, a method for screening a ligand having a binding affinity to a protein such as a specific receptor or antigen present on the cell membrane of a target tissue or a substance modified with this ligand with the guidance of the ability to avoid the internalization mediated by the binding to the receptor, for example, the ratio of the constant of dissociation rate constant ($K_{off}$) to the constant of internalization rate constant ($K_{int}$) to the receptor (i.e., $K_{off}/K_{int}$) and the strength of the binding affinity; and medicinal preparations which can be accumulated and retained around a receptor, composed of the above-described ligand and a physiologically active protein or a low-molecular weight chemical bonded thereto.

Fig. 1

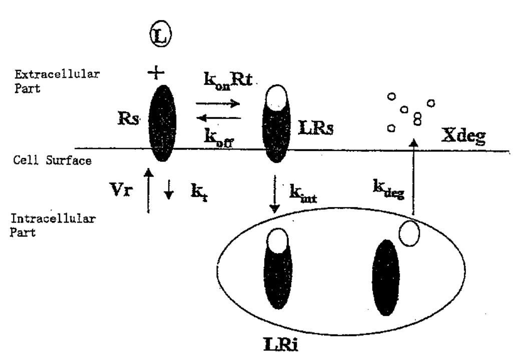


EP 1 207 391 A1

Printed by Jouve, 75001 PARIS (FR)

## Description

Technical Field

The present invention relates to a method for screening a ligand having properties which enable it to accumulate and retain medicinal preparations around a specific receptor on the cell membrane employing the guidance of the bond affinity for the receptor and the ability to avoid the internalization mediated by the receptor in the cell, and medicinal preparations, which can be accumulated and retained around a receptor, composed of the ligand and a physiologically active protein or a low-molecular weight drug thereto.

Further, the present invention provides targeting DDS preparations by the screening method of the present invention.

Background Art

In recent years, development of medicinal products composed of various physiologically active proteins have been attempted by producing these using recombinant gene technology, however it was quite difficult in practice.

Physiologically active proteins are expected to exhibit strong physiological activities in trace amounts as well as acting locally at the targeting site by physiological means. They are also expected to disappear immediately after achieving the therapeutic effect. However, generally, even though a part of the administered drug exhibits its therapeutic effect after arriving at the objective active site, the majority of the other parts are distributed into other tissues, then metabolized and excreted. During this process, large side effects may be exhibited. However, high dose administration may have to be performed in order to obtain the necessary amount at the target site. In addition, there are the following problems: (1) since the physically active protein is easily decomposed by the action of enzymes in vivo; and (2) since such a protein may have multiple physiological activities with low targeting directionality, severe side effects can be produced due to distribution to the non-targeted organs or peripheral cells.

As for the most effective procedure to solve such problems, it has long been recognized that in order to obtain the optimum therapeutic effects of the physiologically active protein, the importance of the medication method must be considered. In other words, the importance of transferring the drug selectively into the objective site where the pharmaceutical action should be expressed, in the necessary amount and at the critical time, [namely drug delivery system (DDS)] has been strongly recognized. Among other possibilities, the targeting (targeting directionality) for selectively transferring the drug into the target site is absolutely required in order to achieve effective pharmacotherapy. The targeting DDS (T-DDS), through which the physiologically active protein is accumulated into the target organ or cells by controlling the biotransformation of the physiologically active protein by means of the drug delivery system (DDS), has drawn attention. This is the case for many therapeutic objectives using physiologically active proteins.

However, the only successful known case of T-DDS at present is the case of IL-2-diphtheria toxin fused protein, which can selectively attack the T cell cutaneous lymphoma, which is expressing interleukin-2-receptor (IL-2R). [In the U.S., this drug has been approved by the government. Nichols, J. et al., Eur. J. Cancer, 33(1), 34-36 (1997)], but efforts to produce a highly applicable organ specific DDS have not been successful.

A therapeutic method for treatment of liver cancer wherein a combined preparation of lipophilic antitumor agent SMANCS (a conjugate of synthetic polymer styrene-maleate copolymer and protein-based antitumor agent neocarzinostatin)/lipidol (an oily contrast media) is administered intraarterially into the liver in order to gradually infuse it into tumor tissue in the liver has been reported [Oncologia, 15: 9-104 (1995)]. However, the development of a hepatic targeting therapy, which can be applied to the wide range of drugs (especially proteins) for treatment of liver disease, not by such the specific method of local administration but by the possibility for frequent systemic administration, is expected.

Especially, a variety of procedures have been developed for establishing hepatic targeting DDS using physiologically active proteins produced for treatment of liver diseases such as viral hepatitis, liver cirrhosis and liver cancer. Since the liver has the primary functions of metabolizing and decomposing substances, the accumulated proteins are immediately decomposed. Consequently, it is difficult to express the physiological activity at the local area in the liver.

We have found that when a ligand, which has a lower binding affinity for the asialoglycoprotein receptor (ASGP-R), the receptor of which is expressed specifically on hepatocytes in the liver, than the natural sugar chain ligand (asialoorosomucoid), is used as a hepatic recognizing element, the decomposition of the physiologically active protein accumulated in the liver and internalized into the cells, could be avoided in some extent. We had obtained knowledge that a hybrid of an interleukin-2 (IL-2) and a synthetic low binding affinity ligand {for example three-branched type galactose ligand [hereinafter designates as $(Gal)_3$]} with one-to-one hybridization prepared by using a protein-modifying enzyme transglutaminase exhibits liver accumulation and is difficult to internalize into hepatocytes in the liver, and also strongly reduces tumors, as compared with the unmodified IL-2 in a mouse hepatic tumor model. As a result, we have filed patent application relating to a physiologically active protein modified by low affinity ligand for

ASGP-R, in which the physiologically active protein could have possibility for targeting the liver (WO 98/13381).

It is known that ASGP-R, which carries uptake of plasma glycoprotein into the liver, recognizes galactose (hereinafter designates as Gal) or N-acetylgalactosamine (hereinafter designates as GalNAc), which have branched terminal sugar chains, and binds with them, subsequently it internalizes glycoprotein into cells by receptor-mediated endocytosis and finally decomposes it [Ashwell, G. and Harford, J., Ann. Rev. Biochem., 75(3): 531-554 (1982)]. Previously, there have been many attempts to internalize the drug selectively into cells by enhancing its binding affinity with ligands having branched Gal [Wu G. Y. et al., J. Biol. Chem., 263: 4429-4432 (1982); Merwin, J. R. et al., Bioconjugate Chem., 5: 612-620 (1994)]. No attempts like the use of the low affinity ligand by us are known.

The physiologically active protein modified by using low affinity ligand to ASGP-R, invented by us, was the first case wherein systemically administered physiologically active proteins could be accumulated in the liver and also exhibit pharmacological action.

We used ELISA to measure the concentrations of our compounds in liver and plasma after intravenous administration into mice. The results of these assays showed that levels of the low affinity ligand modified physiologically active protein, such as the three-branched galactose ligand modified interleukin 2 [hereinafter designates as $(Gal)_3$-IL-2] were decreased in a manner related to the time-course. Considering that the organ which determines disappearance of $(Gal)_3$-IL-2 systemically is mainly the liver, these results indicate that the suppressing effect by means of $(Gal)_3$ modification is insufficient for complete disappearance of IL-2 in the liver. Namely, although decomposition of $(Gal)_3$-IL-2 accumulated in the liver can partly escape from ASGP-R-mediated endocytosis after binding to the ASGP-R, other molecules may be decomposed as a result of internalization into the hepatocytes in the liver by endocytosis.

Consequently, the modification using the parameter of the affinity to the receptor of the target organ is insufficient for a complete targeting DDS, and it was found that further improvement was necessary to obtain the complete targeting DDS. Especially, in order to obtain a complete DDS for targeting the ligand, the theory of avoiding as much as possible the internalization into cells via specific receptors such as ASGP-R on the cell membrane should be developed, and development of a method for ligand screening based on such a theory is required.

## Disclosure of the Invention

An aspect of the present invention is to develop a method for screening a ligand, which can avoid the internalization thereof into cells, employing the guidance of characteristics of such a ligand, by understanding the characteristics of the ligand having capabilities for accumulating specifically in the target tissue as a result of avoiding internalization mediated by the receptor as much as possible, and collecting the ligand around the specific receptor such as ASGP-R on the cell membrane by binding affinity after systemic administration and to find the optimized ligand. Another aspect of the present invention is to provide the tissue-targeting DDS preparations which can make it possible to accumulate and retain the protein around the receptor by linking it with an optimum ligand and a physiologically active protein by covalent bonding.

## Brief Description of the Drawings

Fig. 1 shows a kinetic model of a ligand and a receptor on the cell surface.

Fig. 2 shows the surface-bound radioactivity (●), the internalized radioactivity (□), the radioactivity of degradation product (×) and the radioactivity of intact $^{125}$I-labeled IL-2 (Δ) of $(Gal)_3$-IL-2 in liver cells of mice expressed in a time-dependent manner (min.).

Fig. 3 shows the surface-bound radioactivity (●), the internalized radioactivity ( □ ), the radioactivity of degradation product (×) and the radioactivity of intact $^{125}$I-labeled IL-2 (Δ) of $(GalNAc)_3$-IL-2 in liver cells of mice expressed in a time-dependent manner (min.).

Fig. 4 shows the time course of internalization and degradation of $(Gal)_3$-IL-2 by the primary cultured mouse hepatocytes. In Fig. 4: filled dots (●) show the surface-bound radioactivity; filled squares (■) show the internalized radioactivity; and open triangles (Δ) show the degradation product.

Fig. 5 shows the time course of internalization and degradation of $(GalNAc)_3$-IL-2 by the primary cultured mouse hepatocytes. In Fig. 5: filled dots (●) show the surface-bound radioactivity; filled squares (■) show the internalized radioactivity; and open triangles (Δ) show the degradation product.

Fig. 6 shows the time course of internalization and degradation of IL-2 by primary cultured mouse hepatocytes. In Fig. 6: filled dots (●)show the surfaced-boundradioactivity; filled squares (■) show the internalized radioactivity; and open triangles (Δ) show the degradation product.

Fig. 7 shows a physiologically-based pharmacokinetic model for ligand-IL-2.

Fig. 8 shows results of a simulation of plasma levels of ligand-IL-2 conjugates. In Fig. 8, a bold solid line shows

$(Gal)_3$-IL-2; a fine line shows $(GalNAc)_3$-IL-2; and a broken line shows unmodified IL-2.

Fig. 9 shows results of a simulation of the concentration profiles of ligand-IL-2 hybrids in the extracellular space in the liver. In Fig. 9, a bold solid line shows $(Gal)_3$-IL-2; a fine line shows $(GalNAc)_3$-IL-2; and a broken line shows unmodified IL-2.

Fig. 10 shows results of a simulation of IL-2R occupancy of ligand-IL-2 conjugates. In Fig. 10, a bold solid line shows $(Gal)_3$-IL-2; a fine line shows $(GalNAc)_3$-IL-2; and a broken line shows unmodified IL-2.

Fig. 11 shows results of a simulation of the time profiles of IL-2 concentrations in plasma having various $k_{int}$ and $k_{off}$. In the figure: (A) $k_{int}$ = 0.0896/min.; (B) $k_{int}$ = 0.00896/min.; and (C) $k_{int}$ = 0.000896/min.

In the figure: broken lines (1 in each figure), $k_{off}$ = 0.209/min.; dotted lines (2 in each figure), $k_{off}$ = 2.09/min.; fine dotted lines (3 in each figure), $k_{off}$ = 20.9/min.; solid lines (4 in each figure), $k_{off}$ = 0.0209/min.; and fine broken lines (5 in each figure), $k_{off}$ = 0.00209/min.

Fig. 12 shows results of a simulation of the time profiles of IL-2 concentrations in the extracellular space in the liver using ligands having various $k_{int}$ and $k_{off}$. In the figure: (A) $k_{int}$ = 0.0896/min.; (B) $k_{int}$ = 0.00896/min.; and (C) $k_{int}$ = 0.000896/min.

In the figure: broken lines (1 in each figure), $k_{off}$ = 0.209/min.; dotted lines (2 in each figure), $k_{off}$ = 2.09/min.; fine dotted lines (3 in each figure), $k_{off}$ = 20.9/min.; solid lines (4 in each figure), $k_{off}$ = 0.0209/min.; and fine broken lines (5 in each figure), $k_{off}$ = 0.00209/min.

Fig. 13 shows results of a simulation of IL-2R occupancy in the liver using ligands having various $k_{int}$ and $k_{off}$. In the figure: (A) $k_{int}$ = 0.0896/min.; (B) $k_{int}$ = 0.00896/min.; and (C) $k_{int}$ = 0.000896/min.

In the figure: broken lines (1 in each figure), $k_{off}$ = 0.209/min.; dotted lines (2 in each figure), $k_{off}$ = 2.09/min.; fine dotted lines (3 in each figure), $k_{off}$ = 20.9/min.; solid lines (4 in each figure), $k_{off}$ = 0.0209/min.; and fine broken lines (5 in each figure), $k_{off}$ = 0.00209/min.

Fig. 14 shows tissue uptake clearance and Kp value of $(Gal)_3$-IL-2 after 3 min. at 50 μg as IL-2/kg in the mouse.

Fig. 15 shows tissue uptake clearance of $(GalNAc)_3$-IL-2 after 3 min. at 50μg as IL-2/kg in the mouse.

Best Mode for Carrying Out the Invention

We have found that in order to accumulate drugs such as physiologically active proteins specifically in the target tissue, it is important to balance conflicting conditions. We have also found that a ligand, which has a binding affinity to the specific receptor in the target tissue, must have the ability to avoid internalization into the cells in the target tissue, and must have the proper strength of the binding affinity (if the ability to avoid internalization is high, the binding affinity is decreased due to increased $k_{off}$ value). Especially, we have found that in cases in which the drug, such as a physiologically active protein, has an objective that involves not directly targeting the cells in the target tissue but for treating a lesion by activating the immune system around the target cells, it is important to sustain the activity so that the drugs, such as physiologically active protein, has specific affinity to the cells of the target tissue but is not internalized rapidly into the said cells. The same is true in cases in which the drug has an objective of exhibit physiological actions through another receptor on the target tissue as a result of targeting to the target receptor. Especially in cases in which the target tissue is the liver, which metabolizes chemical substances, if the drug is rapidly internalized into the liver parenchymal cells, it is rapidly decomposed and metabolized by the liver parenchymal cells, and the sustaining time for the activity is too short, even if the integration rate of the drug is increased.

Consequently, even if the affinity to the target tissue is increased, and the rate of internalization to the target tissue is improved, simultaneous inactivation of the drug proceeds rapidly by decomposition, and the metabolic actions for the drug at the target tissue cells are not effective. As a result, most of the administered drug does not exhibit the desired therapeutic effect and a large amount of the drug should be administered.

We have studied extensively based on such knowledge and have found that in order to improve drug efficacy, it is important to consider not only the affinity to the target tissue but also the factors for efficiently retaining the drug around the target tissue cells.

Generally, a ligand having binding affinity for a specific receptor binds specifically to the said receptor. According to this principle, although it is possible to accumulate the ligand specifically in the target tissue, most of the ligand specifically bound to the receptor of the target tissue cells is internalized into said cells, because of its affinity, by the action of receptor-mediated endocytosis or non-specific fluid phase endocytosis. As a result, the amount of drug retained around the target tissue cells decreases rapidly. However, the ligand bound with the receptor does not always integrate by internalization into cells but may again be retained around the target tissue cells by dissociating from the receptor. In this way, though the ligand bound with the cell surface receptor of the target tissue is integrated by internalization into cells, or is retained again around the cells by dissociation from the receptor, this is caused by different factors from these affecting affinities to the specific receptor of the cells.

In the present invention, the relationship between the ratio, in which the ligand bound to the receptor is internalized into cells, and the ratio, in which the ligand is dissociated from the receptor and retained again around the cells, is designated as "ability to avoid internalization". The ligand having a large ability to avoid internalization is bound with the receptor of the target tissue cells, but the ratio of internalizing it into cells of the target tissue is low, so that the ligand is retained around the cells of the target tissue for a long time, and as a result, duration in the target tissue is extended. The ligand having specific binding affinity to the receptor is generally integrated by internalizing most of the bound ligand into the cells, and is metabolized within a short time. Contrary to that, with some ligands, the ligand bound with the receptor is easier to dissociate than to internalize into cells. It was thought that as a result of binding such a ligand with a drug such as a physiologically active protein, the ligand might be retained around the cells after accumulation in the target tissue. Finding a ligand which dissociates easily means selecting a ligand having a large dissociation rate constant $k_{off}$.

Generally, since a strength of binding affinity of a ligand to a receptor is expressed by the equation:

$$1/Kd = k_{on}/k_{off}$$

wherein $k_{on}$ is a binding rate constant and $k_{off}$ is a dissociation rate constant. If $k_{off}$ becomes larger, the binding affinity becomes lower and the accumulation efficacy to the target tissue is decreased. We have found that a targeting DDS preparation, which has a long duration time in the target tissue, could be obtained by properly balancing two conflicting conditions such as the "ability to avoid internaliztion" and the intensity of the binding affinity. We have also found a method for screening such ligands, and this lead to completing the present invention.

Accordingly, the present invention relates to a method for screening a targeting DDS preparation to a target tissue, said method for screening comprising a ligand having a binding affinity to a protein such as a specific receptor or antigen present on the cell membrane of a target tissue or a substance modified with said ligand employing the guidance of the ability to avoid the internalization mediated by the binding to the receptor and the strength of the binding affinity. More particularly, the present invention pertains to the method for screening the ligand or the substance modified with said ligand wherein the ability to avoid the internalization mediated by the binding to the specific receptor is a ratio ($k_{off}/k_{int}$) of the dissociation rate constant ($k_{off}$) and the internalization rate constant ($k_{int}$) to said receptor; the ratio ($k_{off}/k_{int}$) is 1 or more; and the dissociation constant (Kd) involved in the binding affinity is 10 mM or less.

More particularly, the screening method of the present invention relates to the method of screening the ligand or the substance modified with the ligand comprising incubating the ligand or the substance modified with the ligand having the binding affinity to the specific receptor or the protein such as an antigen (hereinafter designates as receptors) present on the cell membrane in the presence of free cells or cultured cells which express said receptor, washing the ligand or the substance modified with the ligand, incubating at low temperature for suppressing internalization into the cells by an addition of the labeled ligand to said receptors, and screening the ligand or the substance modified with the ligand in which amount of binding of the labeled ligand to the cell surface is higher as compared with the amount of binding in case of adding initially the unlabeled said affinity ligand; or the method of screening the ligand or the substance modified with the ligand comprising incubating the ligand or the substance modified with the ligand having the binding affinity to the specific receptors present on the cell membrane in the presence of free cells or cultured cells which express said receptors, washing the ligand or the substance modified with the ligand with a buffer containing a chelating agent or an acidic buffer, incubating at low temperature for suppressing internalization into the cells by adding the labeled ligand to said receptors, and screening the ligand or the substance modified with the ligand in which amount of binding of the labeled ligand to the cell surface is higher when compared with the amount of binding in the case in which the ligand having the ratio of the dissociation rate constant and the internalization rate constant after binding with the receptors being 1 or more is added.

Further, the present invention relates to the device which can perform the screening method of the present invention herein described earlier, the reagent kit performing the same, the ligand appropriate to the targeting DDS preparations screened by such a method, the compounds appropriate to the ligand, and the pharmaceutical compositions which comprised of the physiologically active substance having the ligand appropriate to the targeting DDS preparations screened by such method, and the pharmaceutically acceptable carrier.

The present invention will be explained in detail in the following, and, at first, terms used in this specification are explained.

A dissociation rate constant ($k_{off}$): A value indicated by dividing the dissociation rate of the ligand from the receptor by the amount of binding on the surface area at that time. Concretely, to illustrate the calculation equation in case of calculating the value in the screening method of the present invention, it can be expressed by the following equation.

$$K_{off} = X_{off} / \int_0^t X_s \, dt$$

wherein $X_{off}$ is the cumulative amount of dissociated ligand and $X_s$ is the amount of binding on the surface area.

Namely, $K_{off}$ can be obtained by dividing the cumulative amount of the dissociated ligand ($X_{off}$) by the integral value of the amount of binding on the surface area ($X_s$) up to that time.

Internalization rate constant ($k_{int}$): A value obtained by dividing the internalization rate of the ligand bound to the receptor by the amount of binding on the surface area at that time. Concretely, to illustrate the calculation equation used in the case of calculating the value in the screening method of the present invention, it can be expressed by the following equation.

$$K_{int} = X_{int} / \int_0^t X_s \, dt$$

wherein $X_{int}$ indicates the cumulative amount of integrated ligand and $X_s$ indicates the amount of binding on the surface area.

Namely, $K_{int}$ can be obtained by dividing the cumulative amount of the internalized ligand ($X_{int}$) by the integral value of the amount of binding on the surface area ($X_s$) up to that time.

Dissociation constant (Kd): A value obtained by dividing the dissociation rate constant ($k_{off}$) by the binding rate constant ($k_{on}$) of the receptor. It is a barometer of the affinity between the receptor and the ligand. This is shown by the following equation.

$$Kd = k_{off} / k_{on}$$

In order to elucidate conditions of the ligand accumulating and being retained in the liver, we have already reported the synthetic method of various ligands (WO 98/13381).

Among these ligands, we have first examined two types of ligands for asialoglycoprotein receptor on hepatocytes, i.e. the three-branched galactose ligand [$(Gal)_3$] of the following formula:

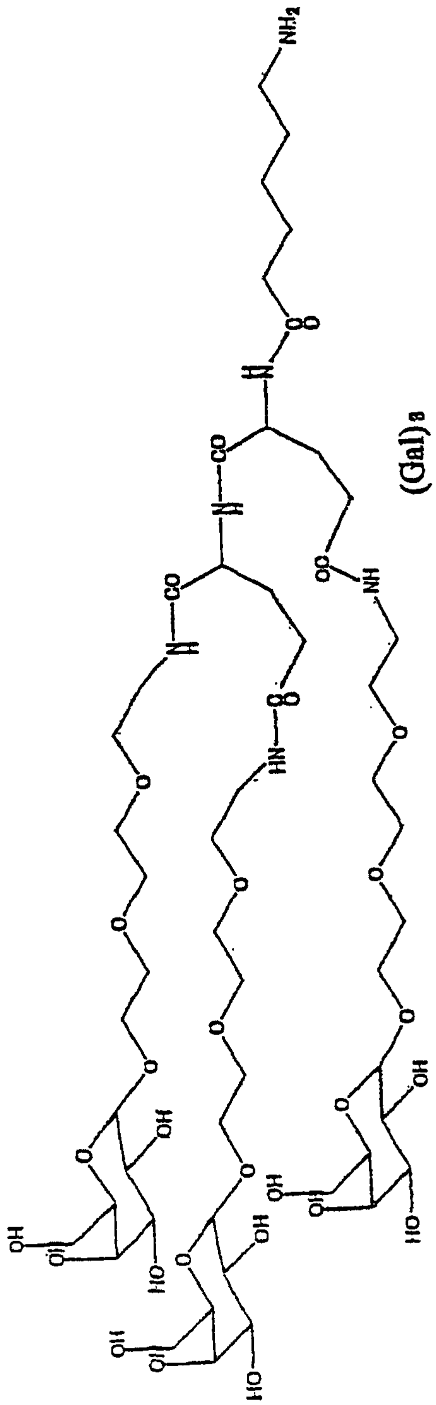

and the three-branched N-acetylgalactosamine ligand [$(GalNAc)_3$] of the following formula:

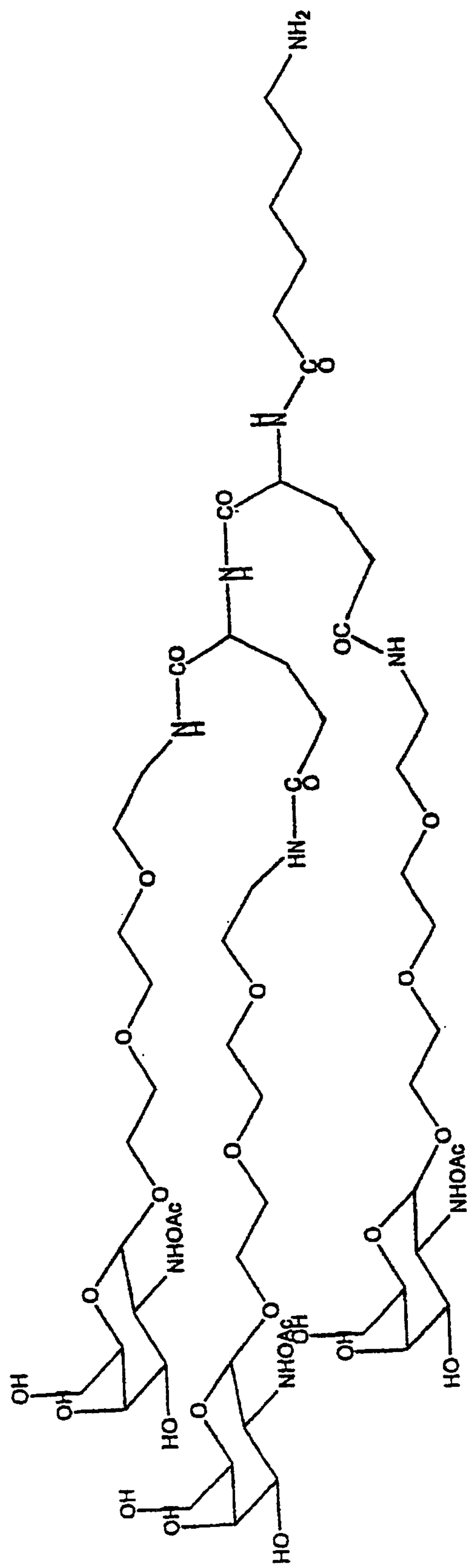

(GalNAc)$_3$

and their binding forms to IL-2. At first, the Kd value, which is an index of the binding affinity to ASGP-R, was calculated. Results are shown in Table 1.

Table 1,

| Binding inhibitory constant (Ki) of each ligand for binding of $^{125}$I-ASOR of each ligand to mouse hepatocytes | | | |
|---|---|---|---|
| | ASOR | $(GalNAc)_3$ | $(Gal)_3$ |
| 1 | 0.0074 | 2.95 | 681 |
| 2 | 0.0044 | 0.65 | 351 |
| 3 | 0.0100 | 1.37 | 292 |
| Ave. (μM) | 0.0072 | 1.65 | 354 |
| s. d. | 0.0028 | 0.96 | 266 |

Since a binding inhibitory constant (Ki) is thought to be a bond dissociation constant, the Kd value for each ligand was calculated from the results of Ki in Table 1. As a result, it was found that the Kd value of $(GalNAc)_3$ was 1.65 μM, indicating relatively high affinity, whereas that of $(Gal)_3$ was 354μM, in which the affinity was approximately 1/200 of $(GalNAc)_3$, indicating a low affinity ligand. Tumor degeneration effects of conjugates of ligands having different affinities as such and IL-2, [i.e. $(Gal)_3$-IL-2 and $(GalNAc)_3$-IL-2], were compared including IL-2 alone in a mouse hepatic tumor model. Results are shown in Table 2.

Table 2, Antitumor effects of (Gal)3-IL-2, (GalNAc)3-IL-2 and IL-2 in mouse hepatic tumor model

| | Physiological Saline | IL-2 | | $(Gal)_3$-IL-2 | | $(GalNAc)_3$-IL-2 | |
|---|---|---|---|---|---|---|---|
| | | low dose | high dose | low dose | high dose | low dose | high dose |
| Size of tumor (mm) | 6.83 | 1.60 | 1.55* | 0.65* | 0.53* | 3.72 | 3.86 |
| (S. D.) | (6.32) | (0.39) | (1.18) | (1.01) | (0.91) | (5.61) | (4.54) |
| Number of cured | 0/5 | 0/7 | 0/6 | 4/7** | 5/7** | 0/7 | 0/6 |
| Number of died | 2/7 | 0/7*** | 1/7 | 0/7*** | 0/7 | 0/7*** | 1/7 |

In Table 2, * : $p < 0.05$ (Dunnett's t-test)

** : $p < 0.01$ (Fisher's protected LSD)

*** : $p < 0.05$ (Fisher's protected LSD)

The intensities of the antitumor effects were in the order of $(Gal)_3$-IL-2 > IL-2 > $(GalNAc)_3$-IL-2. Since the target of IL-2 is the antitumor effector cells, in which the IL-2 receptor of the liver extracellular fluid is expressed on the surface, this indicated that IL-2 could efficiently activate the effector cells by the action of the $(Gal)_3$ ligand. On the

other hand, when a high affinity ligand $(GalNAc)_3$ was bound, although accumulation to the liver was improved, it was shown that the effector cell could not be efficiently activated in the local region of the liver. Accordingly, in order to elucidate the reason for the usefulness of the action of the $(Gal)_3$ ligand, kinetic analysis on internalization to the hepatocytes was performed.

The kinetic model for the internalization of the ligand for the receptor or proteins such as antigens on cells is explained.

Fig. 1 shows a kinetic model of ligand and receptor on the cell surface.

In Fig. 1, the lower side is the intracellular part and the upper side is the extracellular part. Between them, the cell surface exists. Receptor (Rs) is generally projected to the extracellular part through the cell membrane and is bound with the ligand which exists in the extracellular part.

The receptor (Rs) in Fig. 1 is bound with the extracellular ligand at a first-order rate constant ($k_{on}Rt$) to form ligand-receptor complex (LRS). The thus formed ligand-receptor complex (LRs) is internalized into the cell at a rate known as the internalization rate constant ($k_{int}$) or the ligand and the receptor are dissociated at a rate known as the dissociation rate constant ($k_{off}$).

The ligand in the ligand-receptor complex (LRi) internalized into the cell is decomposed at a degradation rate constant ($k_{deg}$) and is released into the extracellular part as the degradation product ($X_{deg}$). In Fig. 1, Vr indicates the 0-order rate constant of the appearance of the receptor at the cell surface and $k_t$ indicates the rate constant when the receptor which is not bound with the ligand is stored into the cell.

Experiments in the internalization of ligand were performed by the pulse-chase experiment.

The results of changes in the internalization of $^{125}I$ labeled ligand, $^{125}I$-$(Gal)_3$-IL-2 and $^{125}I$-$(GalNAc)_3$-IL-2 into mice primary cultured hepatocytes are shown in Fig. 2 and Fig. 3. In Fig. 2 and Fig. 3 : closed circles (●) show the amounts of bound fraction on the receptor surface; open squares (□) show the amounts of internalized fraction into the cells; open triangles (Δ) show the amounts of dissociated fraction to the extracellular part; and crosses (×) show the amounts of fraction of the degradation product. These values are shown in the form of a distribution volume (μl/mg cell protein) divided by the added concentration.

In this experimental system, since the amount of specific fraction mediated through ASGP-R was exceeded by 90% or more, changes of the specific fraction are observed in all cases in each amount of fraction. As a result, differences in changes of internalization of both ligands were observed. Namely, $(Gal)_3$-IL-2 bound with ASGP-R on the cell surface was mainly dissociated into the medium by incubation at 37°C and the ratio internalized into the cell was low as compared therewith. Contrary to that, in $(GalNAc)_3$-IL-2, the internalization was the main effect and the ratio of dissociation fraction tended to be lower as compared with the internalization fraction.

Further, integration plots of the internalization and the dissociation (X axis: area under the time curve (AUC) of the amount of surface binding up to t min.; and Y axis: amount of internalization or dissociation after t min.) were performed, and internalization and dissociation rate constants [$k_{int}$ and $k_{off}$ ($min^{-1}$)] were calculated from the slope. Results are shown in Table 3.

Table 3,

| Rate constants of $^{125}I$-$(Gal)_3$-IL-2 and $(GalNAc)_3$-IL-2 in internalization to hepatocytes | | | |
|---|---|---|---|
| | $k_{int}$($min^{-1}$) [S.D.] | $k_{off}$($min^{-1}$)[S.D.] | $k_{off}/k_{int}$ |
| $(Gal)_3$-IL-2 | 0.0344[0.0110] | 0.0804[0.0110] | 2.34 |
| $(GalNAc)_3$-IL-2 | 0.0186[0.00515] | 0.0118[0.00257] | 0.601 |

Numerical values in Table 3 indicate the mean of 3 repeats of the experiments and numerical values in [ ] indicate standard deviations (S.D.).

As a result, no large differences in $k_{int}$ were observed [$(Gal)_3$-IL-2: 0.034 $min^{-1}$ and $(GalNAc)_3$-IL-2: 0.0184 $min^{-1}$]. However, $k_{off}$ of $(Gal)_3$-IL-2 was about 8 times higher than that of $(GalNAc)_3$-IL-2 [$(Gal)_3$-IL-2: 0.0804 $min^{-1}$ and $(GalNAc)_3$-IL-2: 0.0118 $min^{-1}$].

A ratio of $k_{off}$ and $k_{int}$ ($k_{off}$ / $k_{int}$), i.e. the ability to avoid the internalization, at this time, was 2.34 in $(Gal)_3$-IL-2, whereas it was 0.601 in $(GalNAc)_3$-IL-2, showing a large difference between them.

Next, experiments on internalization were performed by a continuous incubation method and the results were examined.

Results of changes in the internalization of $^{125}I$ labeled ligand, $^{125}I$-$(Gal)_3$-IL-2, $^{125}I$-$(GalNAc)_3$-IL-2 and $^{125}I$-IL-2 into mice primary cultured hepatocytes are shown in Fig.4, Fig. 5 and Fig. 6. In Fig. 4 - 6: closed circles (●) show the amount of bound fraction on the cell surface; closed squares (■) show the amount of internalized fraction into the cells; and open triangles (Δ) show the amount of fraction of the degradation product. These values are shown in the form of a distribution volume (μl/mg cell protein) divided by the added concentration.

In this experimental system, specific fractions mediated by ASGP-R to liberate free GalNAc and other non-specific fractions were separated and evaluated. In the cases of $(Gal)_3$-IL-2 and $(GalNAc)_3$-IL-2, as in the above pulse chase method, the specific fractions occupied 80% or more. Since, in the experiment with IL-2, free GalNAc was observed in the majority of cases and undischarged binding, internalization and degradation were observed, the total amounts of each fraction were shown.

The results show that the changes in internalization of $(Gal)_3$-IL-2 and $(GalNAc)_3$-IL-2, the binding to the hepatocytes surface, and the internalization into cells were all observed immediately after initiation of the incubation, and amount of degradation in the incubation medium was increased after reaching the constant state. This is typical of endocytosis mediated by the receptor. Proviso that the absolute value of the distribution volume is 4 - 5 times higher in $(GalNAc)_3$-IL-2 than in $(Gal)_3$-IL-2, indicating that $(GalNAc)_3$-IL-2 tends to be high in its discharge clearance in the liver. Based on these results, the changes in each fraction of both compounds were performed the Fitting using the Napp procedure [Hisaka, A. et al., J. Pharmacokinet. Biopharm., 26(5), 495-519 (1998)], and the rate constant of internalization was calculated. The fitting using Napp was performed by fixing the ratio of $k_{off} / k_{int}$ obtained by the pulse chase method. Namely, $k_{int} = k_{off} \times 0.428$ for $(Gal)_3$-IL-2 and $k_{int} = k_{off} \times 1.576$ for $(GalNAc)_3$-IL-2 were used.

Table 4, Rate constants of $^{125}$I-$(Gal)_3$-IL-2 and $^{125}$I-$(GalNAc)_3$-IL-2 in internalization into hepatocytes

| | $k_{int}$ ($min^{-1}$) | $k_{off}$ ($min^{-1}$) [S.D.] | $k_{deg}$ ($min^{-1}$) [S.D.] | $k_{on}$Pt (μl/min/mg cell protein |
|---|---|---|---|---|
| $(Gal)_3$-IL-2 | 0.0896 | 0.2094 (0.0806) | 0.0183 [0.0023] | 0.9864 [0.0520] |
| $(GalNAc)_3$-IL-2 | 0.0451 | 0.0286 (0.0053) | 0.0232 [0.0029] | 2.4329 [0.1074] |

As a result, the $k_{int}$ and $k_{off}$ were almost equal to the values obtained by the pulse chase method and each of dissociation rate constant [$k_{deg}$ ($min^{-1}$)] and binding clearance [$k_{on}$Rt (ìl/min/mg cell protein)] was obtained. No large differences between $(Gal)_3$-IL-2 and $(GalNAc)_3$-IL-2 for $k_{deg}$ were observed, but $k_{on}$Rt, for $(GalNAc)_3$-IL-2 was found to be approximately a 2.5 times higher value than $(Gal)_3$-IL-2.

Further, IL-2 showed degradation as a result of a non-specific internalization without mediating ASGP-R, though the mechanism of the internalization is unknown. A slope, which is an index of the degradation rate constant, for the change was approximately 1/3.33 of that for $(Gal)_3$-IL-2. Consequently it was determined that the disappearance in the liver in vivo could not be neglected in the case of the unmodified IL-2. According to the ratio of the gradient (f = 3.33), the clearance in the liver (CLh) of IL-2 was estimated as 2.94 ml/min/kg by the method shown in the example below.

Next, a kinetic model of the ligand in vivo is explained. In order to explain differences in the drug effects of $(Gal)_3$-IL-2, $(GalNAc)_3$-IL-2 and IL-2 used in the above experiments, a pharmacokinetic model based on physiological knowledge was constructed. That model is illustrated in Fig. 7.

The parameters used are pharmacokinetics and kinetic parameters, and the physiological parameters obtained by in vivo and in vitro experiments. These are shown below.

$k_{on}Rt$ ; association clearance of the ligand to the receptor [a)]
$k_{off}$ ; dissociation rate constant of the ligand-receptor complex [a)]
$k_{int}$ ; internalization rate constant of the ligand-receptor complex [a)]
$k_{deg}$ ; degradation rate constant of the internalized ligand-receptor complex [a)]
F ; coefficient of correlation from in vitro to in vivo [b)]
Vb ; distribution volume in plasma [c)]
Ve ; volume of extracellular fluid from liver (0.2 × 1.3 × 1000/22 = 11.8 ml/kg)
$Q_H$ ; flow rate of plasma in the liver [c)]
$CL_{other}$ ; external clearance of the liver [d)]
$CL_h$ ; non-specific clearance in the liver [e)]
Dose ; dose of the ligand ($5 \times 10^4$ ng/kg)
$Kd_{IL2}$ ; dissociation constant for IL-2 receptor (0.155 ng/ml)

Note: (a) is experimental values (refer to Table 3)
(b) is an estimated value obtained from in vitro and in vivo experiments and is set as 36.9.
(c) is values obtained from in vivo experiments.
(d) is used as the experimental value obtained from actual clearance.
(e) is the value obtained from an internalization experiment using IL-2 in vitro.

Since the main organ for distribution and disappearance of the ligand modified IL-2 is the liver, the simple compartment physiological pharmacokinetic model consisting of extracellular fluid and the blood circulation system of the liver was used. Simulation was performed using Stella [Bogen et al., Science, 246: 138-142 (1989)] to estimate the calculation of in vivo pharmacokinetics. An experimentally observed non-specific disappearance clearance in the liver was added as a model for IL-2, and binding clearance for ASGP-R was set as 0. The rate equation for the ligand-IL-2 conjugates concentration in each fraction is shown in the following.

(In the plasma fraction)

$$dCb/dt = (-Q_H{}^*Cb + Qh{}^*Ce)/Vb$$

(In liver extracellular fraction)

$$dCe/de = (Q_H{:}Cb - k_{on}RT{}^*Ce{}^*F + k_{off}{}^*Xs - Q_H{}^*Ce)/Ve$$

(Liver cell surface binding fraction)

$$dXs/dt = k_{on}Pt{}^*Ce{}^*F\ (k_{off} + k_{int}){}^*Xs$$

(Liver intracellular fraction)

$$dXi/dt = k_{int}*Xs - k_{deg}*Xi$$

(Decomposed fraction)

$$dX_{deg}/dt = k_{deg}*Xi$$

(Receptor occupancy ratio)

$$Receptor\ Occupancy = Ce/(Kd + Ce)$$

In this case, fate of the ligand-IL-2 or IL-2 after the extracellular fluid is assumed to be following two routes: ① binding to ASGP-R surface ② internalization to hepatocytes or dissociation to extracellular fluid ③ degradation by means of the mechanism of endocytosis (A); and ① ' binding to antitumor effector cell surface in extracellular fluid (② activation of the effector cells) (B). The antitumor effect of IL-2 in the liver region is thought to increase by proceeding amount and residence time of IL-2 to transfer into route (B). On the contrary, if a ratio of amount of IL-2 transferring into route (A) is too high, since decrease of residence in blood occurs caused by excess degradation, IL-2 can not exhibit its pharmacological action in the liver region.

Among the parameters explained earlier, as for (a), values obtained from data from the uptake experiment of $(Gal)_3$-IL-2, and (b) on uptake clearance by organs except for the liver, the values for renal clearance of $(Gal)_3$-IL-2 were used. The liver clearance of IL-2 ($CL_{NSP}$) in (c) was calculated as the ratio of the degradation rate from non-specific uptake of IL-2; and the degradation rate of $(Gal)_3$-IL-2 obtained by continuous incubation is set as equal to a ratio of each of the liver intrinsic clearance values. The intrinsic clearance of $(Gal)_3$-IL-2 ($CL_{int}$ h) herein is represented by the equation:

$$CL_{int}\ h\ (Gal) = F*k_{on}Rt\ (Gal) * k_{int}\ (Gal) / [k_{off}\ (Gal) + k_{int}\ (Gal)]$$

wherein F is the aforementioned conversion constant and its calculation will be described later.

Among the parameters used, the liver extracellular volume (Ve) was calculated according to the report by Miyauchi et al. that its volume is approximately 20% of organ weight [S. Miyauchi, et al. J. Pharmacokin. Biopharm. 15: 25-38 (1987)].

Q (the liver plasma flow rate) and F [conversion constant of $k_{on}Rt$ (binding clearance) obtained by the in vitro to the in vivo model ] were determined as follows. Namely, the respective liver uptake clearance $CL_{uptake}Li$ of $(Gal)_3$-IL-2 and $(GalNAc)_3$-IL-2 was obtained by examination of in vivo pharmacokinetics after intravenous injection of $^{125}I$-labeled compound. This may be thought to reflect mainly the binding clearance ($k_{on}Rt$) calculated from the in vitro experiments. Intrinsic clearance in relation to uptake in the liver ($CL_{int}$, uptake) should be:

$$CL_{int},\ uptake = k_{on}Rt \times F$$

wherein F is a conversion constant from in vitro to in vivo. Consequently, according to Well-stirred model, the above is as follows:

$$CL_{uptake}Li = Q_H * CL_{int},\ uptake / (Q_H + CL_{int},\ uptake)$$

With the proviso that $CL_{int}$, uptake = F $\times$ ($k_{on}Rt$ , vitro)

With regard to this, the values of $Q_H$ and F were calculated by solving the respective simultaneous equations for $(Gal)_3$-IL-2 and $(GalNAc)_3$-IL-2.

In addition, since no decrease in the biological activities resulting from modification was observed in the in vitro biological activity assay using IL-2 dependent CTLL-2 cells, the dissociation constants of ligand-IL-2 and IL-2 to IL-2 receptor were set all at the same value (Kd = 10 pM; high affinity).

According to these results, in order to elucidate the cause for the differences in kinetics in vivo between the modified IL-2 with two types of ligand and the unmodified IL-2 used in the comparison with their respective pharmacokinetics, a predictive calculation using the physiological model was performed. It was based on the values obtained by in vivo and in vitro experiments. Results of simulations of the transition of time for: plasma levels of ligand-IL-2 hybrid (Fig. 8); liver extracellular fluid level (Ce ext) (Fig. 9); and occupancy ratio (RO) of IL-2R by conjugates (Fig. 10) are shown in Fig. 8, Fig. 9 and Fig. 10. In Fig. 8, Fig. 9 and Fig. 10: bold solid lines indicate $(Gal)_3$-IL-2; fine solid lines indicate $(GalNAc)_3$-IL-2; and broken lines indicate unmodified IL-2.

For $(GalNAc)_3$-IL-2, which produced the lowest tendency of the drug effect, it was found that its disappearance from plasma and liver extracellular fluid, from the initial stage after administration to about 10 minutes, was very fast as compared with the others. Consequently, it was confirmed that the blood concentration of this ligand was largely decreased due to its rapid disappearance, mediated by ASGP-R after administration. However, its retainability after 10 minutes was the highest. This may be due to the gradual release of the fraction that is strongly bound to ASGP-R into the circulating blood. Though the occupancy ratio of IL-2 receptor, which might be an index for pharmacological action (Fig. 10), was the lowest up to about 55 minutes after administration, the decrease after 10 minutes of administration was relatively slow.

IL-2, which produced the second highest tendency of the drug effect, was the highest in its plasma level and in its liver extracellular level (Ce ext) immediately after the administration of IL-2. No retainability in the blood or in the liver extracellular fluid was observed thereafter, and the concentration was reversed by administration of $(Gal)_3$-IL-2 at 30 minutes after the initial administration and by $(GalNAc)_3$-IL-2 at about 50 minutes after the initial administration. As for RO, the same tendency was observed, and although it was highest as compared with others for about 30 minutes after administration, its decrease was the fastest. Consequently, it is suggested that IL-2 has a problem of retainability when time has elapsed after administration, to some extent.

Contrary to that, $(Gal)_3$-IL-2, which produces the highest tendency of the drug effect, showed intermediate changes exhibiting both characteristics during the changes in time indicated for $(GalNAc)_3$-IL-2 and IL-2. Namely, both the plasma levels and Ce ext levels, immediately after administration showed intermediate values between them, and the retainability after 10 minutes later was almost between them. As a result of having such characteristics, RO showed a slightly lower level than IL-2 at 30 minutes after administration, but no big differences were noted and the highest level was maintained for 80 minutes.

Results of comparisons of the pharmacokinetics parameters calculated by the previously described predicted calculations, i.e. mean retaining time [MRT (min)], total clearance [$CL_{total}$ (ml/min/kg)], distribution [Vdss (ml/kg)], 75% occupancy time of IL-2 receptor (min) and area under the curve (AUC) of the liver extracellular fluid level [$AUC_{0-\infty}$ (Ce ext) (ng*min/ml)] are all shown in Table 5.

Table 5,

| Pharmacokinetics of $(Gal)_3$-IL-2, $(GalNAc)_3$-IL-2 and IL-2 calculated by the predicted calculations | | | |
|---|---|---|---|
| Parameter | $(Gal)_3$-IL-2 | $(GalNAc)_3$-IL-2 | IL-2 |
| MRT (min) | 11.1 | 9.72 | 8.33 |
| $Cl_{total}$ (ml/min/kg) | 10.6 | 22.2 | 5.62 |
| Vdss (ml/kg) | 117 | 216 | 46.8 |
| 75% occupancy time of IL-2R (min) | 56 | 37 | 48 |
| $AUC_{0-\infty}$ (Ceextra) (ng*min/ml) | 38012 | 7557 | 67277 |

In comparison with the systemic parameters, the total clearance ($CL_{total}$) of $(GalNAc)_3$-IL-2 was the highest, and that of $(Gal)_3$-IL-2 was about 1/2 thereof, and IL-2 showing the lowest value was about 1/4 thereof. Distribution volume (Vdss), which is an index for a volume of distributing drug, was also reflect with the transitional levels and was in the order of $(GalNAc)_3$-IL-2 > $(Gal)_3$-IL-2 > IL-2. According to the theory of pharmacokinetics, decreasing rate of the blood level and the liver extracellular fluid level of ligands is determined by a balance (ratio) of disappearance ability ($CL_{total}$) and a distributibity (Vdss). Calculating the mean retaining time (MRT), which is one of parameters indicating the balance, $(Gal)_3$-IL-2 is the highest. This might be due to having proper affinity and integration rate of $(Gal)_3$-IL-2 to

ASGP-R, showing the effect to retain in the liver extracellular fluid with suppressing disappearance from blood as well as binding with ASGP-R. As a result, $(Gal)_3$-IL-2, which exhibited the highest drug effect, was shown to have well balanced properties as compared with IL-2 and $(GalNAc)_3$-IL-2 in systemic disappearance and retainability.

Comparing with AUC [$AUC_{0-\infty}$ (Ce ext)] of the liver extracellular fluid level, which might be one of index exhibiting exposure of the ligand to IL-2 receptor of the liver, in both ligand modified compounds, $(GalNAc)_3$-IL-2 is about 1/4.4 of $(Gal)_3$-IL-2, reflecting difference between both drug effects. However, result indicating that $AUC_{0-\infty}$ (Ce ext) of IL-2 is higher than that of $(Gal)_3$-IL-2 is conflicting at a glance with the result that the drug effect of IL-2 is inferior than that of $(Gal)_3$-IL-2. The reason why $AUC_{0-\infty}$ (Ce ext) of IL-2 is high is due to high blood level from immediately after administration to 10 minutes later. Since effect of IL-2 and two compounds modified with ligand is exhibited through binding to IL-2R, this index may not reflect higher blood retainability of $(Gal)_3$-IL-2.

As a result of calculating the 75% occupancy time of IL-2R, it reflects potency of drug effect, showing in the order of $(Gal)_3$-IL-2 > IL-2> $(GalNAc)_3$-IL-2. Consequently, although size of $AUC_{0-\infty}$ (Ce ext) is one of important factor of the drug effect of IL-2, it is suggested that the retainability of effective drug level in the liver extracellular fluid in order to properly saturate IL-2R is important.

From the examinations up to now, among two ligands used in this time, it is demonstrated that $(Gal)_3$ has two balance of "disappearance from blood" and "retainability in the extracellular fluid" most properly. Next, in order to design the ligand superior in expression of drug effect, simulation with various setting conditions were performed in the present physiological model based on parameters of $(Gal)_3$-IL-2. Results are shown: transition of plasma level in Fig. 11; transition of liver extracellular fluid level in Fig. 12; and transition of occupancy ratio in IL-2 receptor (RO) in Fig. 13, respectively.

Each of Fig. 11, Fig. 12 or Fig. 13 is a value wherein $k_{int}$ is fixed and $k_{off}$ is changed. Namely, (A) $k_{int}$ is 0.896/min [a value in the case of $(Gal)_3$-IL-2]; (B) $k_{int}$ is 0.00896/min (1/10 of A); (C) $k_{int}$ is 0.000896/min (1/100 of A). A value of $k_{off}$ in each figure shows that 0.209/min, which is a value in the case of $(Gal)_3$-IL-2, is centered (broken line, 1 in each figure), and 10 times thereof (dotted line, 2 in each figure), 100 times thereof (fine dotted line, 3 in each figure), 1/10 times thereof (solid line, 4 in each figure) and 1/100 time thereof (fine broken line, 5 in each figure) are shown.

In case of $k_{int}$ = 0.0896/min [(A) in Fig. 11, Fig. 12 and Fig. 13], it is shown that depending on increase in $k_{off}$ value, ligand-IL-2 conjugates level in the plasma and the liver extracellular fluid is increased and retainability thereof is also improved. Similarly, it is also shown that the occupancy ratio in IL-2 receptor is high in case of high $k_{off}$ value, moreover it retains for a long time [in Fig. 13, (A)]. Such effects may be due to suppressing clearance from the liver as a result of increasing $k_{off}$ value (due to that majority of the ligand bound with ASGP-R is not internalized but dissociated into the extracellular fluid), and reserving in the circulating blood and the liver extracellular fluid. Consequently, reason why the drug effect is stronger in $(Gal)_3$-IL-2 than in $(GalNAc)_3$-IL-2 in the liver cancer model is presumed that $(Gal)_3$-IL-2, wherein $k_{off} > k_{int}$, is higher the liver extracellular fluid level and the retainability that $(GalNAc)_3$-IL-2, wherein $k_{off} < k_{int}$, to increase in the occupancy in IL-2R.

As the results, it was found that in order to overcome the intracellular integration, a value of $k_{off}$ higher than $k_{int}$ is important. Proviso that if value of $k_{off}$ is increased to 100 times of the measured values of $(Gal)_3$-IL-2, the liver extracellular fluid concentration disappears gradually. This may be due to that even in the settled $k_{int}$, effect of uptake in the liver (internalization) is so strong [this also corresponds to $(Gal)_3$-IL-2] that if $k_{off}$ value settled for overcome such the effect, dissociation from the receptor (ASGP-R) becomes very fast, as a result, the determination factor for disappearance from blood causes disappearance from other organs such as kidneys.

As a result for performing the simulation by decreasing $k_{off}$ value to 1/10 and 1/100, it was shown that retainability of the liver extracellular fluid level and the occupancy ratio to IL-2 receptor are increased by increasing $k_{off}$ value in some extent compared to the value of $k_{int}$ [Fig. 12 and Fig. 13 (B) and Fig. 12 and Fig. 13 (C)]. Compared with results in Fig. 12 (B) and (C), such circulating effect tends to be strong when the internalization rate is low. Proviso that in both cases, any of $k_{off}$ values have optimum ranges and if that is too fast, the retainability is inversely decreased. This means that if dissociation is too fast, affinity to receptor (ASGP-R) becomes lower and the receptor effect at the retaining site can not be exhibited. As such, a case that if the dissociation from ASGP-R is faster as compared with the circulation, it is preferable for maintaining retainability in blood, but contrary to that if it becomes faster in some extent, a function as the retaining site of ligand in ASGP-R is lost. Consequently, it is found that absolute value of $k_{int}$ as well as a balance of $k_{int}$ and $k_{off}$ is important for maintaining a proper occupancy ratio in IL-2 receptor.

Results of the kinetic model analysis in the above three specimens are summarized as follows.

(1) Efficiency of disappearance from blood represented by $CL_{total}$ was mot advantageous for IL-2; most disadvantageous for $(GalNAc)_3$-IL-2; and $(Gal)_3$-IL-2 was intermediate between them. The reason is that for $(GalNAc)_3$-IL-2 and $(Gal)_3$-IL-2 are suffered from endocytosis mediated ASGP-R, whereas IL-2 is not. A reason why disappearance efficiency of for $(GalNAc)_3$-IL-2 is high is due to high liver clearance (CLh) as proved in the in vitro experiment.
(2) Effect of retainability expressed by Vdss in the liver extracellular fluid is the most advantageous for $(GalNAc)_3$-IL-

2; the most disadvantageous for IL-2; and $(Gal)_3$-IL-2 was intermediate between them. The reason is that $(GalNAc)_3$-IL-2 and $(Gal)_3$-IL-2 can be retained for long time by binding with ASGP-R, whereas IL-2 can not be retained.

(3) Considering that experimentally obtained drug effect is in the order of $(Gal)_3$-IL-2 > IL-2 > $(GalNAc)_3$-IL-2, importance in the above two factors is suggested. Namely, $(Gal)_3$-IL-2 can disappear from blood with moderate efficiency and may be maintained its blood level in some extent as well as retaining with-affinity to the receptor properly.

(4) Though it is difficult to search in vivo kinetic parameters showing balance between such the two factors, as one example, if MRT is calculated, $(Gal)_3$-IL-2, for which surely the highest drug effect can be observed, is the highest. Further, calculating the occupancy ratio in the receptor with considering affinity to IL-2R, $(Gal)_3$-IL-2 is the longest for occupying 75%; $(GalNAc)_3$-IL-2 is the shortest. Consequently, it is found that analysis using this kinetic model can explain difference of drug effect between two types of ligand modified IL-2 and unmodified IL-2 in some extent.

As explained, the balance between two pharmacokinetic parameters in "disappearance efficiency from blood" and "retainability in the liver extracellular fluid" is extremely important in expressing drug effect of the present ligands, and using the said physiological model, it is indicated that kinetics / evaluation of drug effect / estimation with considering these two factors can be possible. Among three specimens used in the present experiments, the best balanced $(Gal)_3$-IL-2 can perform activation of antitumor effector cells efficiently in the liver region by IL-2. Reason for indicating the strongest drug effect thereof may be as indicated by the kinetic discussion shown by the above experiments.

Based on the above results, in case of considering the ligand necessary for manufacturing targeting DDS preparation with well balanced two pharmacokinetic parameters of "disappearance efficiency from blood" and "retainability in the liver extracellular fluid", it is important to balance between the internalization rate constant ($k_{int}$) and the dissociation rate constant ($k_{off}$) to the receptor having functions, in which the target tissue cells can internalize drugs to metabolize and decompose. As a result, it is indicated that a screening should be made with the guidance of the ability to avoid the internalization mediated by the bonding to the receptor shown by the ratio ($k_{off}$ / $k_{int}$) and the strength of the binding affinity to the receptor ($1/Kd = k_{on}/k_{off}$).

The ability to avoid the internalization in the present invention is explained in detail as follows.

(1) The ability to avoid the internalization indicated by a ratio ($k_{off}$ / $k_{int}$) is preferably 1 or more.
(2) The internalization rate constant ($k_{int}$) is preferably as small as possible.
(3) The dissociation rate constant ($k_{off}$) is preferably suppressed to the proper limit.

Making the dissociation rate constant ($k_{off}$) is set to larger than the internalization rate constant ($k_{int}$), i.e. ($k_{off}$ / $k_{int}$ >1): 1) liver clearance becomes low as well as lowering the disappearance efficient from blood, as a result, the liver extracellular fluid level and occupancy ratio in IL-2R can be maintained at high levels. In any case of the internalization rate constant, if the dissociation rate constant ($k_{off}$) is increased, an area under the drug concentration-time curve (AUC) can be increased.

As a result that the internalization rate constant ($k_{int}$) is made as small as possible, the liver clearance caused by the internalization can be made lower and the retainability of the ligand-protein conjugates in the liver extracellular fluid can be improved.

Further, the retainability in the liver extracellular fluid is increased by enlarging the dissociation rate constant, but if it exceeds too large, effect of the retainability is decreased. This is due to loss of function as a reservoir by ASGP-R. As the dissociation rate constant ($k_{off}$) increases, the factor determining the disappearance curve from the blood is converted from the supply to the blood by dissociation from ASGP-R to the disappearance by the kidneys and other organs, a kind of flip-flop is seen. Consequently, it is preferable to suppress the dissociation rate constant ($k_{off}$) below the value occurring such the flip-flop.

The present invention provides an indication in the screening of the ligand which is important in the manufacture of the targeting DDS preparation. Consequently, a method of the present invention is not limited by the drug as an active ingredient, ligand and concrete conditions of the receptor or proteins such as antigens on the target tissue cell surface and to, provide an index of screening for selection of the ligand. In the following, embodiments of the present invention will be concretely explained but the present invention is not limited within such concrete embodiments.

Examples of the ligands used in the present invention are any of ligands having affinity for the target tissue as the targeting DDS preparation, and the ligands themselves may have pharmaceutical actions, but it is preferable that the ligands themselves have no physiological activities. Examples of such ligands are not limited, if they have the functional groups bound for drugs without showing any adverse effects in vivo. Such ligands can be screened by the screening method of the present invention, further the most preferable ligands can be designed as a result of analysis of the kinetic studies according to the disclosure of the present invention.

Further, it is necessary that the ligands must have affinity for the specific receptor of the cells to some extent,

preferably to have affinity in some extent such as the dissociation constant of 10 mM or less.

Examples of the specific receptor or the proteins such as antigens on the cell surface of the target tissue in the present invention may be to have affinity for the ligand, preferably not to have affinity for drugs. For constructing the targeting DDS preparation, the said receptor is preferably specific for the tissue, but according to the disclosure of the present invention, it is not always necessary to be a tissue specific receptor, but is preferably the receptor wherein the ligand can accumulate to the targeted tissue. For example, in case that the liver is the target tissue, asialoglycoprotein receptor (ASGP-R) which exists prevalently in the liver parenchymal cells is preferable.

Examples of concrete conditions for the receptor and the proteins such as antigens on the cell surface in the present invention are thought to satisfy the any of the following three points.

1. To have the receptor density (Rt) that: $Rt \times k_{int}/Kd$ > intrinsic clearance in the other organs, wherein Rt can be substituted by Bmax of the cell. surface protein.

In this respect, Bmax means the binding volume, and is a value obtained by standardizing the maximum binding amount calculated by Schatchard plot analysis in the binding experiments with the cell counts or the cell protein quantity. Experimentally, it can be calculated by the measured value from the equation:

$$Bmax = Cb^*(Cf + Kd)/Cf$$

wherein Cb is the binding concentration; Cf is the non-binding concentration; and Kd is the binding dissociation constant.

2. The cell surface receptor or the protein are expressed in the target tissue. For example, almost perfect receptor such as asialoglycoprotein receptor.

3. The cell surface receptor or the protein are expressed in the target organs as compared with the other organs. For example, it is preferable that the ratio of the expression rate in the target organ and the expression rate in the other organs exceeds more than 1.

Examples of the receptors which fulfilled such conditions may be, in addition to the asialoglycoprotein receptor (ASGP-R) on the liver parenchymal cells, the mannose receptor expressed for some extent selectively on the liver non-parenchymal cells. Further, although the epidermal growth factor (EGF) receptor, the insulin receptor, the transferrin receptor and the folate receptor have low cell specificity for expression, the Rt is high, consequently the organs having the high expression specificity can be used.

Further, the receptors having higher expression in the specific cancer cells than the normal cells can be used as the target. In addition, examples of the proteins expressed on the cell membrane other than the receptor are various transporters and antigens as the targets. With the proviso that since designing and screening of ligands for the target protein on the cell membrane are easier for the receptor than the other expressed protein, it is preferable to use the receptors.

Examples of drugs of the present invention are advantageously to have the physiological activities, and can be the drugs having physiological activities in themselves. However, even if it has no physiological activity in itself, carriers for the physiologically active substances such as liposome and lipid microsphere can be used. Drug itself having the above described ligand function can also be used.

Preferable examples of drugs of the present invention are the physiologically active proteins which have less effect for their activities by binding with the ligand and possibility to change largely the in vivo kinetics. The preferable drugs in the present invention are to exhibit activity by specifically internalizing into the lesioned cells in the target tissue, or to act for the immune system cells. Further, examples of the drugs of the present invention are preferably the drugs which are expressed in the region in vivo at the critical time and the necessary amount, and carrying'various functions, and are the drugs which can be administered frequently with large amount for obtaining effect in vivo, because when the drugs are administered systemically, various adverse effects may be generated in the distribution other than the target site and the disappearance occurs rapidly.

The drugs used in the present invention can be constructed DDS preparation, which can increase accumulation efficiency of the drug into the target cells or the organs as well as exhibiting continuous effect by combining with the ligand of the present invention.

Examples of such drugs are, concretely, physiologically active proteins, for example, cytokines such as interleukins, interferon $\alpha/\beta/\gamma$ and tumor necrosis factor (TNF), cell proliferation regulatory factor such as hepatocyte growth factor (HGF) and epidermal growth factor (EGF) and antioxidant enzyme such as super oxide dismutase (SOD), catalase and thioredoxin. These physiologically active proteins can be not limited their origins and can be originated from animals, plants and microorganisms . These can be the proteins produced by integrating genes of these proteins or mutants into E. coli, yeast or Chinese hamster ovary cells and expressing them. Further, it may be a chimera with other

proteins. The physiologically active proteins of the present invention are preferably purified as much as possible before use in order to suppress effect of the coexisting proteins as minimum as possible.

The subject of the present invention also includes low molecular weight drugs which have problems of toxicity by distribution in the region other than the target site, for example antitumor agent such as adriamycin, mitomycin C or methotrexate, and antiviral agent such as azidothymidine (AZT) or adenine arabinoside (araA). In addition, natural proteins such as albumin and globulin, various monoclonal antibodies or their parts, polysaccharides such as dextran, chitin, chitosan and inulin, poly amino acids such as polylysine and polyglutamic acid, synthetic resins such as divinyl ether maleic anhydride copolymer (DIVEMA), styrene maleic anhydride copolymer (SMA), polyethylene glycol (PEG) and polyvinyl alcohol, and lipid aggregate carriers such as liposome and lipid microsphere, which are paid attention as the carriers for such the high toxic low molecular drugs, can be the subject of the present invention, since these carriers can be regulated their in vivo kinetics by introducing ligand on their surface.

The ligand of the present invention and the drug can be bound by the conventional methods. The binding is preferably covalent bond, but is not limited. The binding between the ligand and the drug can be made by chemical means, but it can be made also by enzymatic method using transaminase, etc.

Examples of introducing the ligand can be mentioned as follows. In case that the drug is protein, it is preferable in the development of drugs that the complex introduced with the ligand has uniform structure and its biological activity should not be reduced. For that purpose, it is preferable to use the site specific modification, in which the ligand is introduced into the specific Gln residue by applying transglutaminase of the alkylamine introduced ligand developed by us (Japanese Patent Appln. Hei 6-198187), but it is not limited within this.

Further, examples of the other introduction methods may be the selective modification, in which the ligand introduced with SPDP [N-succinimidyl-3-(2-pyridyldithio) propionate], SMPT [succinimidyloxycarbonyl-á-(2-pyridyldithio) toluene] or SMCC [succinimidyl-4-(N-maleimidomethyl) cyclohexane-1-carboxylate] is bound with Cys residue (Goodson and Katre , 1990, Biotechnology 8, 343-346, and Benhar et al. J. Biol. Chem. 269, 13398-13404). Further, a method by Gaertner et al. wherein aminooxy derivative is introduced specifically into N-terminal amino group of the natural or gene recombinant protein, in which N-terminal amino acid of the protein is Ser or Thr, by controlling the reaction condition such as pH, etc. (Bioconjugate Chem. 1996, 7, 38-44). Random modification method such as amino group modification wherein the ligand, to which the functional group such as trichloro-S-triazine, carboxylimidazole or succinimidyl succinate is introduced by conventional methods, is bound with Lys residue, can also be applied. Such method is less selectivity with possibility to inhibit activity of the physiologically active protein and is not the preferable method, but it can be applied in the present invention, if the biological activity of the physiologically active protein is not influenced.

In case that the ligand is the sugar chain, the ligand-protein complex can be prepared directly by preparing N-binding or 0-binding sugar chain protein in the production system in the cell culture. In this case, the sugar chain of the expressed sugar chain protein is further modified or the specific sugar chain is removed to prepare idealistic ligand.

In the method of introducing ligand to the low molecular weight drug or carrier, in addition to the selection of modification applied to the functional groups of the modifiable drug or carrier, the degradability for efficiently exhibiting the pharmacological action intracellularly or extracellularly, namely removability of the ligand as like in the prodrug, should be considered. For that means, various methods, for example, cyanogen bromide, periodate oxidation, carbodiimide, glutaraldehyde, mixed anhydride and SPDP reagent can be mentioned.

In performing the screening method of the present invention, as shown in the previous example of the experiments, individual kinetic values are determined by the experiments and the optimum ligand is selected based on the index disclosed in the present invention. However, in order to determine the individual kinetic values, exact and quantitative experiments should be performed. Consequently, the present invention is not limited within the methods based on the previously described experiments. It is well understandable to the person skilled in the art 'that optimization of the ligand can be made by the more simple methods unless contrary to the spirit of the present invention.

Examples of the simpler and more concrete screening method of the present invention based on the disclosure of the present invention can be mentioned as follows.

The ligand or the substance modified with the ligand can be screened by incubating the ligand or the substance modified with the ligand having the binding affinity to the specific receptor or the protein such as an antigen present on the cell membrane in the presence of free cells or cultured cells which express said receptor, washing the ligand or the substance modified with the ligand, incubating at low temperature for suppressing internalization into the cells by an addition of the labeled ligand to said receptors, and screening the ligand or the substance modified with the ligand in which amount of binding of the labeled ligand to the cell surface is higher as compared with the amount of binding in case of adding initially the unlabeled said affinity ligand.

Alternatively, the ligand or the substance modified with the ligand can be screened by incubating the ligand or the substance modified with the ligand having the binding affinity to the specific receptors present on the cell membrane in the presence of free cells or cultured cells which express said receptors, washing the ligand or the substance modified with the ligand with a buffer containing a chelating agent or an acidic buffer, incubating at low temperature

for suppressing internalization into the cells by adding the labeled ligand to said receptors, and screening the ligand or the substance modified with the ligand in which amount of binding of the labeled ligand to the cell surface is higher when compared with the amount of binding in the case in which the ligand having the ratio of the dissociation rate constant and the internalization rate constant after binding with the receptors being 1 or more is added.

Accordingly, to provide a method, which can select the optimum ligand from the relative index based on the disclosure of the present invention without determining the absolute value in the kinetics, is included in the present invention.

In the present invention, in the condition of screening the ligand having ability to avoid the internalization ($k_{off}/k_{int} > 1$), $k_{off}$ having a value as large as possible, namely having the binding affinity ($1/Kd = k_{on}/k_{off}$) as low as possible, is selected, however since the intensity of the binding affinity has direct correlation with the targeting efficiency to the target tissue, maintaining the targeting preparation should have a premise to maintain the binding affinity in some extent. Concretely, the intrinsic clearance, which is the treating ability intrinsically belonging to the tissues or organs, by the ligand or the receptor with the ligand or substance modified by said ligand, i.e. a value represented by $Rt*k_{int}/Kd$ (proviso that Rt indicates the receptor density) should be larger that the intrinsic clearance in the other tissues or organs.

Actually, simulation of the kinetics by changes of kinetic parameters of the ligand was examined. In case that the values of $k_{on}$ and $k_{deg}$ of $(Gal)_3$ ligand used in the simulation were maintained as they are; the value of $k_{int}$ was lowered to 1/10 or 1/100 and fixed to that value; and the value of $k_{off}$ is changed to 10 or 100-fold of $(Gal)_3$ ligand, the changes of IL-2 levels in the liver extracellular fluid showed that the retainability thereof is lowered in any cases when the value of $k_{off}$ is increased to 10-fold or more. This may be due to that the ability to avoid the internalization ($k_{off}$ / $k_{int}$) is settled to high only by focusing the value of $k_{off}$ and increasing its value, as a result the binding affinity (1/Kd) calculated by the $k_{off}$ becomes too low and the targeting efficiency to the target is decreased, thereby the intrinsic clearance in the other organs exceeds the intrinsic clearance by ASGP-R. According to the results of this simulation, if $k_{on}$ of the ligand is assumed to be constant in case of targeting the ASGP-R, the $k_{off}$ should be lower than the Kd value in the case of 10-fold (approximately 3.5 mM). Consequently, in order that the ligand maintains the targeting efficiency to the target and has the ability to avoid the binding, the ligand must have the binding affinity having the Kd value 10 mM or less, preferably 3.5 mM of less.

The previously described screening method of the present invention is concretely explained by exemplifying the hepatocytes to ASGP-R.

The cultured hepatocytes and various ligands [including $(Gal)_3$] were incubated at 37°C for several minutes. After washing the receptor and the binding fraction to the cell surface with ice-cooled buffer or acidic buffer containing chelating agent, incubation was conducted with the highest affinity ligand, $^{125}$I-asialoorosomucoid (ASOR) at 4°C, then the ligand in which the binding amount with $^{125}$I-ASOR is higher than the addition of $(Gal)_3$, i.e. the ligand, which is more difficult to internalize than the $(Gal)_3$, can be screened. The ligand used in the screening is preferably having the branched galactose (Gal) or N-acetylgalactosamine (GalNAc) and having the binding affinity for some extent with the dissociation constant for ASGP-R on the hepatocytes being 10 mM or less.

For example, the ligand having for some extent smaller binding affinity to ASGP-R can be prepared by reductive amination of the skeletal part having two primary amino groups and lactose by using $NaBH_3CN$. The ligand having lysine and ornithine as the skeletal part is prepared, and the screening method of the present invention described previously can be performed. Further, the ligand is prepared by binding the Gal or GalNAc, which has the spacer having various length bound by amide bond with the branched chain skeletal of glutamic acid, and the aforementioned screening method of the present invention can be conducted.

According to the previously described method, $(Gal)_3(6)$ ligand represented by the following formula having higher ability to avoid the integration than $(Gal)_3$ used in the previous experiment.

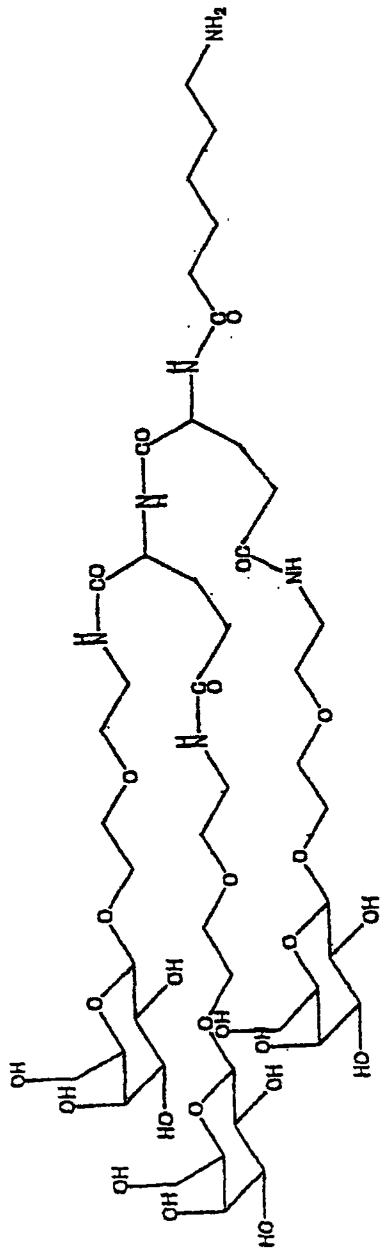

(Gal)$_3$(6)

This (Gal)$_3$(6) has 5 atoms of a spacer between amide group of the (Gal)$_3$ ligand and galactose and has 6 atoms in the spacer including oxygen atom in the galactose. The compound corresponds to the general formula (II) or (V) wherein n is 1 and $R_6$ is $-CH_2CH_2-$.

In order to assay the binding affinity of (Gal)$_3$(6) ligand and (Gal)$_3$ ligand to ASGP-R, the binding inhibition experiment of ASOR to the cultured liver cells was performed. It was found that the binding inhibitory constant (Ki) of (Gal)$_3$(6) ligand, i.e. Kd value 0.369 μM, was larger than that of (Gal)$_3$ ligand (0.157 μM), and the value was 10 mM or less. Namely, it was indicated that the (Gal)$_3$(6) ligand has the ability to avoid the integration exceeding to the (Gal)$_3$ ligand, and the binding affinity was maintained to the intensity possible to targeting.

In order to prove the retaining effect of the drug in the liver by the (Gal)$_3$(6) ligand, (Gal)$_3$(6) ligand and (Gal)$_3$ligand modified IFN α [(Gal)$_3$(6)-IFNα and (Gal)$_3$-IFNα] were prepared, and compared the changes in the liver level after intravenous administration into mice. The half-life of (Gal)$_3$(6)-IFNαfrom 60 minutes to 120 minutes of decreased levels in the liver was approximately twice as longer than that of the (Gal)$_3$-IFNα. Namely, the since the (Gal)$_3$(6)-IFNαligand has higher in the ability to avoid the internalization into hepatocytes, it can retain in the liver for long time.

The method for screening the ligand having the proper binding affinity and the ability to avoid the internalization by rough estimation of Kd, $k_{int}$ and $k_{off}$ more quantitatively, which is different from the screening method by the relative comparative method, is also known and this method is one of the concrete example of the present invention. This

method is concretely explained by exemplifying the case of the hepatocytes to ASGP-R.

The binding inhibitory experiments of the labeled high affinity ASOR to the cultured hepatocytes were performed in each ligand. Each binding inhibitory constant (Ki), i.e. the binding dissociation constant (Kd) was calculated by the Schatchard plot analysis and the ligand having Kd value of 10 mM or less was selected. Next, Rt (receptor density) of the labeled standard ligand was also calculated. In case that the labeled ligands are used in various ligands for examination, each Kd value was calculated by usual binding experiment.

Selected $^{125}$I labeled ligand or $^{125}$I labeled substance modified by said ligand were incubated at low temperature (4°C) for about 2 hours to bind with the receptor or protein on the cell membrane and washed with cooled isotonic buffer to remove non-specific binding fractions. The cells were incubated for constant time within the time for not to decompose the ligand by uptake with endocytosis, preferably in the range between 10 to 30 minutes at 37°C. Immediately after incubation, the cells were cooled at 4°C and the medium was sampling (designated as ① ). Subsequently, the cells were washed several times with the cooled buffer containing chelating agent or acidic buffer to dissociate the specific binding fraction (designated as ② ), and again cooled and washed several times with the isotonic buffer to dissociate the non-specific fractions. The cells after cooling was cooled at -80°C for about 30 minutes, added 0.1 N NaOH or solution containing cell membrane lytic agent such as Tween solution, and incubated at 37°C for about 2 hours to obtain the lytic cell solution (designated as ③ ).

The labeled amount in above described solutions ① and ③ was measured by radio active counter or spectrofluorometer to calculate the amount of dissociation into the extracellular fluid (① ) and amount of internalization into the cells (③ ). Simultaneously, the surface binding amount (② ) at incubation time 0 hour at 37°C was measured. In addition, it is preferable to perform experiment that excess amount of the cold ligand (100 equivalents or more) was added to the sample ligand to perform the similar uptake experiment and amount of fraction without mediating the receptor was deducted.

The amount of the accumulation dissociation ($X_{off}$) and the amount of accumulation internalization ($X_{int}$) after the constant time calculated by the above result is divided by the AUC of the surface binding amount [area under the surface binding amount at time 0 and the binding amount - time course (trapezoidal method)] to calculate $k_{off}$ and $k_{int}$. The obtained $k_{off}$ / $k_{int}$ larger than 1 is the objective candidate ligand.

Labeling of the ligand can be achieved by a method binding with drug such as the physiologically active protein and labeling the ligand or the drug with $^{125}$I, and a method labeling the introduced Tyr in the ligand with $^{125}$I or modifying with FITC, and among them, the method for labeling with binding drug is preferable for calculating $k_{off}$ and $k_{int}$.

A method for labeling the ligand or the drug bound with ligand in the present invention is preferable to use radioisotope such as the above described $^{125}$I, but the present invention is not limited such like labeling with the radioisotope and the fluorescent labeling with FITC or acridine derivatives can also be used.

Further, there may be a method for roughly calculating $k_{off}$ and $k_{int}$ quantitatively without labeling the ligand itself provided for test. This method applying for the hepatocytes is exemplified and explained concretely.

For calculation of $k_{int}$, the selected ligand is added to the cultured hepatocytes in 50 times concentration or more of the Kd value, preferably 100 times or more, for example, 1000 times of concentration, and is incubated for a time without generating degradation of the ligand caused by endocytosis, preferably 5 minutes to 10 minutes, at 37°C. Separately, the system without ligand is incubated for 0 minutes and for 5 to 10 minutes at 37°C. Then the cells are immediately cooled to 4°C, washed several times with cooled buffer containing chelating agent or acidic buffer to dissociate the specific finding fraction, further washed with cooled isotonic buffer to dissociate non-specific binding fraction. The high affinity ligand such as $^{125}$I-labeled ASOR is added to the cooled cells and allow to stand at 4°C for about 60 minutes. After dissociating the non-specific binding fraction by washing with cooled isotonic buffer, the cells are cooled at -80°C for about 30 minutes, added 0.1 N sodium hydroxide solution or a solution containing cell membrane lytic solution such as Tween solution, and incubated at 37°C for about 2 hours to obtain the cell lytic solution. Amount of labeling in this cell lytic solution is measured by radioactive counter to calculate amount of the surface remained receptor (bound with ligand or free) other than internalized by the endocytosis. The amount of the surface receptor incubated without addition of the ligand at 37°C for 0 minute is set as 100 and the amount of the surface remaining receptor after incubation of each ligand for several minutes is converted into the ratio (%) (% of control) to each control, and each $k_{int}$ is calculated by the following equation on the assumption that amount of the surface receptor up to several minutes is relatively decreased.

$$\% \text{ of control} = 100 * \exp(-k_{int} * \text{time})$$

For calculation of $k_{off}$, the selected ligand is added to the cultured hepatocytes in 50 times concentration or more of the Kd value, preferably 100 times or more, for example, 1000 times of concentration, and is incubated at 40°C for about 60 minutes. Separately, the system without ligand is incubated similarly. After washing several times with cooled isotonic buffer, the high affinity ligand such as $^{125}$I-labeled ASOR is added and incubated at 4°C for several

minutes and for about 20 to 60 minutes (at least 2 points). Thereafter immediately washing several times with cooled isotonic buffer, the cells are cooled at -80°C for about 30 minutes, added 0.1 N sodium hydroxide solution or a solution containing cell membrane lytic solution such as Tween solution, and incubated at 37°C for about 2 hours to obtain the cell lytic solution. Amount of labeling in this cell lytic solution is measured by radioactive counter to calculate amount of the dissociated ligand once bound with receptor as binding amount of the labeled material. Namely, the binding rate for the passed time (increase of the binding amount divided by its passing time) is calculated by reducing the binding amount in the incubation for the several minutes from the binding amount in the ligand non-added system or the ligand added system obtained by each incubation for several ten minutes. The binding rate without addition of the ligand is set as 100 and the binding rate of each ligand is converted into the ratio (%) (% of control) to each control. Value of the ratio (%) for each control is subtracted from 100 and each $k_{off}$ is calculated by the following equation on the assumption that the binding ligand is dissociated at one phase during the said passing time (t); the dissociation of the labeled substance is a negligible level; and the dissociated ligand does not affect to the binding of the labeled substance.

$$k_{off} = - \ln \{[100 - (\% \text{ of control})] / 100\} / t$$

The calculated value obtained by $k_{off}$ and $k_{int}$, i.e. $k_{off} / k_{int}$ lager than 1 is the objective candidate ligand.
The present invention provides a method for screening the ligand with employing guidance of the index of the ability to avoid the internalization mediated by binding with the specific receptor of the target tissue cells and the binding affinity. It also includes a device for performing said screening method and a kit for screening comprising reagents necessary for performing the same.
Important kit for screening method of the present invention is the reagent kit comprising the ligand such as $(Gal)_3$ used as the ligand in the tests hereinbefore as the standard reference. By using such standard reference, the screening method of the present invention can be performed simply within short time.
Further, the present invention includes the ligand obtained by the screening method of the present invention.
The ligand preferable for the present invention can be shown by the general formula:
A compound represented by the following formula (I):

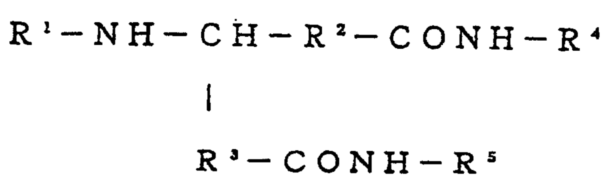

(I)

wherein $R^1$ is hydrogen atom or an organic residue having functional group which can bind with the physiologically active substance,
$R^2$ and $R^3$ are each independently single bond or straight or branched alkylene of $C_{1-5}$,
$R^4$ is a group represented by the following formula (II)

$$-R^6-(O-R^6-)n-Sug \qquad (II)$$

wherein $R^6$ is each independently straight or branched alkylene of $C_{2-5}$, Sug is sugar group, and n is an integer of 1-5, and $R^5$ is $R^4$ hereinbefore, or a group represented by the following formula (III)

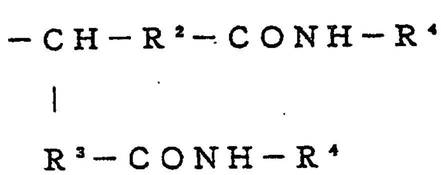

(III)

wherein $R^2$, $R^3$ and $R^4$ are same as above.

In case that $R^1$ in the general formula (I) is hydrogen atom, $R^1$-NH- group is amino group and said amino group can be directly bound with the physiologically active substance. Further, in case that $R^1$ in the general formula (I) is the organic residue having functional group which can bound with the physiologically active substance, it can be bound with the physiologically active substance through the said organic residue.

Examples of the case that $R^1$ in the general formula (I) is the organic residue having functional group which can bound with the physiologically active substance are the organic residues such as amino, carboxyl and hydroxyl which can bound with the physiologically active substance, and are not limited if no deteriorated action in the physiological activity, and are preferably the group of the following formula (VIII):

$$X\text{-}R^b\text{-}Y\text{-} \qquad \text{(VIII)}$$

wherein X represents functional group which can bind with the physiologically active substance;
$R^b$ represents linker group binding with X and Y;
Y represents single bond or carbonyl group.

Example of $R^b$ in the formula (VIII) is an inert organic group which does not inhibit physiological activity binding with X and Y, and is open chain or cyclic hydrocarbon group. More concretely, these are straight or branched alkylene, phenylene and mono- or poly-(oxyalkylene). Examples of straight or branched alkylene are straight or branched alkylene of $C_{1-15}$, preferably $C_{3-15}$, more preferably $C_{3-10}$, further preferably $C_{3-7}$, preferably straight chain alkylene, for example butylene, pentylene and hexylene. More than one carbon atom in this alkylene can be replaced by oxygen atom or nitrogen atom, and this alkylene can have the substituents in necessary.

X is a functional group, which can bind with the physiologically active substance, preferably physiologically active protein, for example amino, carboxyl, hydroxyl and phosphate. Y is a group which bind with a terminal amino group in the general formula (I) and the linker group, and may be a single bond directly bind with each other, or a functional group which can construct amino group derivatives with such as carbonyl and sulfonyl

Further, preferable example of $R^1$ is a group represented by the formula:

$$H_2N\text{-}R^a\text{-}CO\text{-}$$

wherein $R^a$ is straight or branched, preferably straight alkylene of $C_{1-15}$. Examples of the alkylene of $R^a$ are straight or branched alkylene of $C_{1-15}$, preferably $C_{3-15}$, more preferably $C_{3-10}$, further preferably $C_{3-7}$, preferably straight chain alkylene, for example butylene, pentylene and hexylene.

Examples of the alkylene of $R^2$ and $R^3$ in the general formula (I) are straight or branched alkylene of $C_{1-5}$, preferably $C_{1-3}$, preferably straight chain alkylene, for example methylene, ethylene and propylene. Such alkylene groups may have substituents. $R^2$ and $R^3$ in the general formula (I) are preferably, including neighboring carbonyl, carbon atoms and amino, acidic amino acid such as glutamic acid and aspartic acid. $R^4$ in the general formula (I) is a group represented by the formula (II) hereinbefore, and a part of - $R^6$-(O- $R^6$-)n- in the formula (II) hereinbefore is a spacer constructing between amide group and sugar group having binding ability to the receptor, and may be a group which can maintain proper distance between them, and is preferably constructed by alkyleneoxy group as shown in the formula (II) hereinbefore. Examples of alkylene ($R^6$) in such alkyleneoxy group are straight or branched alkylene of $C_{2-5}$, preferably $C_{2-3}$. Numeral n in the general formula (II) is an integer of 1-5, preferably 1-3. Example of Sug in the general formula (II) can be a group which can bind with the receptor of cells, preferably sugar group. Example of sugar group is the sugar or its derivatives having binding ability to the receptor. In case that the receptor is asialo receptor, sugar is preferably galactose or its derivatives. Examples of the sugar derivatives are sugar in which hydroxyl group is replaced by amino group and its acylated derivatives.

In the present invention, numbers of atoms of the spacer part - $R^6$-(O- $R^6$-)n- plus numbers of oxygen atoms of the aforementioned Sug group are used for expression of the ligand. For example, a number of 6 in parenthesis of $(Gal)_3(6)$ shows numbers of atoms in the spacer part is 5 and adding a number of oxygen in the sugar chain results in 6.

$R^5$ in the general formula (I) is preferably the aforementioned $R^4$ or the group represented in the formula (III). When $R^5$ is the aforementioned $R^4$, the formula has two branches, and in the present specification, this is designated as "two branched type" ligand, and when $R^5$ is the formula (III) hereinbefore, the branches in the ligand is 3, which is designated as "three branched type" ligand.

In the aforementioned preferable ligand of the present invention, the novel compound provided by the present invention for the first time is included, consequently the present invention provides useful novel chemical substance as the ligand of the present invention.

The compound represented by the following formula (IV) can be mentioned as the compound of the present invention.

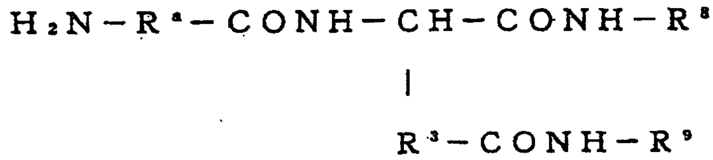

(IV)

wherein $R^a$ is a straight or a branched alkylene of $C_{1-15}$,
$R^3$ is a straight or a branched alkylene of $C_{1-5}$,
$R^8$ is a group represented by the following formula (V):

$$-R^{10}-(O-R^{10}-)n-Sug \qquad (V)$$

wherein $R^{10}$ is each independently represented by a straight or a branched alkylene of $C_{2-5}$,
Sug is a sugar group or derivatives thereof, and
n is an integer of 1-5,
and
$R^9$ is $R^8$ hereinbefore, or a group represented by the following formula (VI)

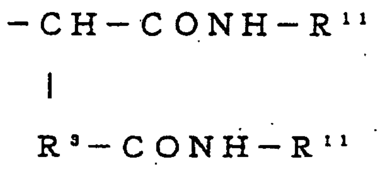

(VI)

wherein $R^3$ is a straight or a branched alkylene of $C_{1-5}$,
$R^{11}$ is represented by the following formula (VII):

$$-R^{10}-(O-R^{10}-)m-Sug \qquad (VII)$$

wherein $R^{10}$ is each independently represented by a straight or a branched alkylene of $C_{2-5}$,
Sug is a sugar group or derivatives thereof, and
m is an integer of 1-5. Proviso that m is the integer different from n in the above formula (V). Alkylene group, etc in the general formula (IV) - (VII) are the same as explained in the aforementioned general formula (I).

The ligand of the present invention can be used as the component of the therapeutic medicinal composition, and also can be used as DDS preparation by binding the said ligand with drugs such as the physiologically active protein.

Consequently, the present invention provides the physiologically active substance containing the ligand obtained by the screening method of the present invention and the medicinal composition consisting of pharmaceutically acceptable carrier.

Effective dose of the medicinal preparation of the present invention can be adjusted by the physiologically active substance as the used drug. Dosage of the active ingredient in the medicinal composition of the present invention can be smaller than the conventional medicinal composition used as the physiologically active substance per se as the medicinal composition. This is due to that the active ingredient is modified by the ligand of the present invention, accumulating the large amount of it in the target tissue and less metabolizing and degrading by cells of the target tissue. Furthermore, in the tissues other than the target tissue, the drug level can be as small as possible and the side effect caused by drug is low.

Consequently, the medicinal composition of the present invention provides the novel medicinal composition

comprising the physiologically active protein, for example cytokines such as IL-2, IL-6 and interferon, as the active ingredient, since the drugs, which have to administered in large amount by the conventional method of pharmaceutical formulation, is only administered in small dosage. Consequently, the therapeutic field using these active ingredients can be expanded.

The medicinal composition of the present invention can be an administration by the same administration method as of the drugs which is not modified by the ligand of the present invention.

The method of the present invention is explained concretely by examples, but the present invention is not limited within these examples.

Example 1

Production of $(Gal)_2(9)$

A structure of $(Gal)_2(9)$ is shown as follows.

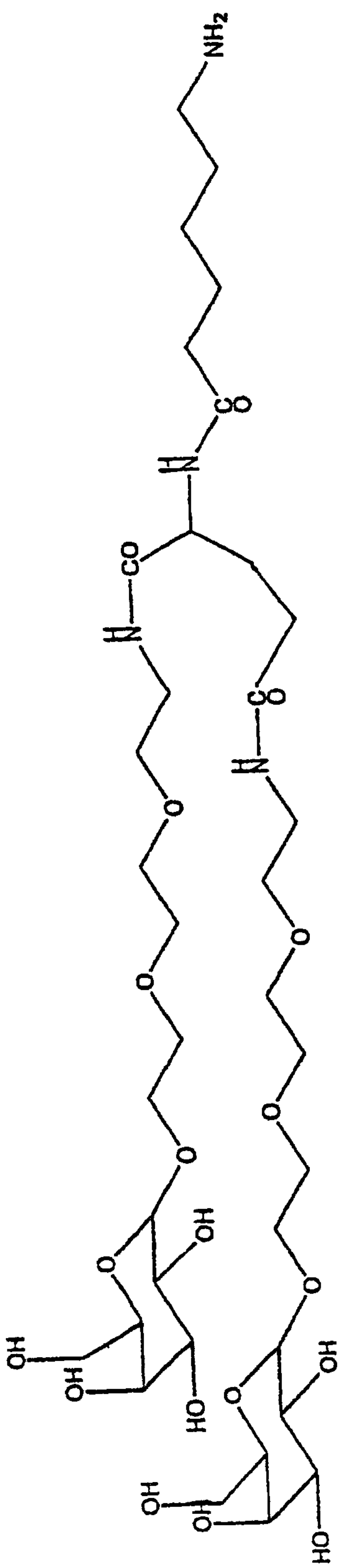

$(Gal)_2(9)$

The synthetic scheme of $(Gal)_2(9)$ is illustrated as follows.

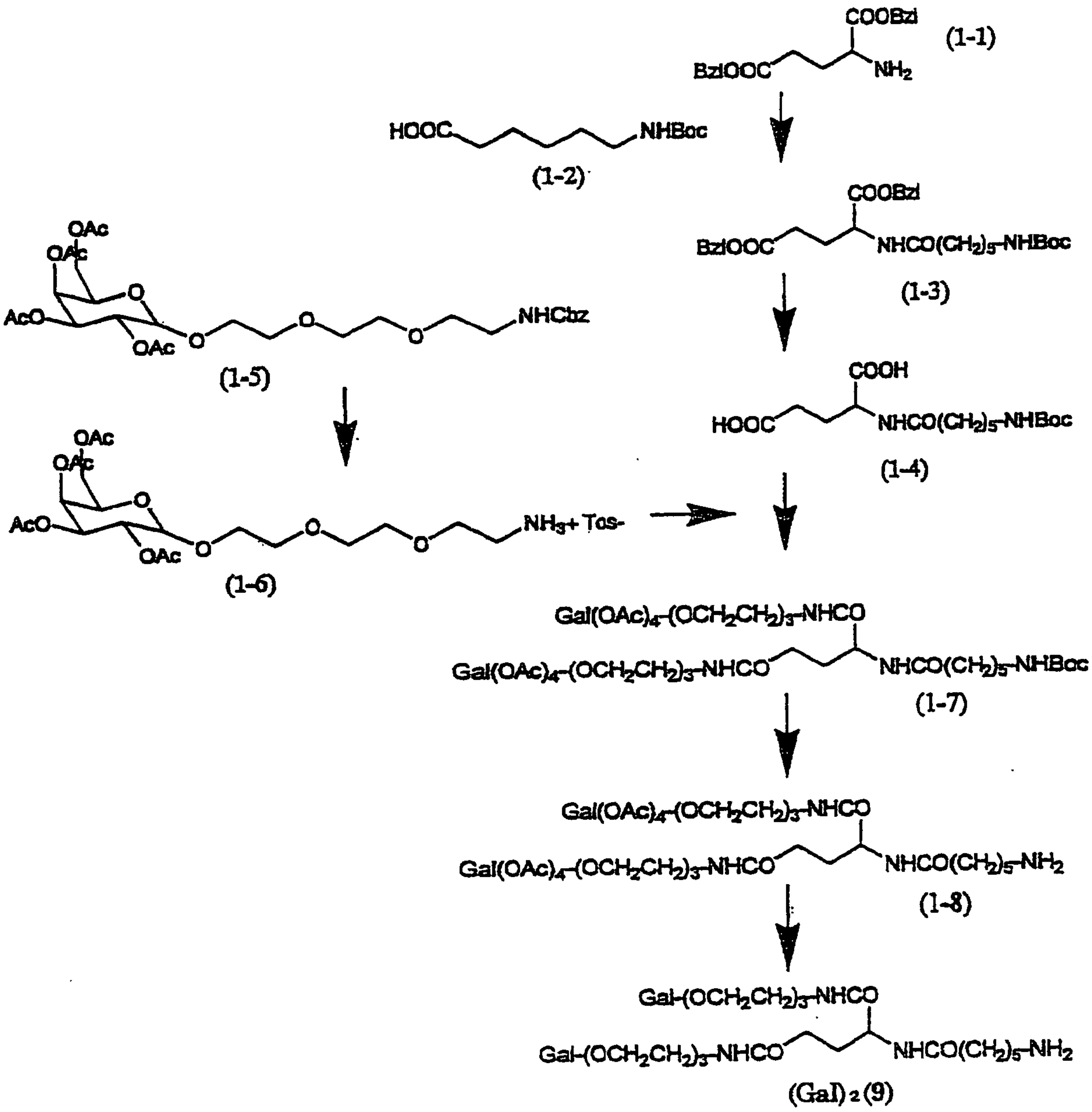

(1-1) (1-2) (1-3) (1-4) (1-5) (1-6) (1-7) (1-8) (Gal)2(9)

(1) Production of compound (1-3)

Glu(Obzl)-Obzl · Tos ($\alpha,\gamma$-dibenzylethyl tosyl salt of L-glutamic acid, compound 1-1, 31.6 g) was dissolved in dimethylformamide (200 ml), ice cooled and triethylamine (7.1 g) and N-Boc-6-aminohexanic acid (compound 1-2, 16.1 g) were added and stirred. After dissolving, HOBt (1-hydroxybenzotriazol hydrate, 9.42 g) and WSC [1-ethyl-3-(3-dimethylaminopropyl) carbodiimide hydrochloride, 13.4 g] were added gradually and stirred in the ice-bath for overnight. The mixture was diluted with ethyl acetate, washed with 10% aqueous citrate, water, 5% aqueous sodium carbonate and water, in this order, dried with magnesium sulfate, filtered and removed the solvent by distillation to obtain the objective compound (1-3) (35.6 g).

$^1$H-NMR($CDCl_3$): $\delta$

7.30-7.40(10H, m), 6.18(1H, d, J=7.5Hz), 5.16(2H, s), 5.10(2H, s), 4.63-4.71(1H, m), 4.5-4.6(1H, br(t)), 3.04-3.13(2H, m), 2.28-2.52(2H, m), 2.20-2,28(1H, m), 2.16(2H, t, J=7.5Hz), 1.96-2.08(1H, m), 1.56-1.68(2H, m), 1.41-1.50(11H, m), 1.30-1.38(2H, m)

(2) Production of compound (1-4)

The compound (1-4) (29.5 g) was dissolved in tetrahydrofuran (950 ml) and ethyl acetate (950 ml), added 5% palladium-carbon (27.1 g) and stirred under hydrogen atmosphere for overnight. The reaction mixture was filtered and removed the solvent by distillation to obtain the objective compound (1-4) (18.8 g) as solid.
$^1$H-NMR($CDCl_3$): δ
6.20-6.32(1H, br), 4.90-5.00(1H, br), 4.55-4.63(2H,m), 3.03-3.14(2H, m), 2.43-2.56(2H, m), 2.18-2.33(3H, m), 2.02-2.13(1H, m), 1.58-1.69(2H, m), 1.30-1.52(4H, m)

(3) Production of compound (1-8)

A compound (1-5) (30 g) synthesized by a method described in PCT/JP95/00298 (WO 96/06181) and p-toluene sulfonic acid (9.3 g) were dissolved in ethanol (645 ml), added 5% palladium-carbon (9.3 g) and stirred under hydrogen atmosphere for overnight. The catalyst was filtered off and the solvent was removed by distillation. The residue was dissolved in acetonitrile, azeotropically distilled, added acetonitrile and neutralized by adding N-methylmorpholine. This is designated as A-solution.
The compound (1-4) (5.2 g) was dissolved in dimethylformamide (100 ml), added N-hydroxysuccinimide (3.2 g) and dicyclohexylcarbodiimide (5.8 g) were added under ice-cooling, and the A-solution hereinabove [de-Cbz of the compound (1-5), 15.5 g] was slowly added dropwise and stirred at 4°C for overnight. Insoluble matters were filtered, washed the residue with ethyl acetate, concentrated, washed with 10% aqueous citric acid, saturated brine, 5% aqueous sodium hydrogen carbonate and saturated brine, in this order, dried by magnesium sulfate, filtered and removed the solvent by distillation. The residue was applied to the silica-gel chromatography and eluted by chloroform to chloroform : methanol : aqueous ammonium = 20/1/0.1 followed by 15/1/0.1 to obtain the compound (1-7) (4.6 g).
The thus obtained compound (1-7) (4 g) was dissolved in 50% trifluoroacetic acid-dichloromethane solution (30 ml), stirred for 1 hour, distilled off the solvent and azeotropically distilled twice with ethanol. The mixture was dissolved in methanol (24 ml), neutralized by 28% sodium methylate solution and distilled off the solvent to obtain the compound (1-8) (3.0 g).

$$\text{FAB-MS: M + Na}^+ \text{ 869.4 (MW: 846.9)}$$

(4) Production of $(Gal)_2(9)$

The compound (1-7) (3g) was dissolved in methanol (24 ml), added 28% sodium methylate solution (2.4 ml) under ice-cooling, and temperature was allowed to change to room temperature after completing the addition, and stirred for 1 hour. The reaction mixture was neutralized by cation exchange resin Dowex 50W-X4 ($H^+$, Dow Chemical Co.), removed the resin and distilled off the solvent. The residue was dissolved in 0.1% trifluoroacetic acid and purified several times by the reverse phase HPLC using Inertsil ODS-2 column (GL Science Inc.) to obtain the objective $(Gal)_2$ (9) (660 mg). The said compound was analyzed by the reverse phase HPLC using Inertsil ODS-2 column (GL Science Inc.) (A buffer: 0.1% trifluoroacetic acid, B buffer: 0.1% trifluoroacetic acid, 80% acetonitrile, 0 min.: A 95%, 30 min. : A 75%, linear gradient) to detect peak having absorption at 210 nm in retention time 19.9 minutes (purity: above 95%).
ESI-MS: $MH^+$845.54 (MW 846.9)

Example 2

Production of $(Gal)_3(6)$

The synthetic scheme of $(Gal)_3(6)$ is illustrated as follows.

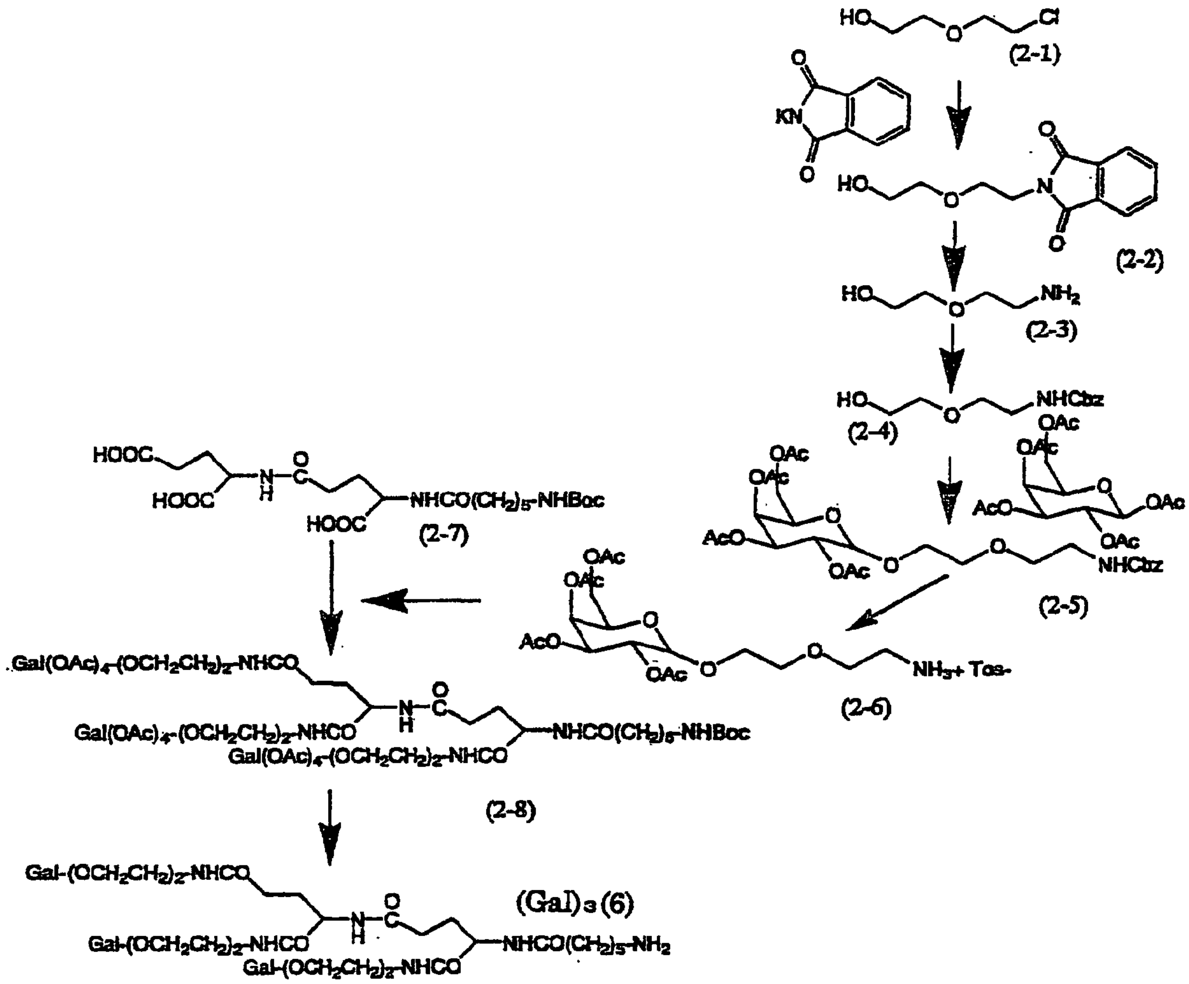

(2-1) (2-2) (2-3) (2-4) (2-5) (2-6) (2-7) (2-8) (Gal)3(6)

(1) Production of compound (2-2)

Dimethylformamide (1 L) was added to 2-chloro-ethoxyethanol [compound (2-1), 120 g] and potassium phthalimide (192 g), stirred at 100°C for overnight. After cooling the reaction mixture, chloroform (300 ml) was added, filtered and concentrated the filtrate to obtain the objective compound (2-2) (255 g).
$^1$H-NMR($CDCl_3$): δ
7.59-7.90(4H, m), 3.83-3.91(2H, m), 3.57-3.75(6H, m), 2.75-2.97(1H, br)

(2) Production of compound (2-3)

The compound (2-2) (255 g) was dissolved in ethanol (4.4 L) and added 80% aqueous hydrazine (77.9 g) with stirring. The reaction mixture was refluxed for 2 hours, filtered the cooled reaction mixture and removed the solvent. Dichloromethane (800 ml) was added to the residue and concentrated the extract to obtain the objective compound (2-3) (120 g).
$^1$H-NMR($CDCl_3$): δ
4.40-4.90(3H, br), 3.30-3.80(6H, m), 2.70-3.00(2H, m)

(3) Production of compound (2-4)

The compound (2-3) (103 g) was dissolved in water (1.15 L), added 5 times alternately sodium hydrogen carbonate (21.4 g) and benzyl chloroformate (40.1 g) dissolved in toluene (86 ml) with stirring under ice-cooling, and the reaction mixture was stirred at room temperature for overnight. After extraction by adding ethyl acetate, the mixture was dried with magnesium sulfate, filtered and removed the solvent by distillation. The residue was applied to the silica-gel chromatography, eluted with hexane/ethyl acetate = 1/1 to ethyl acetate only and distilled off the solvent of

the main fraction to obtain the compound (2-4) (110 g).
$^1$H-NMR($CDCl_3$): δ
7.25-7.40(5H, m), 5.30-5.55(1H, br) , 5.10(2H, s) , 3.60-3.70(2H, m), 3.45-3.60(4H, m), 3.25-3.45(2H, m), 2.60-2.75 (1H, br(t))

(4) Production of compound (2-5)

β-D-galactopyranose pentaacetate (75 g) and the compound (2-4) was dissolved in dichloromethane (1.5 L), added boron trifluoride-diethyl ether complex (54.5 g) and stirred for overnight. The reaction mixture was diluted with dichloromethane, washed with saturated aqueous sodium hydrogen carbonate and dried the organic layer with magnesium sulfate. The mixture was filtered and distilled off the solvent. The residue was applied on the silica-gel chromatography, eluted with hexane/ethyl acetate = 1/1, and distilled off the solvent of the main fraction to obtain the compound (2-5) (65 g).
$^1$H-NMR($CDCl_3$): δ
7.25-7.40(5H, m), 5.35-5.40 (1H, m), 5.30-5.35 (1H,br), 5.21(1H, dd, J=8Hz, 10Hz), 5.11(2H, s), 5.01(1H, dd, J=3.2Hz, 10.4Hz), 4.54(1H, d), 4.09-4.20(2H, m), 3.85-3.95(2H, m), 3.65-3.75(1H, m), 3.50-3.65(4H, m), 3.35-3.40(2H, m), 2.13 (3H, s), 2.04(3H, s), 2.03(3H, s), 1.98(3H, s)

(5) Production of compound (2-8)

The compound (2-5) (40 g) and p-toluene sulfonic acid (13.4 g) was dissolved in ethanol (860 ml), added 5% palladium-carbon (12.4 g), and stirred under hydrogen atmosphere for overnight. The catalyst was filtered and the solvent was distilled off. The residue was dissolved in acetonitrile and azeotropically distilled twice, added acetonitrile and neutralized with N-methylmorpholine. This is designated as A-solution.

A compound (2-7) (4 g) synthesized by a method described in PCT/JP95/00298 (WO 96/06181) was dissolved in dimethylformamide (20 ml), added N-hydroxysuccinimide (2.84 g) and dicyclohexylcarbodiimide (5.1 g) were added under ice-cooling, and the A-solution hereinabove [compound (2-7), 16.0 g] was slowly added dropwise and stirred at 4°C for overnight. Insoluble matters were filtered, washed the residue with ethyl acetate, concentrated, washed with 10% aqueous citric acid, saturated brine, 5% aqueous sodium hydrogen carbonate and saturated brine, in this order, dried with magnesium sulfate, filtered and removed the solvent by distillation. The residue was applied to the silica-gel chromatography and eluted by chloroform to chloroform/methanol/aqueous ammonium = 25/1/0.1 followed by 20/1/0.1 to obtain the compound (2-8) (3.2 g).
ESI-MS: $MH^+$ 1741.6 (MW 1741.8)

(6) Production of $(Gal)_3(6)$

The compound (2-8) (3 g) was dissolved in 50% trifluoroacetic acid-dichloromethane solution (15 ml), stirred for 1 hour, distilled off the solvent and azeotropically distilled twice with ethanol. The mixture was dissolved in methanol (24 ml), neutralized by 28% sodium methylate solution, added said 28% sodium methylate solution (1.2 ml) under ice-cooling, and temperature was allowed to change to room temperature after completing the addition, and stirred for 1 hour. The reaction mixture was neutralized by cation exchange resin Dowex 50W-X4 ($H^+$, Dow Chemical Co.), removed the resin and distilled off the solvent. The residue was dissolved in 0.1% trifluoroacetic acid and purified several times by the reverse phase HPLC using Inertsil ODS-2 column (GL Science Inc.) to obtain the compound (2-9) (660 mg). The said compound was analyzed by the reverse phase HPLC using Inertsil ODS-2 column (GL Science Inc.) (A buffer: 0.1% trifluoroacetic acid, B buffer: 0.1% trifluoroacetic acid, 80% acetonitrile, 0 min.: A 95%, 30 min.: A 75%, linear gradient) to detect peak having absorption at 210 nm in retention time 17.4 minutes (purity: above 95%).
ESI-MS: $MH^+$1137.7 (MW 1137.2)

Example 3

Preparation of $(Gal)_3$-IL-2

Preparation, isolation and purification of $(Gal)_3$-IL-2 were performed by the same method as in PCT/JP97/01915. As a result, according to the analysis of SDS-PAGE, the specimen with high purity showing 1 band (1.52 mg, recovery 15.2%) was obtained. IL-2 biological activity of $(Gal)_3$-IL-2 measured by a method of Gills et al. [J. Immunol., 120, 2027 (1978)] was $9.21 \times 10^6$ unit/mg (specific activity to unmodified IL-2 was 107%) and showed to maintain biological activity of IL-2.

Example 4

Preparation of $(GalNAc)_3$-IL-2

Preparation, isolation and purification of $(GalNAc)_3$-IL-2 were performed by the same method as in PCT/JP97/01915. As a result, according to the analysis of SDS-PAGE, the specimen with high purity showing 1 band (1.51 mg, recovery 20%) was obtained. IL-2 biological activity of $(GalNAc)_3$-IL-2 measured by a method of Gills et al. [J. Immunol., 120, 2027 (1978)] was $8.45 \times 10^6$ unit/mg (specific activity to unmodified IL-2 was 98%) and showed to maintain biological activity of IL-2.

Example 5

Preparation of $(Gal)_3$-IFNα

Preparation, isolation and purification of $(Gal)_3$-IFNα were performed by the same method as in PCT/JP97/01915. As a result, according to the analysis of SDS-PAGE, the specimen with high purity showing 1 band 31.3µg (recovery 31%) was obtained. IFNα biological activity of $(Gal)_3$-IFNα measured by a method of Watanabe, et al. (Lymphokines and Interferons: A Practical Approach, IRL Press, Oxford, 1987) was $93 \times 10^8$ IU/mg (specific activity to unmodified IFNá was 104%) and showed to maintain biological activity of IFNα.

Prepared $(Gal)_3$-IFNα and unmodified IFNα was subjected to reductive carboxymethylation and trypsinization, then the peptide map of the reaction mixture was compared by LC/MS and the disappearance of the only peaks derived from amino acid sequence including Gln102 was in $(Gal)_3$-IFNα was observed. As a result, it was confirmed that $(Gal)_3$ was site-specifically bound with a position at Gln102 of IFNα (human IFNα-2b) by an action of transglutaminase of microbial origin.

Example 6

Preparation of $(Gal)_3(6)$-IFNα

Preparation, isolation and purification of $(Gal)_3(6)$-IFN α were performed by the same method as in PCT/JP97/01915. As a result, according to the analysis of SDS-PAGE, the specimen with high purity showing 1 band 68.5µg (recovery 34%) was obtained.

Example 7

Preparation of $(Gal)_3(9)$-IFNα

Preparation, isolation and purification of $(Gal)_3(9)$-IFN α were performed by the same method as in PCT/JP97/01915. As a result, according to the analysis of SDS-PAGE, the specimen with high purity showing 1 band 58.2µg (recovery 29%) was obtained.

Example 8

Binding inhibition experiment

Binding inhibition experiments of ASOR to mouse liver cells using each ligand were performed according to a method of Biessen, et al. [Biessen, E.A.L. et al., J. Biol. Chem., 271(45): 28024-28030 (1996)].

The liver parenchymal cells (in 4 ml Falcon tube, 0.6 - $1.0 \times 10^6$/0.5 ml in ice bath) was added to D-PBS containing 2% BSA. Inhibitor 0.25 ml prepared for various concentration with D-PBS containing 2% BSA was added thereto. $^{125}$I-ASOR 0.25 ml (22 nM) in D-PBS containing 2% BSA was added therein and stirred gently at 4°C for 2 hours.

The mixture was centrifuged at 50G for 1 minute, discarded the supernatant solution, added D-PBS containing 2% BSA and stirred, then again centrifuged at 50G for 1 minute, and discarded the supernatant medium. This procedure was repeated again and the medium was measured by γ-counter.

Thereafter, 0.1 N NaOH containing 10% SDS 1 ml was added thereto, and the protein concentration was measured by Lowry method using the protein assay kit (Biorad Inc.).

In addition, the binding inhibition experiment of 1 types of ligand for binding with ASOR in mouse isolated liver cells was performed according to the method described above.

As a result, depending on the increase in the added concentration of $(GalNAc)_3$ and $(Gal)_3$, $^{123}$I-ASOR was dissociated with almost monophasic manner. Data of dissociation obtained by three repeated experiments were analyzed using non-linear single site model to calculate the binding inhibition constant (Ki). Result is shown in Table 1 hereinbefore.

Large differences were observed between two types of the ligand. Ki value of $(GalNAc)_3$ was about 230 times (1.7 μM) larger than that of ASOR, whereas that of $(Gal)_3$ was about 50000 times (350 μM) larger than that. The binding inhibition constant (Ki) is thought to be the binding dissociation constant (Kd), and Kd value of each ligand was calculated from these results.

Example 9

Binding inhibition experiment 2

In the binding inhibition experiment according to the method of Biessen, et al. performed in example 8, a concentration of added $^{125}$I-ASOR was 22 nM. Kd value of ASOR is approximately several nM and the concentration of added ASOR is higher that the actual Kd value, consequently, in the experiment in example 8, the binding inhibition by each ligand is difficult to generate and the Kd value is estimated too high. In order to evaluate newly synthesized ligand[$(Gal)_3$(6) and $(Gal)_2$(9)], calculation of more exact Ki value is necessary. Then, the concentration of adding $^{125}$I-ASOR was set sufficiently lowered than its Kd value as well as improving the experimental condition and the binding inhibition experiment was performed again.

Mouse primary cultured hepatocytes, which were cultured for overnight on the 12 well plate, isolated by collagenase perfusion described in examples of Berry, et al., were washed twice with D-MEM (pH 7.4) (each 2 ml) containing D-PBS, 0.1% BSA and 20 mM Hepes, added said D-MEM medium (1 ml), and preincubated at 37°C for 60 minutes. The cooled D-PBS (pH 5.0) containing 10 mM EDTA was added to the cells, allowed to stand under ice-cooling for 4 minutes, and washed twice with cooled D-PBS (2 ml). The plate was set on the ice bath, and a mixture 0.4 ml simultaneously containing 0.6 nM $^{125}$I-ASOR and each cold ligand adjusted to various concentration in said D-MEM medium was added to the cells in each well, and allowed to leave at 4°C for 60 minutes. The cells were washed, on the ice bath, 4 times with cooled D-PBS (1 ml), allowed to leave at -80°C for more than 30 minutes, added 0.1 N aqueous sodium hydroxide (0.5 ml), incubated at 37°C for more than 1 hour, and measured the cell lytic solution by using γ-counter to calculate amount of bound $^{125}$I-ASOR on the surface of cells.

Depending on increase in added concentrations in each ligand, $^{125}$I-ASOR was dissociated in monophasic manner. The binding inhibition curve under low temperature from the result of twice [once for $(GalNAc)_3$] experiments was used to perform Fitting in the model on the assumption that the receptor was inhibited in one site and ASOR was not received binding inhibition by ligand other than the receptor. As a result, Ki value of $(GalNAc)_3$, $(Gal)_3$, $(Gal)_3$(6) and $(Gal)_3$(9) was 0.00333 μM, 0.157 μM, 0.369μM and 1.05 μM, respectively. As a result, it was indicated that the binding affinity of the newly synthesized ligand to the asialoglycoprotein receptor was about one over several of $(Gal)_3$ ligand. Since each Kd value was lower than 10 mM, theoretically, it was estimated that targeting efficiency for asialoglycoprotein was maintained.

Experiment 10

Antitumor effect of drugs

S908 tumor cells, $7.5 \times 10^4$ cells, were inoculated in B10D2 mice, female, 6 weeks old, (7 mice in a group, 7 groups in total 49mice) to develop tumor. From day 7 after tumor cell inoculation, drug was administered twice a day, for 5 days, total 10 times. Group 1 was a control group and physiological saline was administered. For the group 2, IL-2, $1.0 \times 10^4$ units (150 μ l) in one administration were administered (low dose group). For the group 3, IL-2, $4.0 \times 10^4$ units (150 μl) in one administration were administered (high dose group). For the group 4, $(Gal)_3$-IL-2, $1.0 \times 10^4$ units (150 μl) in one administration (low dose group). For the group 5, $(Gal)_3$-IL-2, $4.0 \times 10^4$ units (150 μl) in one administration were administered (high dose group). For the group 6, $(GalNAc)_3$-IL-2, $1.0 \times 10^4$ units (150 ìl) in one administration were administered (low dose group). For the group 7, $(GalNAc)_3$-IL-2, $4.0 \times 10^4$ units (150 μl) in one administration were administered (high dose group).

After 28 days of tumor cell inoculation, animals were dissected and cure and size of tumors were observed.

The statistical analyses of drugs were performed by multiple tests. Antitumor effects were analyzed by one parameter ANOVA (Dunnett's-t-test). Case numbers and mortal numbers were analyzed by Fisher's protected LSD. Results are shown in Table 2 before.

On antitumor effect, $(Gal)_3$-IL-2 groups exhibited significant effects in the high and low administration groups as compared with the control group. In IL-2 groups, significant effect was observed only in the high dose group as

compared with the control group. $(GalNAc)_3$-IL-2 groups exhibited no significant effects in both groups. On the curative case numbers, only $(Gal)_3$-IL-2 groups exhibited significant effects in the high and low administration groups as compared with the control, IL-2 and $(GalNAc)_3$-IL-2 groups. On numbers of mortal cases, only in low dose groups of $(Gal)_3$-IL-2, IL-2 and $(GalNAc)_3$-IL-2, significant effect was observed as compared with the control group. In total, it was indicated that antitumor effects of three samples are: $(Gal)_3$-IL-2 > IL-2 > $(GalNAc)_3$-IL-2, in this order.

Example 11

In vivo kinetics of ligand modified IL-2

$^{125}$I-labeled $(Gal)_3$-IL-2 ($6.9 \times 10^6$ cpm/μg) or $^{125}$I-labeled $(GalNAc)_3$-IL-2 ($2.0 \times 10^6$ cpm/μg) prepared by chloramine-T method was intravenously injected into the tail vein of mice (C57BL/6, CRJ Inc.), 6 weeks old, 50μg/kg converted to IL-2, and after constant time, blood and major organs (heart, liver, kidneys, lungs, spleen, stomach, small intestine, large intestine and muscle) were collected. Plasma was obtained after collection of blood, centrifuged at 10000 rpm for 3 minutes. Tissue levels of specimen were measured by TCA precipitation fraction for plasma and direct assay for each organ using γ-counter.

The uptake clearance of $^{125}$I-labeled $(Gal)_3$-IL-2 and $^{125}$I-labeled $(GalNAc)_3$-IL-2 (amount of uptake for 3 minutes/AUC for 3 minutes) after examining in vivo kinetics and Kp value (amount of uptake for 3 minutes/plasma level for 3 minutes) are shown in Fig. 14 and Fig. 15, respectively.

As a result, the uptake clearance in the liver (CLuptakeLi) and the liver Kp (Kpliver) of both $(Gal)_3$-IL-2 and $(GalNAc)_3$-IL-2 per body weight are higher than those in other organs. Consequently, it was found that main organ is the liver, by which the systemic disappearance and degradation for both ligands are determined.

CLuptakeLi per 1 kg of organ (ml/min/g liver) for both ligands are 0.34 for $(Gal)_3$-IL-2 and 0.50 for $(GalNAc)_3$-IL-2, showing difference in both compounds. It is indicated that the uptake efficiency of $(GalNAc)_3$-IL-2 is about 1.5 times higher than that of $(Gal)_3$-IL-2 in vivo.

Example 13

Isolation and cultivation of mouse hepatocytes

Isolation of the hepatocytes was performed according to the modified method of Berry, et al. [Berry, M. N. et al., J. Cell. Biol. 43: 506-520 (1969)]. Namely, cell suspensions after collagenase perfusion of mouse (C57BL/6, CRJ Inc.), male, 6-10 weeks old, were filtered, centrifuged and allowed to leave at 4°C in Falcon tube without plating. Cell suspensions of the similarly treated 2 - 3 mice were combined, centrifuged at 50g for 1 minutes for 3 - 4 times to obtain mouse liver parenchymal cells, viability more than 90%, $6.0 \times 10^7$ - $1.1 \times 10^8$ cells.

Cells were suspended in D-MEM (Cat. No. 31600-091, Gibco Inc.) containing streptomycin (100 μg/ml), penicillin (100 U/ml), Hepes (20 mM) and FBS (10%, Cat. No. 26140-0791, Gibco Inc.) ($3.0 \times 10^5$ /ml), added into 12 well collagenase coated microplate (Code No. 4515-010, IwakiCo.) each 1 ml, and incubated at 37°C, 5% $CO_2$, for overnight to use for uptake experiment.

Example 14

Uptake experiment into mouse hepatocytes by pulse-chase method

Experiment on uptake in mouse hepatocytes by pulse-chase method was performed with reference to a method by Kuwabara, et al. [Kuwabara, T. et al. Am. J. Physiol. 193: 73-84 (1996)]. $^{125}$I-labeled compound was used the similar samples as used in example 11.

Mouse hepatocytes obtained in example 12 were added into 12 well plate. D-PBS (pH 7.4) 12 ml was added, incubated at 4°C and washed twice with D-PBS. Further, the cells were washed twice with ice-cooled D-MEM containing 0.1% BSA and 25 mM Hepes (pH 7.4).

D-MEM solution containing ice cooled $^{125}$I-labeled $(Gal)_3$-IL-2 or $^{125}$I-labeled $(GalNAc)_3$-IL-2, 200 μl (0.65 μg/ml as IL-2; 42 nM), 0.1% BSA and 25 mM Hepes (pH 7.4) was added thereto. Further, D-MEM containing ice-cooled 0.1% BSA and 25 mM Hepes (pH 7.4), 200μl, was added in the presence of or without presence of 200 mM GalNAc. These were allowed to leave at 4°C for 2 hours.

Then, the cells were washed 4 times with ice-cooled D-MEM, 500μl, containing 0.1% BSA and 25 mM Hepes (pH 7.4). D-MEM, 500μl, containing 0.1% BSA and 25 mM Hepes (pH 7.4) were added to adjust temperature to 37°C, and maintained at 37°C, for 0 minute, 1 minute, 2 minutes, 3.5 minutes, 5 minutes, 7 minutes, 10 minutes, 20 minutes or 30 minutes, respectively, then each sample was immediately cooled to 4°C by ice bath. The thus obtained each

sample was divided into two parts, and one part of them was treated as follows.

The one of them was further divided into each 150 µl, and another part of 150µl was used for quantitation of amount of $^{125}$I. This quantitative value was designated as ⑥ .

To the other 150 µl, 20% TCA, 150 µl, was added, allowed to leave for 10 minutes, centrifuged at 3500 rpm for 10 minutes and separated the precipitate. Amount of $^{125}$I in the solution was quantitatively measured. This quantitative value was designated as ⑦ .

The remained solution cooled at 4°C by ice bath was washed twice with ice-cooled D-MEM, 500 µl, containing 0.1% BSA and 25 mM Hepes (pH 7.4), added ice-cooled D-PBS (pH 7.4), 1 ml, allowed to stand at 4°C for 10 minutes, and added ice-cooled D-PBS, 1 ml, containing 20 mM EGTA (pH 7.4), allowed to stand at 4°C for 10 minutes. Amount of $^{125}$I was quantitatively measured. This quantitative value was designated as ② .

Further, ice-cooled D-PBS (pH 7.4), 1 ml, was added. Amount of $^{125}$I was quantitatively measured. This quantitative value was designated as ③ .

Subsequently, the mixture was allow to stand at -80°C for more than 30 minutes, added 0.1 N-NaOH, 0.5 ml, and allowed to stand at 37°C for 2 hours. Amount of $^{125}$I was quantitatively measured. This quantitative value was designated as ④ .

Further, 0.1 n-NaOH, 0.25 ml, was added and washed. Amount of $^{125}$I was quantitatively measured. This quantitative value was designated as ⑤ .

Various values were calculated by the following equations with reference to the above quantitative data.

[Amount of internalized substance] =④+⑤

[Amount of dissociated substance] = (⑥-⑦*2)e*500/150

[Amount of degraded substance] =⑦*500/150

[Amount of substance bound to cell surface] =②+③

The total protein in the cells was determined as follows. Samples in 3 wells in the plate after overnight cultivation, which were not used for uptake experiment, were quantitatively measured by Lowry method (Bio Rad Inc.) and mean values were calculated.

Differential equation used in this test is as follows.

$$dLRs/dt = k_{on}Rt{*}L - (k_{off} + k_{int}){*}\ LRs$$

$$dLRi/dt = k_{int}{*}LRs - k_{deg}{*}Lri$$

$$dX_{deg}/dt = k_{deg}{*}LR$$

Experiments were conducted using labeled compound as tracer, and numbers of receptor (Rt) were fixed to constant. Since the decrease of the concentration of the ligand-IL-2 conjugate (L) was very small, L is fixed to constant. In total, three repeated experiments were performed, and analysis was performed each experiment and the kinetic parameters were calculated.

Amounts of the obtained radioactivity were all divided by concentration of added solution (cpm/µl) and amount of quantitated total protein in one well in each experiment (mg/well) to convert distribution volume (µl/mg cell protein). Concentration of added solution was corrected by multiplying the intact rate calculated by TCA precipitation in each experiment. Data were all determined by subtracting a value added with GalNAc from a value of GalNAc non-addition to prepare data originated from specific uptake mediated by ASGP-R: and binding amount to the cell surface in each time (Bind); amount of uptake (Inter); amount of degradation (Deg); and amount of dissociation (Dis) were calculated as mean value.

Integral plots of uptake (µl /mg) and degradation (µl /mg) for the binding $AUC_{0-t}$ (µl*min/mg) were optimized by means of the least-square method (Multi, weighting 2) [K. Yamaoka, et al., J. Pharmacobio-Dyn. 4: 879-885 (1981)] to obtain the integration rate constant and dissociation rate constant ($k_{int}$ and $k_{off}$: $min^{-1}$).

Example 15

Uptake experiment to mouse hepatocytes by continuous incubation

The mouse liver cells obtained in example 13 were added into 12 well plate, and D-PBS (pH 7.4), 1 ml, was added thereto, incubated at 4°C, and washed twice with D-PBS.

D-MEM solution containing ice cooled $^{125}$I-labeled $(Gal)_3$-IL-2 or $^{125}$I-labeled $(GalNAc)_3$-IL-2, 200 µl (0.65 µg/ml as IL-2; 42 nM), 0.1% BSA and 25 mM Hepes (pH 7.4) was added thereto. Further, D-MEM containing ice-cooled 0.1% BSA and 25 mM Hepes (pH 7.4), 200µl, was added in the presence of or without presence of 200 mM GalNAc.

This was maintained at 37°C, for 1 minute, 5 minutes, 10 minutes, 20 minutes, 30 minutes, 60 minutes, 120 minutes or 240 minutes, then after incubation, each sample was immediately cooled to 4°C by ice bath. The thus obtained each sample was divided into two parts, and one part of them was treated as follows.

The one of them was further divided into each 150 µl, and another part of 150µl was used for quantitation of amount of $^{125}$I. This quantitative value was designated as ⑥ .

To the other 150 ml, 20% TCA, 150 µl, was added, allowed to stand for 10 minutes, centrifuged at 3500 rpm for 10 minutes and separated the precipitate. Amount of $^{125}$I in the solution was quantitatively measured. This quantitative value was designated as ⑦ .

The ice-cooled D-PBS (pH 7.4), 1 ml, was added to the remained solution cooled at 4°C by ice bath, allowed to stand at 4°C for 10 minutes, and added ice-cooled D-PBS, 1 ml, containing 20 mM EGTA (pH 7.4), allowed to stand at 4°C for 10 minutes. Amount of $^{125}$I was quantitatively measured. This quantitative value was designated as ② .

Further, ice-cooled D-PBS (pH 7.4), 1 ml, was added. Amount of $^{125}$I was quantitatively measured. This quantitative value was designated as ③ .

Subsequently, the mixture was allow to stand at -80°C for more than 30 minutes, added 0.1 N-NaOH, 0.5 ml, and allowed to stand at 37°C for 2 hours. Amount of $^{125}$I was quantitatively measured. This quantitative value was designated as ④ .

Further, 0.1 N-NaOH, 0.25 ml, was added and washed. Amount of $^{125}$I was quantitatively measured. This quantitative value was designated as ⑤ .

Various values were calculated by the following equations with reference to the above quantitative data.

[Amount of integrated substance] =④+⑤

[Amount of degraded substance] =⑦*500/150

[Amount of substance bound to cell surface] =②+③

The Total protein in the cells was determined as follows. Samples in 3 wells in the plate after overnight cultivation, which were not used for uptake experiment, were quantitatively measured by Lowry method (Bio Rad Inc.) and mean values were calculated.

Analysis of data obtained in the continuous incubation of three repeated experiments in total was performed in each experiment (n = 3) and the kinetic parameters were calculated.

Every amounts of the obtained radioactivity were, the same as in the previous example 4, divided by a concentration of added solution (cpm/µl) and an amount of quantitated total protein in one well in each experiment (mg/well) to convert the distribution volume (µl/mg cell protein). Concentration of added solution was corrected by multiplying the intact rate calculated by TCA precipitation in each experiment. Data were all determined by subtracting a value added with GalNAc from a value of GalNAc non-addition to prepare data originated from specific uptake mediated by ASGP-R: and binding amount to the cell surface in each time (Bind); amount of uptake (Inter); and amount of degradation (Deg) were calculated as mean value. The obtained data were optimized by using Napp (Numeric Analysis Program for Pharmakokinetics) [H. Hisaka and Y. Sugiyama, J. Pharmacokine. Biopharm. (1999), 26 in press]. The optimization was performed by that ratio of $k_{int}$ and $k_{off}$ was fixed for $(Gal)_3$-IL-2 and $(GalNAc)_3$-IL-2 and applied by combining the non-linear least-squares method and Runge Kutta Gill method to caluculate $k_{int}$ ($min^{-1}$), $k_{off}$ ($min^{-1}$), $K_{deg}$ (dissociation rate constant, $min^{-1}$) and $K_{on}$Rt (binding clearance: $K_{on}$*Rt, ìl/min/mg).

Example 16

Simulation of pharmacokinetics

In order to explain differences in drug effects of $(Gal)_3$-IL-2, $(GalNAc)_3$-IL-2 and IL-2, the pharmacokinetics model based on the physiological knowledge was constructed.

Parameters used in this simulation were the pharmacokinetics parameter, kinetics parameter and physiological parameter obtained by the experiments in examples 4 and 5. Since the main organ for distribution and disappearance of the ligand modified IL-2 is the liver, the simple compartment physiological pharmacokinetic model consisting of extracellular fluid and the blood circulation system of the liver was used. Simulation was performed using Stella [Bogen et al., Science, 246: 138-142 (1989)] to estimate the calculation of in vivo pharmacokinetics.

An experimentally observed non-specific disappearance clearance in the liver was added as a model for IL-2, and binding clearance for ASGP-R was set as 0. The rate equation for the ligand-IL-2 hybrid concentration in each fraction is shown in the following.

(In the plasma fraction)

$$dCb/dt = (-Q_H{*}Cb + Qh{*}Ce)/Vb$$

(In liver extracellular fraction)

$$dCe/de = (Q_H{*}Cb - k_{on}RT{*}Ce{*}F + k_{off}{*}Xs - Q_H{*}Ce)/Ve$$

(Liver cell surface binding fraction)

$$dXs/dt = k_{on}Pt{*}Ce{*}F\ (k_{off} + k_{int}){*}Xs$$

(Liver intracellular fraction)

$$dXi/dt = k_{int}{*}Xs - k_{deg}{*}Xi$$

(Decomposed fraction)

$$dX_{deg}/dt = k_{deg}{*}Xi$$

(Receptor occupancy ratio)

$$Receptor\ Occupancy = Ce/(Kd + Ce)$$

Among the parameters used and described in Table 3 earlier, as for (a), values obtained from data from the

uptake experiment of $(Gal)_3$-IL-2, and (b) on uptake clearance by organs except for the liver, the values for renal clearance of $(Gal)_3$-IL-2 were used. The liver clearance of IL-2 ($CL_{NSP}$) in (c) was calculated as the ratio of the degradation rate from non-specific uptake of IL-2 and the degradation rate of $(Gal)_3$-IL-2 obtained in example 13 is set as equal to a ratio of each of the liver intrinsic clearance values. The intrinsic clearance of $(Gal)_3$-IL-2 herein is represented by the equation:

$$CL_{int}\ h\ (Gal) = F{*}k_{on}Rt\ (Gal) * k_{int}\ (Gal) / [k_{off}\ (Gal) + k_{int}\ (Gal)]$$

(CLint h: the liver intrinsic clearance, F: conversion constant)

Among the parameters used, the liver extracellular volume Ve was calculated according to the report by Miyauchi et al. that its volume is approximately 20% of organ weight [S. Miyauchi, et al. J. Pharmacokin. Biopharm. 15: 25-38 (1987)].

$Q_H$ (the liver plasma flow rate) and F [conversion constant of $k_{on}Rt$ (binding clearance) obtained by the in vitro to the in vivo model] were determined as follows. Namely, the respective liver uptake clearance $CL_{uptake}Li$ of $(Gal)_3$-IL-2 and $(GalNAc)_3$-IL-2 was obtained by examination of in vivo pharmacokinetics after intravenous injection of $^{125}$I-labeled compound. This may be thought to reflect mainly the binding clearance ($k_{on}Rt$) calculated from the in vitro experiments. Intrinsic clearance in relation to uptake in the liver ($CL_{int}$, uptake) should be: $CL_{int}$, uptake = $k_{on}Rt \times F$. Consequently, according to Well-stirred model, the above is as follows:

$$CL_{uptake}Li = Q_H \times CL_{int},\ uptake / (Q_H + CL_{int},\ uptake)$$

With the proviso that $CL_{int}$, uptake = $F \times (k_{on}Rt$ , vitro)

With regard to this, the values of $Q_H$ and F were calculated by solving the respective simultaneous equations for $(Gal)_3$-IL-2 and $(GalNAc)_3$-IL-2.

In addition, since no decrease in the biological activities resulting from modification was observed in the in vitro biological activity assay using IL-2 dependent CTLL-2 cells, the dissociation constants of ligand-IL-2 and IL-2 to IL-2 receptor were set all at the same value (Kd = 10 pM; high affinity).

Example 17

Screening of ligand (1)

A method for screening ligand having high ability to avoid the internalization without radio labeling each ligand and without calculation of $k_{int}$ and $k_{off}$, was performed as follows. Based on the ligand $(Gal)_3$, which was already found that the ability to avoid the internalization was larger than 1 in the other examples, comparative examination on two types of the novel ligands [$(Gal)_3(6)$ and $(Gal)_3(9)$], which fulfilled conditions of the binding affinity wherein the binding dissociation constant to the asialoglycoprotein receptor was lower than 10 mM, was performed.

Concretely, mouse primary cultured hepatocytes, which were isolated by a method of Berry et al. collagenase perfusion method shown in example and cultured overnight on 12 well plate, was washed twice with D-MEM (pH 7.4) (each 2 ml) containing D-PBS, 0.1% BSA and 20 mM Hepes. The same D-MEM medium (1 ml) was added thereto and preincubated at 37°C for 60 minutes. Cooled D-PBS (pH 5.0) containing 10 mM EDTA was added to the cells, allowed to stand under ice cooling for 4 minutes and washed twice with cooled D-PBS (2 ml). The plate was placed on the ice bath, added each ligand in said D-MEM 0.4 ml (n = 3), in the ligand of which the concentration was 100 - 2000 times of Kd value, to the cells in each well, and incubated at 37°C for 10 minutes. After 10 minutes, the plate was placed on the ice bath, added the cooled D-PBS (pH 5.0) containing 10 mM EDTA, allowed to stand under ice cooling for 4 minutes, and washed 4 times with cooled D-PBS (1 ml). D-MEM solution (0.4 ml) of 0.6 nM $^{125}$I-ASOR was added to the cells in each well on the ice bath, and allowed to stand at 4°C for 60 minutes. On the ice bath, they were washed 4 times with cooled D-PBS (1 ml), allowed to stand at -80°C for more than 30 minutes, added 0.1 N sodium hydroxide (0.5 ml), and incubated at 37°C formore than 1 hour. Amount of receptor remained on the surface (ligand bound or free) other than the internalization by endocytosis was calculated by measuring the cell lysis with ã-counter (mean of 3 repeats).

Results are shown in Table 6.

Table 6,

| Bound counts of $^{125}$I-ASOR to mouse liver cells in the screening experiment for each ligand | |
|---|---|
| Ligand | Bound (cpm) |
| - | 2867 |
| $(Gal)_3$ | 1784 |
| $(Gal)_2(9)$ | 1670 |
| $(Gal)_3(6)$ | 2015 |

Result indicates that in any of'ligands, binding amount of ASOR is low as compared with the ligand non-addition system, and the integration of the ligand was indicated. Among them, $(Gal)_3(6)$ showed higher counts compared to the standard $(Gal)_3$, and possibility of the ligand to have higher ability to avoid the integration was suggested.

Example 18

Screening of ligand (2)

A method for screening ligand having high ability to avoid the internalization without radio labeling each ligand to obtain $k_{int}$ and $k_{off}$ and to calculate simply the ability to avoid the internalization (= $k_{int}$ / $k_{off}$) was performed as follows. Based on the ligand $(Gal)_3$, which was already found that the ability to avoid the internalization was larger than 1 in the other examples, comparative examination on three types of the novel ligands [$(Gal)_3(6)$ and $(Gal)_3(9)$; and $(GalMAc)_3$], which fulfilled conditions of the binding affinity wherein the binding dissociation constant to the asialoglycoprotein receptor was lower than 10 mM, was performed.

Concretely, mouse primary cultured hepatocytes, which were isolated by the method of Berry et al. collagenase perfusion method shown in example and cultured overnight on 12 well plate, was washed twice with D-MEM (pH 7.4) (each 2 ml) containing D-PBS, 0.1% BSA and 20 mM Hepes. The same D-MEM medium (1 ml) was added thereto and preincubated at 37°C for 60 minutes. Cooled D-PBS (pH 5.0) containing 10 mM EDTA was added to the cells, allowed to stand under ice cooling for 4 minutes and washed twice with cooled D-PBS (2 ml). Using the thus prepared plate, experiments for calculating $k_{int}$ and $k_{off}$ were performed.

With regard to the experiment for $k_{int}$, the prepared plate was placed on the ice bath, added each ligand in the D-MEM 0.4 ml (n = 3), in which the concentration of the ligand was 100 - 2000 times of Kd value, to the cells in each well, and incubated at 37°C for 10 minutes. Separately, the system without addition of the ligand was incubated for 0 minute and for 10 minutes at 37°C. After the necessary time has passed, immediately the plate was placed on the ice bath, added the cooled D-PBS (pH 5.0) containing 10 mM EDTA, allowed to stand under ice cooling for 4 minutes, and washed 4 times with cooled D-PBS (1 ml). D-MEM solution (0.4 ml) of 0.6 nM $^{125}$I-ASOR was added to the cells in each well on the ice bath, and allowed to stand at 4°C for 60 minutes. On the ice bath, they were washed 4 times with cooled D-PBS (1 ml), allowed to stand at -80°C for more than 30 minutes, added 0.1 N sodium hydroxide (0.5 ml), and incubated at 37°C for more than 1 hour. Amount of receptor remained on the surface (ligand bound or free) other than the integration by endocytosis was calculated by measuring the cell lysis with ã-counter (mean of 3 repeats). The obtained amount of ratio activity was all divided by the concentration of the added solution (cpm/µl) to convert the distribution volume (µl/well).

The distribution volume in the incubation without addition of the ligand at 37°C for 0 minute is set as 100 and the distribution volume after the incubation of each ligand for 10 minutes is converted to the percentage to each control (% of control), and each $k_{int}$ is calculated by the following equation on the assumption that amount of the surface receptor up to 10 minutes is decreased in the monophasic manner.

$$\% \text{ of control} = 100 * \exp(-k_{int} * \text{time})$$

Meanwhile, with regard to $k_{off}$, the prepared said plate was placed on the ice bath, and the said D-MEM containing the ligand with the concentration corresponding to 50 - 2000 times of the Kd value, each 0.4 ml, was added to each well (n = 3), and is incubated at 4°C for 60 minutes. Separately, with regard to the system without ligand, only the said D-MEM medium is added 0.4 ml and incubated. After passing time, the plate was immediately placed on the ice bath, and after washing 4 times with cooled D-PBS (1 ml), continuing to set the plate on the ice bath, the D-MEM solution 0.4 ml of 0.6 nM $^{125}$I-ASOR is added to the cells in each well, and allowed to stand at 4°C for 5 minutes and 20 minutes. After passing time, the plate was immediately placed on the ice bath, washed 4 times with cooled D-PBS

(1 ml), allowed to stand at -80°C for more than 30 minutes, added 0.1 N sodium hydroxide (0.5 ml), and incubated at 37°C for more than 1 hour. The cell lytic solution was measured using γ-counter to calculate amount of the dissociated cold ligand once bound with the receptor. The amount of obtained radio activity is all divided by the concentration of the added solution (cpm/μl) and converted to the distribution volume (μl/well).

With regards to the ligand non-added system and the ligand added system, the binding rate for 15 minutes is calculated by subtracting the distribution volume in the incubation for 5 minutes from the distribution volume in the incubation for 20 minutes. The binding rate without addition of the ligand is set as 100 and the binding rate of each ligand is converted into the percentage to the control (% of control). Value of % control is subtracted from 100 and the value of 100 - (% of control) is calculated, then each $k_{off}$ is calculated by the following equation on the assumption that the amount of the binding ligand is decreased with the monophasic manner during 15 minutes.

$$k_{off} = - \ln [(100 - \% \text{ of control}) / 100] / 15$$

Results are shown in Table 7.

Table 7,

| Rate constants in internalization to liver obtained in the screening experiment for each ligand | | | |
|---|---|---|---|
| Ligand | $k_{int}$ ($min^{-1}$) | $k_{off}$ ($min^{-1}$) | $k_{off}/k_{int}$ |
| $(Gal)_3$ | 0.054 | 0.0566 | 1.048 |
| $(GalNAc)_3$ | 0.0301 | 0.0132 | 0.439 |
| $(Gal)_2(9)$ | 0.0605 | 0.0237 | 0.392 |
| $(Gal)_3(6)$ | 0.0417 | 0.0677 | 1.624 |

The results, the obtained values of $k_{int}$ and $k_{off}$ of each ligand and those of $k_{off}$ /$k_{int}$ calculated based on them are shown in the Table. Results indicate that a value of the basal $k_{int}$ /$k_{off}$ is about 1, whereas only a value of $k_{off}$ /$k_{int}$ for $(Gal)_3(6)$ exceeds that value to 1.6. Consequently, it is shown that $(Gal)_3(6)$ has higher ability to avoid the internalization into the hepatocytes mediated by the asialoglycoprotein receptor than that of $(Gal)_3$. For other ligands, every values of $k_{off}$ /$k_{int}$ is less than 1, and it is shown that the ability to avoid the integration is low.

Example 19

Comparison with retainability of the modified ligand in the liver

$(Gal)_3$-IFNα, $(Gal)_3(6)$-IFNα or $(Gal)_3(9)$-IFNα, 19.9 μg (converted to IFNα)/kg, was intravenously administered to mice (C57BL/6, CRJ Co., 6W, ). After predetermined time [60 minutes, 90 minutes and 120 minutes, proviso that only 2 points of 60 minutes and 120 minutes for $(Gal)_3$-IFNα], blood and the liver were collected. Plasma was collected by the conventional method and the levels of specimens were assayed by using Human IFN α ELISA kit (ENDOGEN Inc.). D-PBS buffer containing 0.1% BSA, 9 volumes was added to the liver, homogenized and centrifuged at 3000 rpm for 10 minutes. The supernatant solution was diluted with buffer for ELISA [0.94% MEM (Minimum Essential Medium), 0.225% $NaHCO_3$ and 5% FBS (Fetal Bovine Albumin), pH 7.2] and concentration of IFNá in the liver was assayed using ELISA kit.

Results are shown in Table 8.

Table 8,

| Disappearance half-lives of ligand modified IFN α in the liver and the plasma | | |
|---|---|---|
| Ligand | Liver $t_{half}(\beta)$ (min) | Plasma $t_{half}(\beta)$ (min) |
| $(Gal)_3$-IFNα | 31 | 45 |
| $(Gal)_3(6)$-IFNα | 58 | 89 |
| $(Gal)_3(9)$-IFNα | 28 | 35 |

As a result, since concentration of each specimen is decreased linearly in the Log plot, transitions in the liver and in the plasma of the obtained each specimen are analyzed using Yamaoka, et al. Line Fitting Program, Multi Runge [Yamaoka, et al. J. Pharmacobiodyn. 6(8), 595-606] and disappearance half-life in β-phase is compared.

As a result, $(Gal)_3(6)$-IFN $\alpha$ exhibits the longest retainability in the liver, and its half-life is 58 minutes and is approximately two times longer than that of $(Gal)_3$-IFN$\alpha$ and $(Gal)_3(9)$-IFN$\alpha$. On the other hand, the longest retainability in plasma is also $(Gal)_3(6)$-IFN$\alpha$, and its half-life is 89 minutes and is approximately two times longer than that of $(Gal)_3$-IFN $\alpha$ and $(Gal)_3(9)$-IFN$\alpha$. Consequently, as a result of binding $(Gal)_3$-(6) ligand, which is the highest in the ability to avoid the internalization into hepatocytes shown by the screening experiments, with IFN$\alpha$, it is proved that the retainability in the liver can be increased as compared with $(Gal)_3$-ligand.

Industrial Applicability

The present invention provides a simple screening method for targeting DDS preparation having long term sustainability.

The ligand obtained by the screening method of the present invention can construct, by the use in combination with drugs having physiologically active action, targeting DDS preparation which is possible to increase accumulation efficiency of drugs into the targeting cells or organs, and is possible to exhibit retained effects. Namely, the drugs such as physiologically active proteins can be accumulated intensively into the target tissue, in addition the accumulated drug in the target tissue can retain for long time and the majority of administered drug activity can make use of the therapeutic objectives. Since administered drugs are mostly metabolized and decomposed in vivo, conventionally large amounts of drugs have to be administered, however according to use of the ligand of the present invention, it is sufficient to administer small amount of drugs required for exhibiting therapeutic effect, as a result, adverse drug effect can also be reduced.

Further, examples of drugs are not always limited to transfer expectedly into the outside of the cells, i.e. extracellular accumulation, but can be drugs for internalization into cells. In the latter case, since the toxicity of drug may be exhibited too high, the drugs, which are not expected to distribute into the other non-targeted organs, further which are able to internalize into the disordered cells other than the normal cells after accumulation around the target receptor, can be selected, as a result, the dosage form with small adverse effect can be prepared by the present invention.

## Claims

1. In a method for screening a targeting DDS preparation to a target tissue, said method for screening comprising a ligand having a binding affinity to a specific receptor or a protein such as antigen present on the cell membrane of a target tissue or a substance modified with said ligand with the guidance of the ability to avoid the internalization mediated by the binding to the receptor and the strength of the binding affinity.

2. The method according to claim 1 wherein the ability to avoid the internalization mediated by the binding to the specific receptor is a ratio ($k_{off}/k_{int}$) of the dissociation rate constant ($k_{off}$) and the internalization rate constant ($k_{int}$) to said receptor, and an intensity of the binding affinity is a reciprocal of the dissociation constant (Kd)[1/Kd, proviso that Kd is a ratio of $k_{off}$ and a binding rate constant ($k_{on}$)].

3. The method according to claim 2 wherein the ligand or the substance modified with said ligand having the ratio ($k_{off}/k_{int}$) of the dissociation rate constant ($k_{off}$) and the internalization rate constant ($k_{int}$) to the receptor being 1 or more is screened.

4. The method according to claim 2 or claim 3 wherein the ligand or the substance modified with said ligand having the dissociation constant (Kd) to the receptor being 10 mM or less is screened.

5. The method according to claim 4 wherein the ligand or the substance modified with said ligand having the smaller internalization rate constant ($k_{int}$) to the receptor as possible is screened.

6. The method according to claim 5 wherein the internalization rate constant ($k_{int}$) to the receptor is smaller than 0.1 $min^{-1}$.

7. The method according to any of claims 1 - 6 wherein the specific receptor present on the cell membrane of the target tissue is asialoglycoprotein receptor (ASGP-R).

8. The method according to any of claims 1 - 7 wherein the target tissue is the liver.

**9.** The method according to any of claims 1 - 8 comprising incubating the ligand or the substance modified with the ligand having the binding affinity to the specific receptor or the protein such as antigen present on the cell membrane in the presence of free cells or cultured cells which expresses said receptor, washing the ligand or the substance modified with the ligand, adding the labeled ligand to said receptor thereto, incubating at low temperature under suppressing internalization into the cells, and screening the ligand or the substance modified with the ligand in which amount of binding of the labeled ligand to the cell surface is higher as compared with the amount of binding in case of adding initially the unlabeled said affinity ligand.

**10.** The method according to claim 9 wherein the specific receptor present on the cell membrane is asialoglycoprotein receptor (ASGP-R) specifically expressed to the hepatocyte surface.

**11.** The method according to claim 9 or claim 10 wherein the ligand has the binding affinity having the dissociation constant (Kd) of 10 mM or less to the asialoglycoprotein receptor (ASGP-R) specifically expressed to the hepatocyte surface.

**12.** The method according to any of claims 1 - 8 comprising incubating the ligand or the substance modified with the ligand having the binding affinity to the specific receptor present on the cell membrane in the presence of free cells or cultured cells which expresses said receptor, washing the ligand or the substance modified with the ligand with a buffer containing chelating agent or acidic buffer, adding the labeled ligand to said receptor therein, incubating at low temperature under suppressing internalization into the cells, and screening the ligand or the substance modified with the ligand in which amount of binding of the labeled ligand to the cell surface is higher as compared with the amount of binding in case of adding the ligand having the ratio of the dissociation rate constant and the internalization rate constant after binding with the receptor being 1 or more.

**13.** The method according to claim 12 wherein the ligand having the ratio of the dissociation rate constant and the internalization rate constant after binding with the receptor being 1 or more is a three branched galactose ligand $[(Gal)_3]$.

**14.** The method according to claim 12 or claim 13 wherein the specific receptor present on the cell membrane is asialoglycoprotein receptor (ASGP-R) specifically expressed to the hepatocyte surface.

**15.** The method according to any of claims 12 - 14 wherein the ligand has the binding affinity having the dissociation constant (Kd) of 10 mM or less to the asialoglycoprotein receptor (ASGP-R) specifically expressed to the hepatocyte surface.

**16.** The method according to any of claims 7 - 15 wherein the labeling is radioisotope labeling or fluorescent labeling.

**17.** The method according to any of claims 1 - 16 wherein the ligand is a sugar or derivatives thereof.

**18.** The method according to any of claims 1 - 13 wherein the substance modified with the ligand is the physiologically active protein.

**19.** The method according to claim 18 wherein the physiologically active protein is interferon $\alpha$ or interferon $\beta$.

**20.** A device comprising being capable of performing the method according to any of claims 1 - 19.

**21.** A reagent kit for performing the method according to any of claims 1 - 19.

**22.** A ligand appropriate to the targeting DDS preparation screened by the method according to any of claims 1 - 19.

**23.** The ligand according to claim 22 wherein the ligand is a compound represented by the following formula (I):

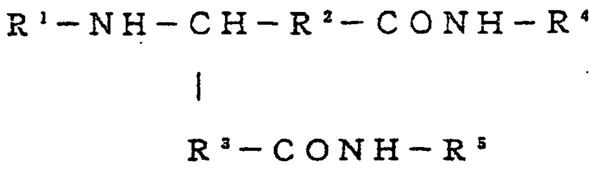

(I)

wherein $R^1$ is hydrogen atom or an organic residue having functional group which can bind with the physiologically active substance,
$R^2$ and $R^3$ are each independently single bond or straight or branched alkylene of $C_{1-5}$,
$R^4$ is a group represented by the following formula (II)

$$-R^6-(O-R^6-)n-Sug \quad (II)$$

wherein $R^6$ is each independently straight or branched alkylene of $C_{2-5}$, Sug is sugar group, and n is an integer of 1-5, and $R^5$ is $R^4$ hereinbefore, or a group represented by the following formula (III)

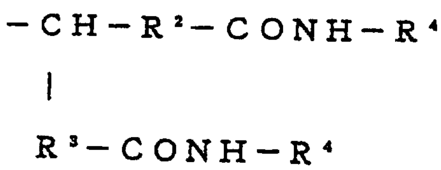

(III)

wherein $R^2$, $R^3$ and $R^4$ are same as above.

**24.** The ligand according to claim 23 wherein $R^1$- is

$$H_2N-R^a-CO-$$

wherein $R^a$ represents straight or branched alkylene of $C_{1-15}$.

**25.** The ligand according to claim 23 wherein a sugar group in Sug group is galactose.

**26.** The ligand according to any of claims 23 - 25 wherein $R^5$ in the formula (I) is $R^4$.

**27.** A compound represented by the following formula (IV):

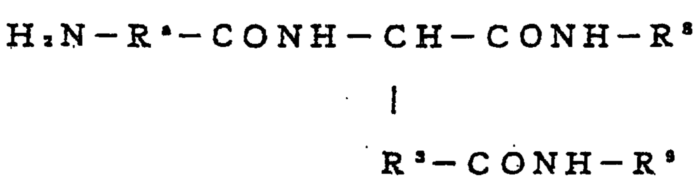

(IV)

wherein $R^a$ is a straight or a branched alkylene of $C_{1-15}$,
$R^3$ is a straight or a branched alkylene of $C_{1-5}$,
$R^8$ is a group represented by the following formula (V):

$$-R^{10}-(O-R^{10}-)n-Sug \quad (V)$$

wherein $R^{10}$ is each independently represented by a straight or a branched alkylene of $C_{2-5}$,
Sug is a sugar group or derivatives thereof, and
n is an integer of 1-5, and
$R^9$ is $R^8$ hereinbefore, or a group represented by the following formula (VI)

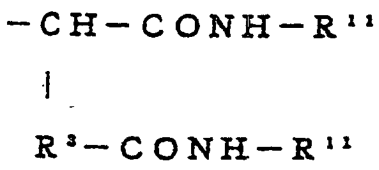

(VI)

wherein $R^3$ is a straight or a branched alkylene of $C_{1-5}$,
$R^{11}$ is represented by the following formula (VII):

$$-R^{10}-(O-R^{10}-)m-Sug \qquad (VIII)$$

wherein $R^{10}$ is each independently represented by a straight or a branched alkylene of $C_{2-5}$,
Sug is a sugar group or derivatives thereof, and
m is an integer of 1-5. Proviso that m is the integer different from n in the above formula (V).

**28.** The compound according to claim 27 wherein the sugar group in Sug or derivatives thereof is galactose.

**29.** A pharmaceutical composition comprising the physiologically active substance having the ligand appropriate to the targeting DDS preparation screened by the method according to any of claims 1 - 19 and a pharmaceutically acceptable carrier.

**30.** The pharmaceutical composition according to claim 29 wherein the physiologically active substance is the physiologically active protein.

**31.** The pharmaceutical composition according to claim 29 or claim 30 wherein the ligand is the ligand maintaining the binding affinity to asialoglycoprotein receptor (ASGP-R), which is specifically expressed in the hepatocyte surface, and the liver recognizing ligand having retainability in the liver extracellular fluid due to having a ratio ($k_{off}/k_{int}$) of the dissociation rate constant ($k_{off}$) and the internalization rate constant ($k_{int}$) to the liver cells being 1 or more.

**32.** The pharmaceutical composition according to claim 31 wherein the ligand is the liver recognizing ligand having the kinetic property, which is an index for intensity of the binding affinity to asialoglycoprotein receptor (ASGP-R) expressed specifically on the hepatocyte surface, consisting of the dissociation constant Kd being 10 mM or less, the internalization rate constant ($k_{int}$) being smaller than 0.1 min-1 and the ratio of $k_{off}/k_{int}$ being larger than 1.

**33.** The pharmaceutical composition according to any of claims 29 - 32 wherein the ligand is a branched galactose (Gal), N-acetylgalactosamine (GalNAc) or derivatives thereof.

Fig. 1

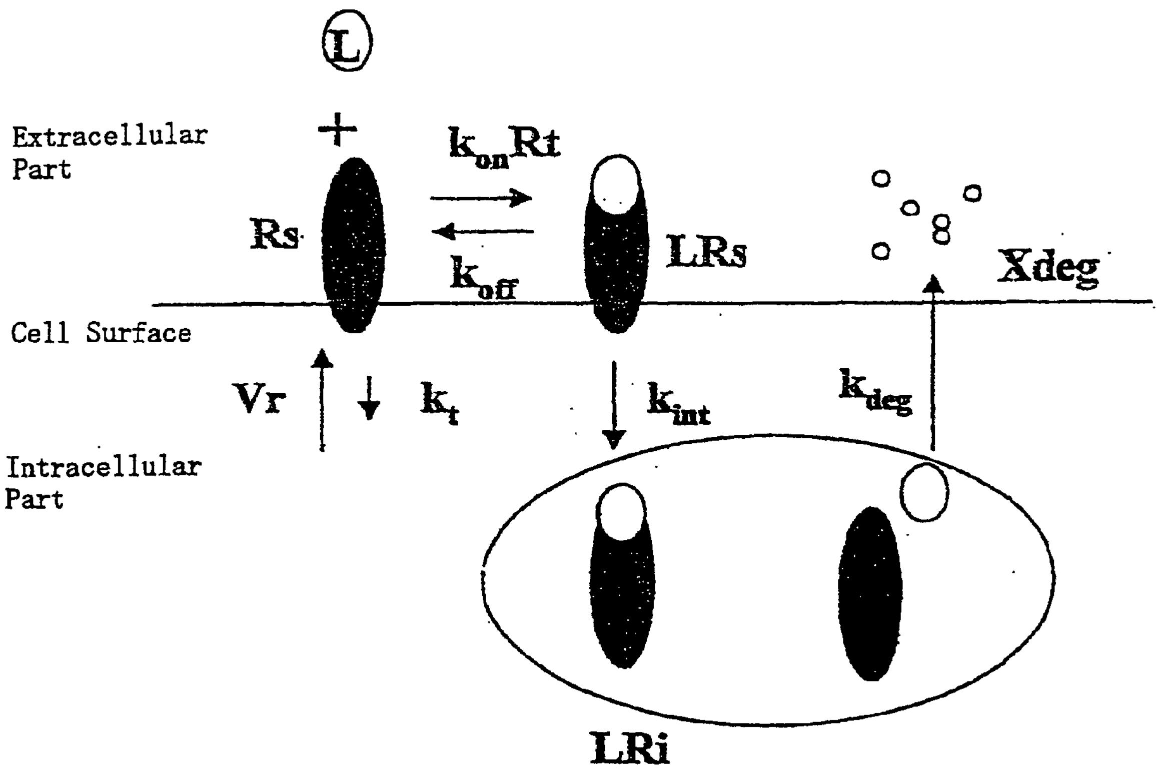
L
+
Extracellular
Part
Rs
$k_{on}Rt$
$k_{off}$
LRs
Xdeg
Cell Surface
Vr
$k_t$
$k_{int}$
$k_{deg}$
Intracellular
Part
LRi

Fig. 2

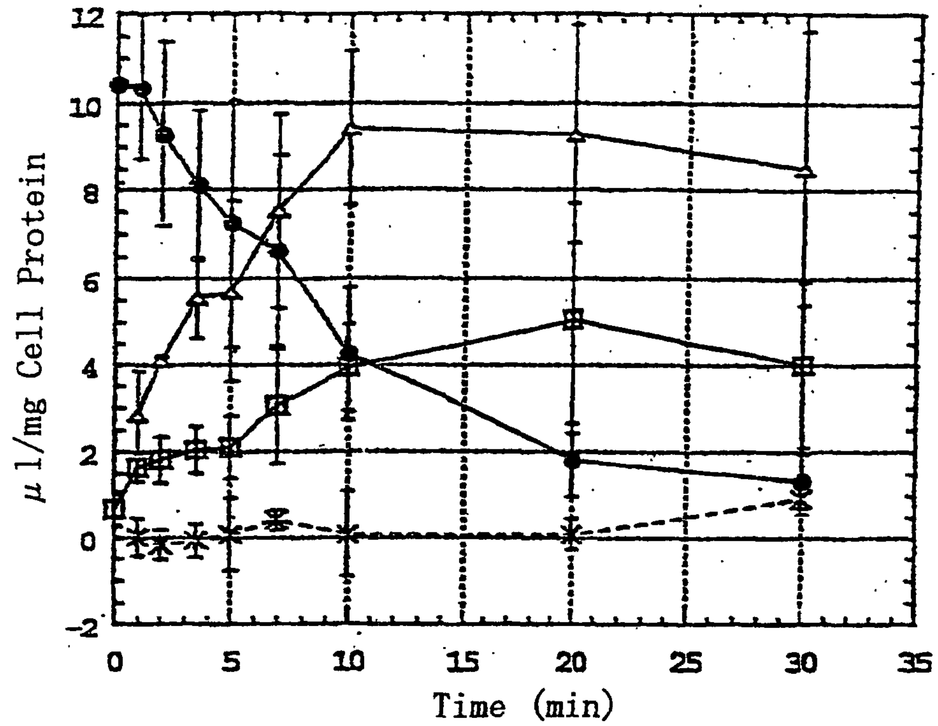
μ l/mg Cell Protein
12
10
8
6
4
2
0
-2
0
5
10
15
20
25
30
35
Time (min)

Fig. 3

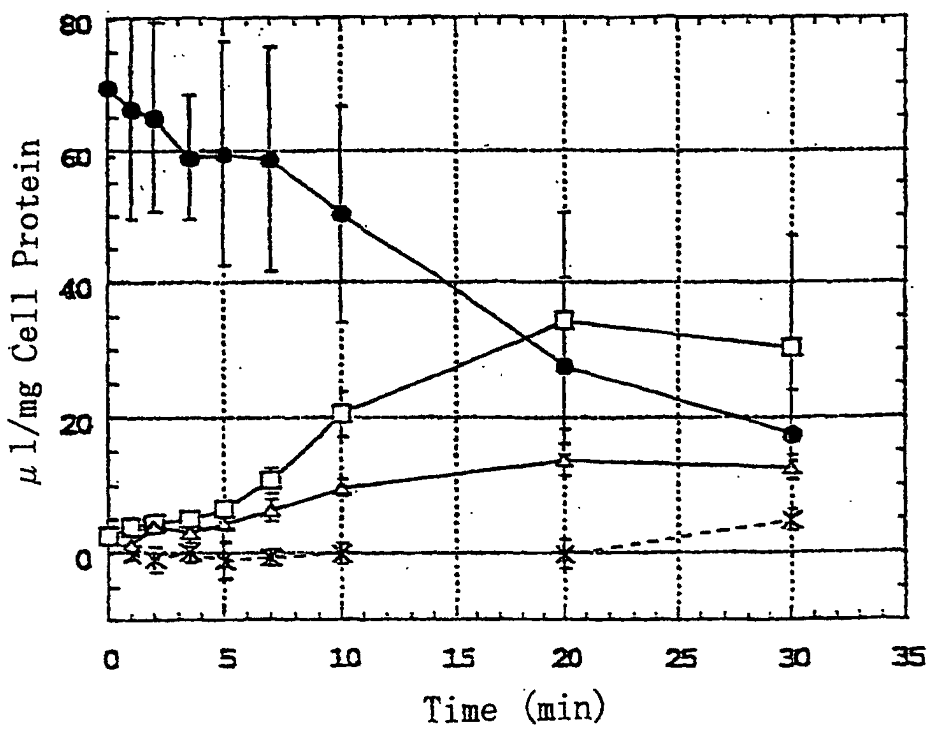
μ l/mg Cell Protein
80
60
40
20
0
0
5
10
15
20
25
30
35
Time (min)

Fig. 4

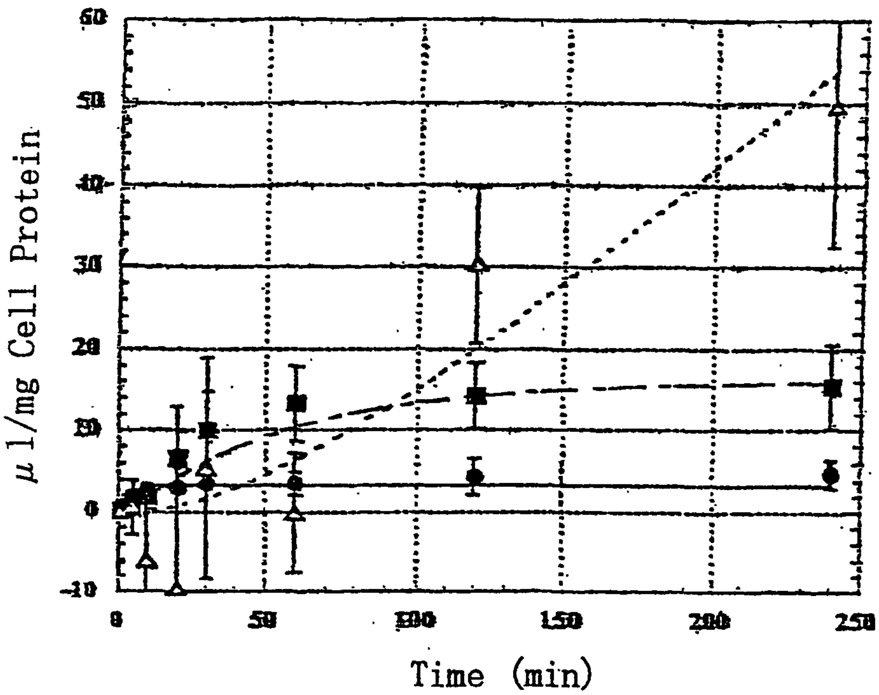
60
50
40
30
20
10
0
-10
μ l/mg Cell Protein
0
50
100
150
200
250
Time (min)

Fig. 5

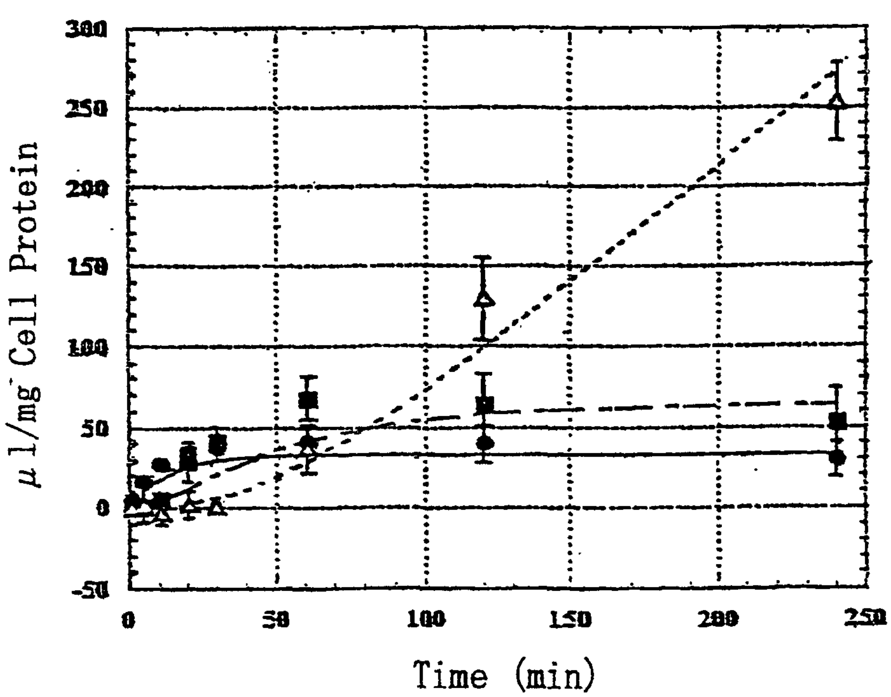
300
250
200
150
100
50
0
-50
μ l/mg Cell Protein
0
50
100
150
200
250
Time (min)

Fig. 6

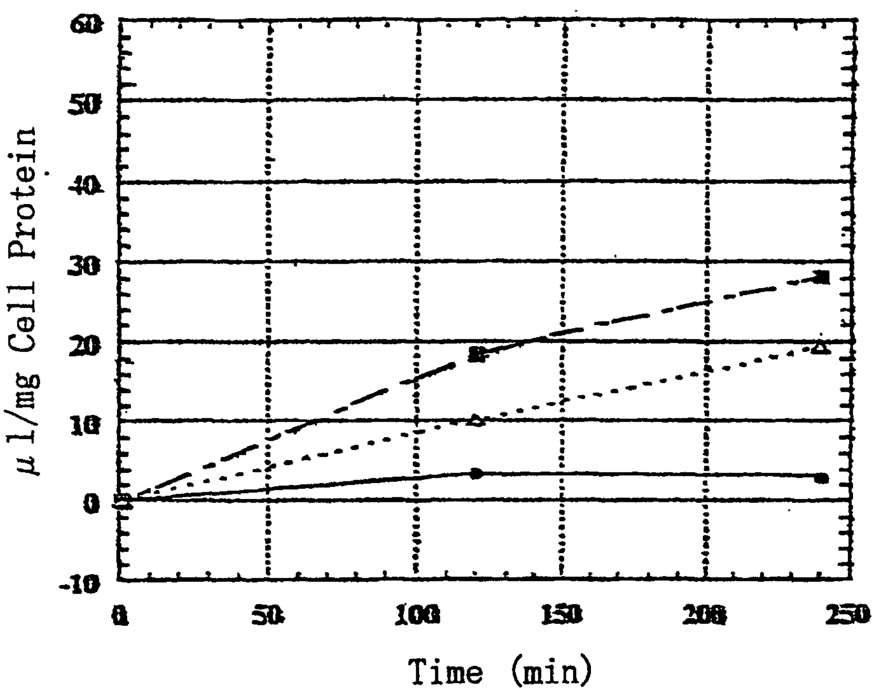
60
50
40
30
20
10
0
-10
0
50
100
150
200
250
μ l/mg Cell Protein
Time (min)

Fig. 7

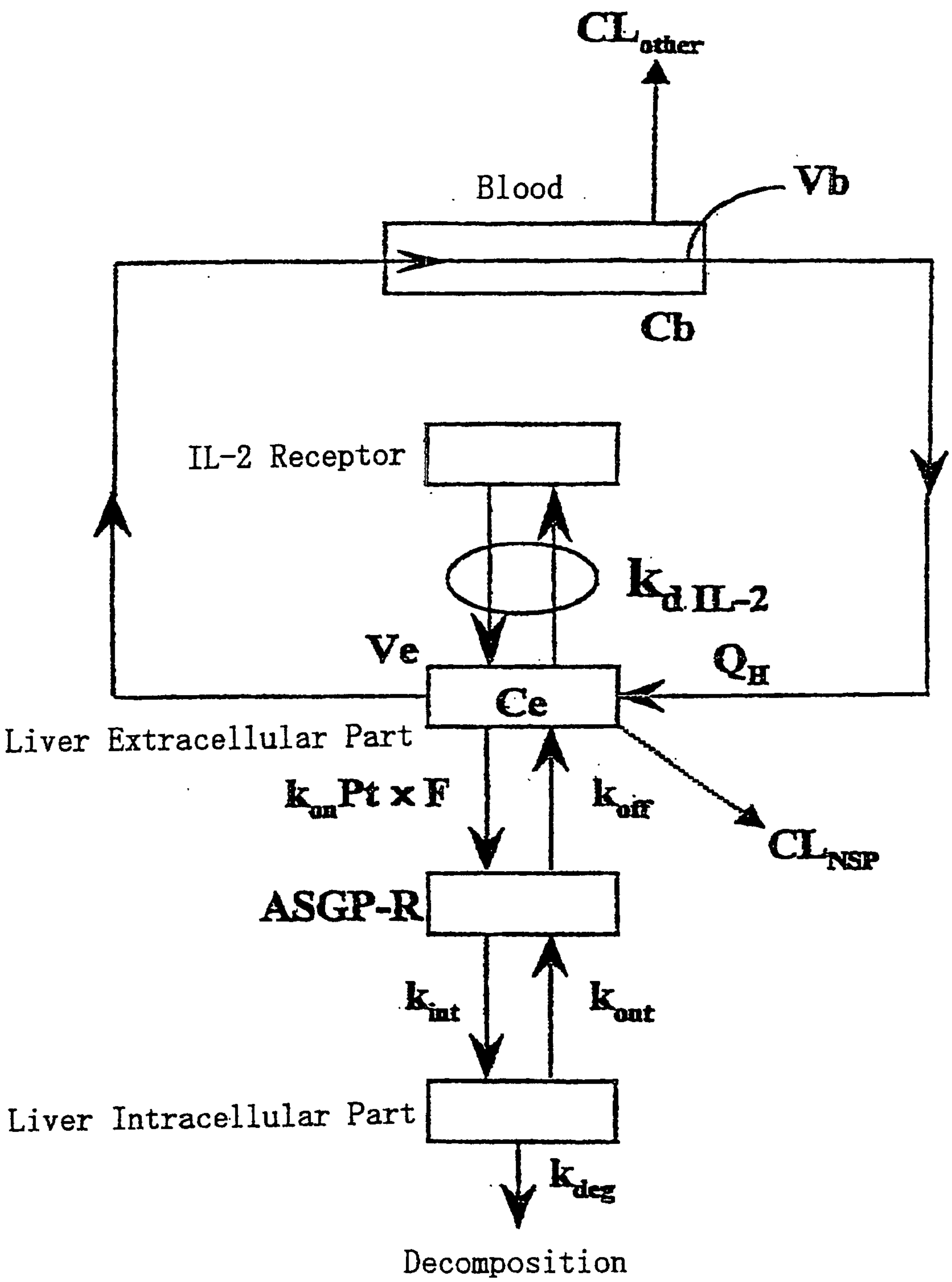
CL other
Blood
Vb
Cb
IL-2 Receptor
kd IL-2
Ve
QH
Ce
Liver Extracellular Part
kon Pt x F
koff
CLNSP
ASGP-R
kint
kout
Liver Intracellular Part
kdeg
Decomposition

Fig. 8

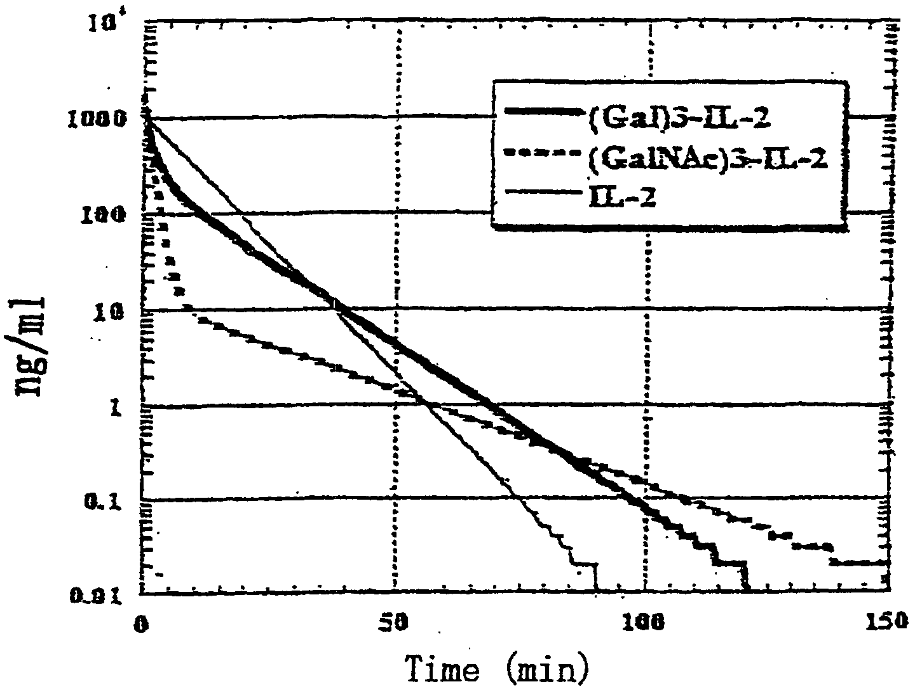
10⁴
1000
100
10
1
0.1
0.01
ng/ml
0
50
100
150
Time (min)
(Gal)3-IL-2
(GalNAc)3-IL-2
IL-2

Fig. 9

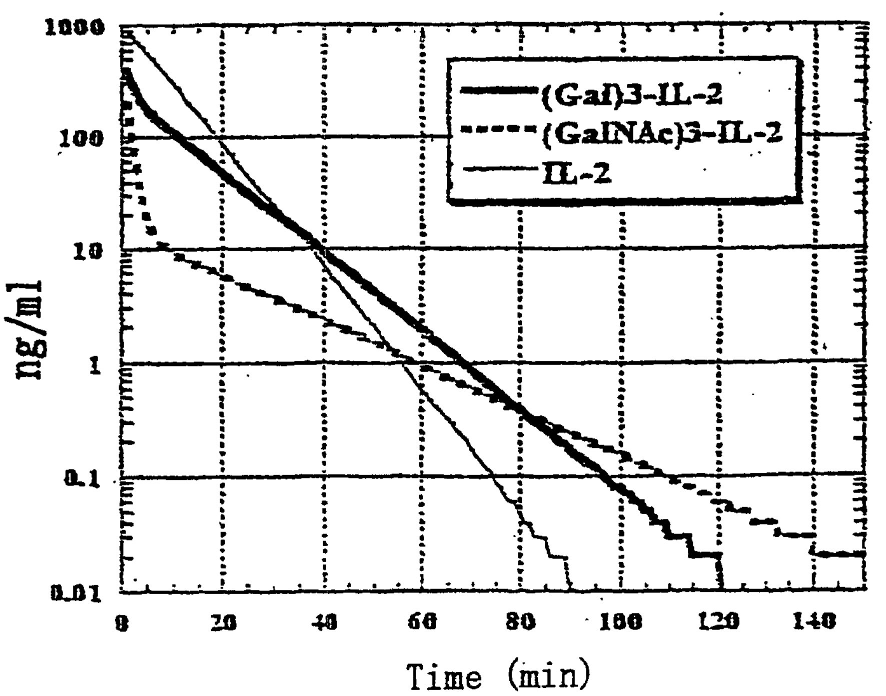
1000
100
10
1
0.1
0.01
ng/ml
0
20
40
60
80
100
120
140
Time (min)
(Gal)3-IL-2
(GalNAc)3-IL-2
IL-2

Fig. 10

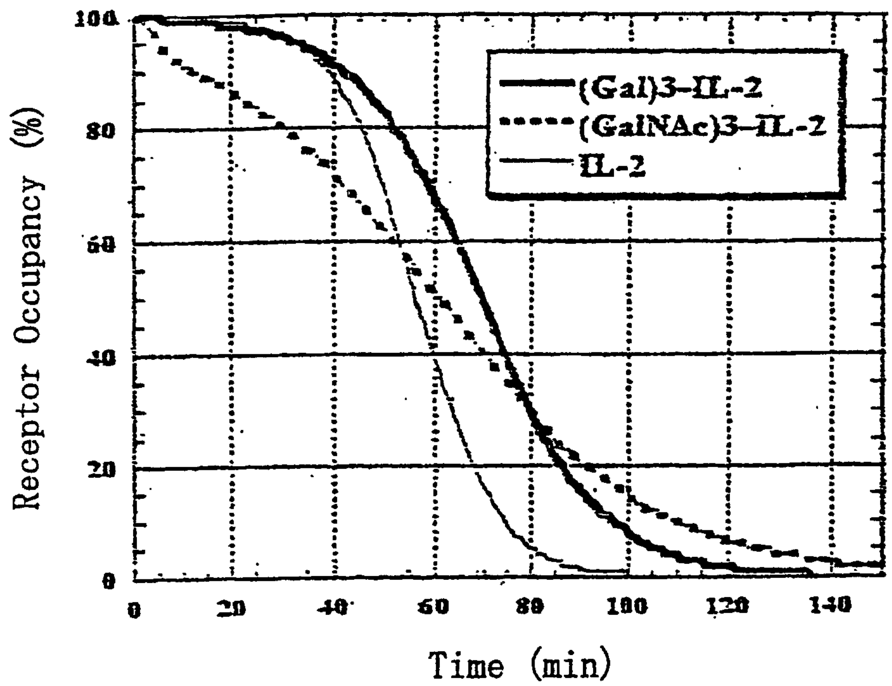
(Gal)3-IL-2
(GalNAc)3-IL-2
IL-2
Receptor Occupancy (%)
100
80
60
40
20
0
0
20
40
60
80
100
120
140
Time (min)

Fig. 11

(A)

(B)

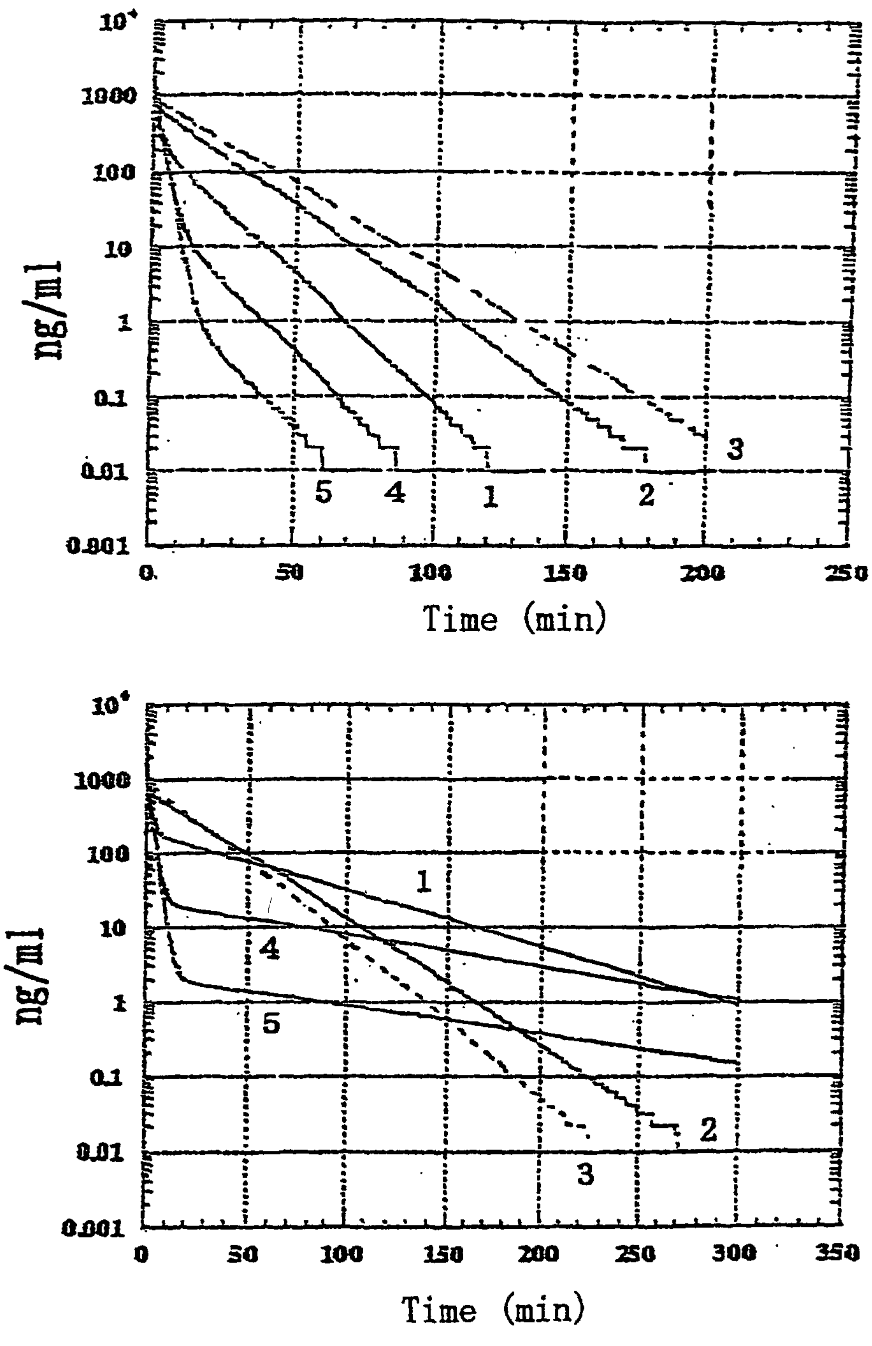

10⁴
1000
100
10
1
0.1
0.01
0.001
ng/ml
0
50
100
150
200
250
Time (min)
5
4
1
2
3
10⁴
1000
100
10
1
0.1
0.01
0.001
ng/ml
0
50
100
150
200
250
300
350
Time (min)
1
4
5
2
3

(C)

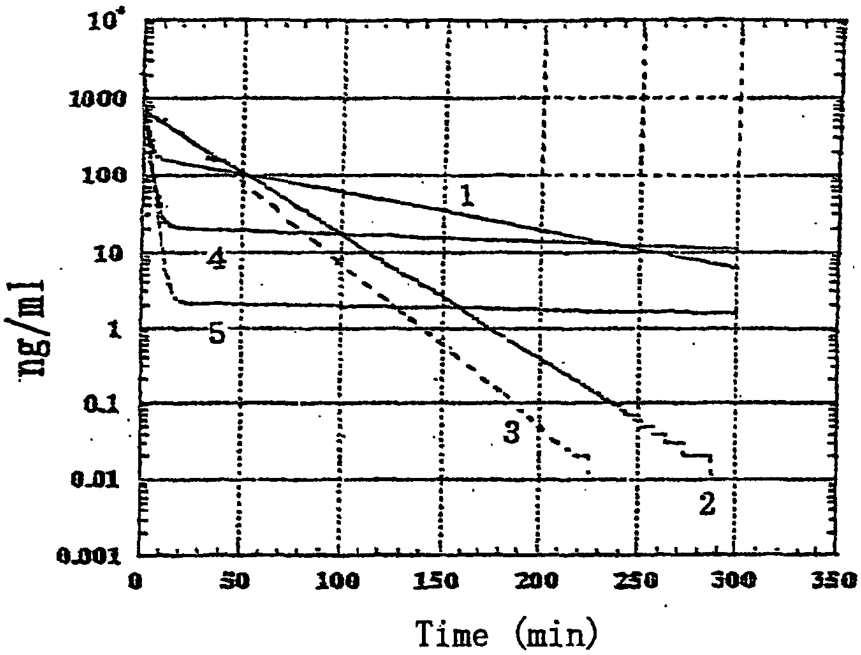
10⁴
1000
100
10
1
0.1
0.01
0.001
ng/ml
0
50
100
150
200
250
300
350
Time (min)
1
2
3
4
5

Fig. 12

(A)

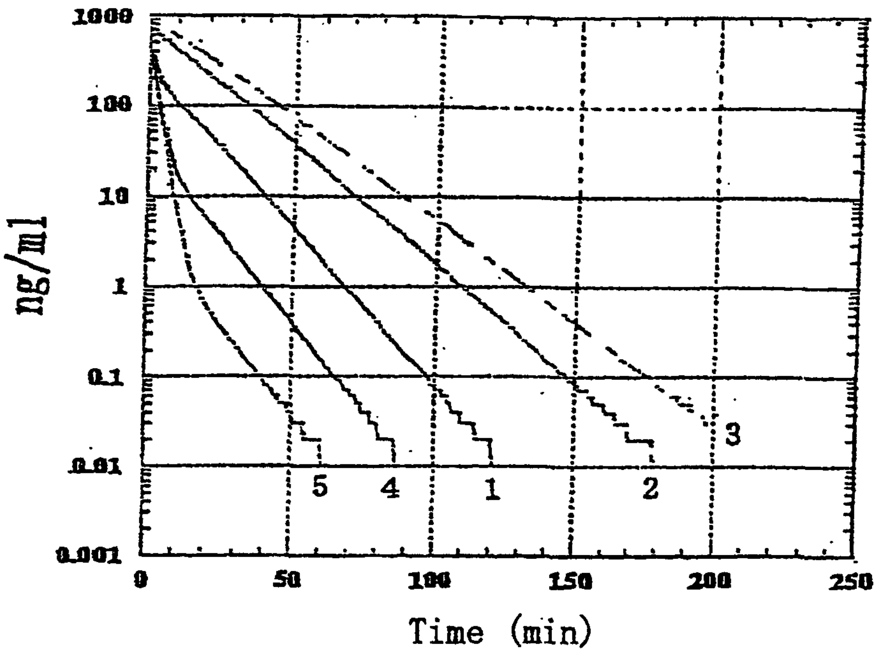
1000
100
10
1
0.1
0.01
0.001
ng/ml
0
50
100
150
200
250
Time (min)
5
4
1
2
3

(B)

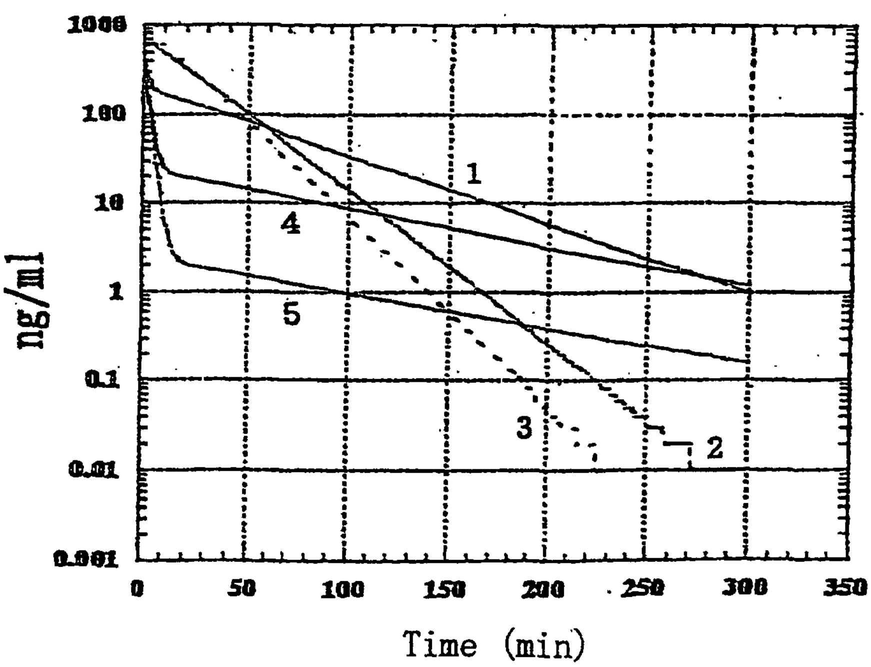
1000
100
10
1
0.1
0.01
0.001
ng/ml
0
50
100
150
200
250
300
350
Time (min)
1
4
5
3
2

(C)

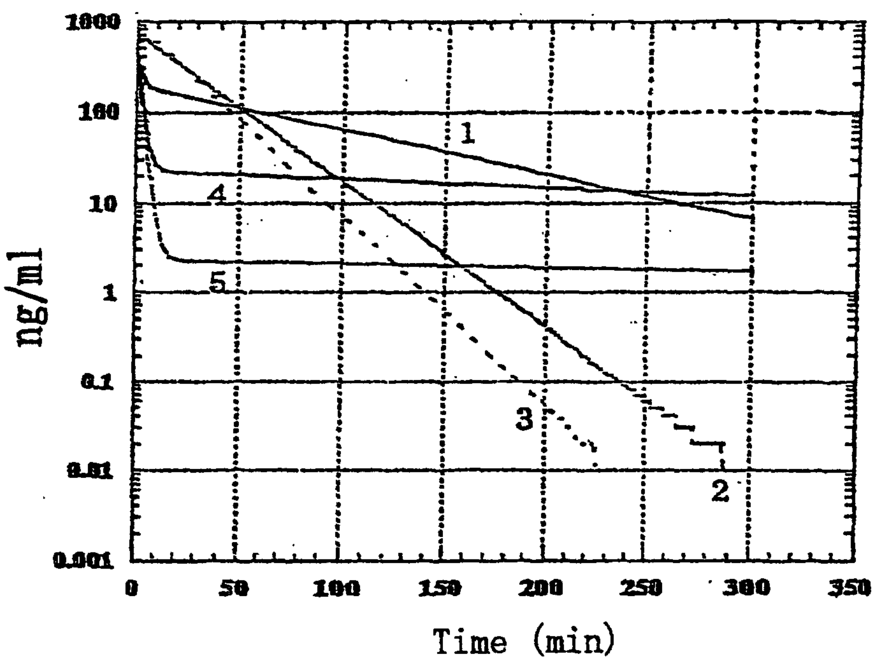
1000
100
10
1
0.1
0.01
0.001
ng/ml
0
50
100
150
200
250
300
350
Time (min)
1
2
3
4
5

Fig. 13

(A)

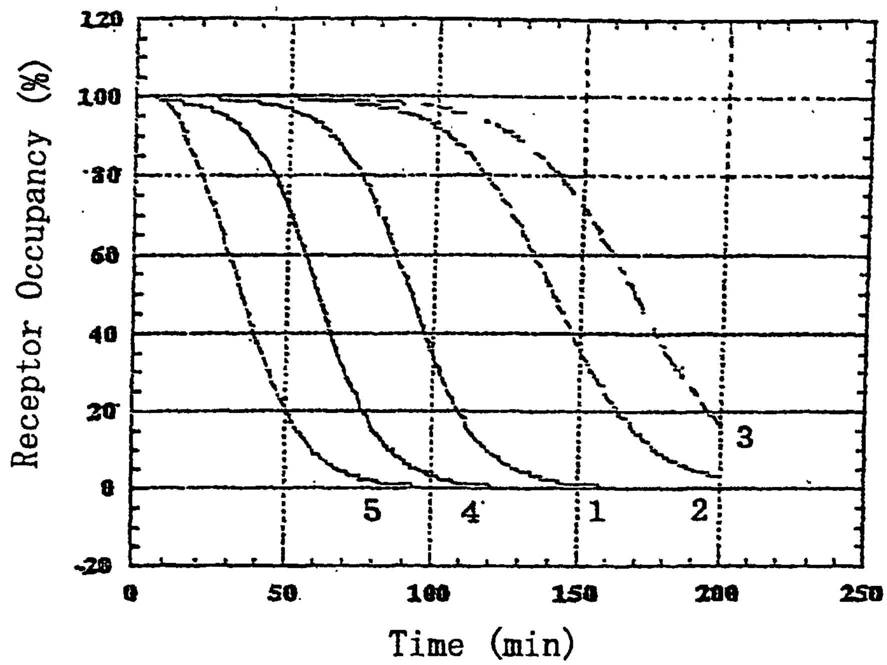
120
100
80
60
40
20
0
-20
Receptor Occupancy (%)
0
50
100
150
200
250
Time (min)
5
4
1
2
3

(B)

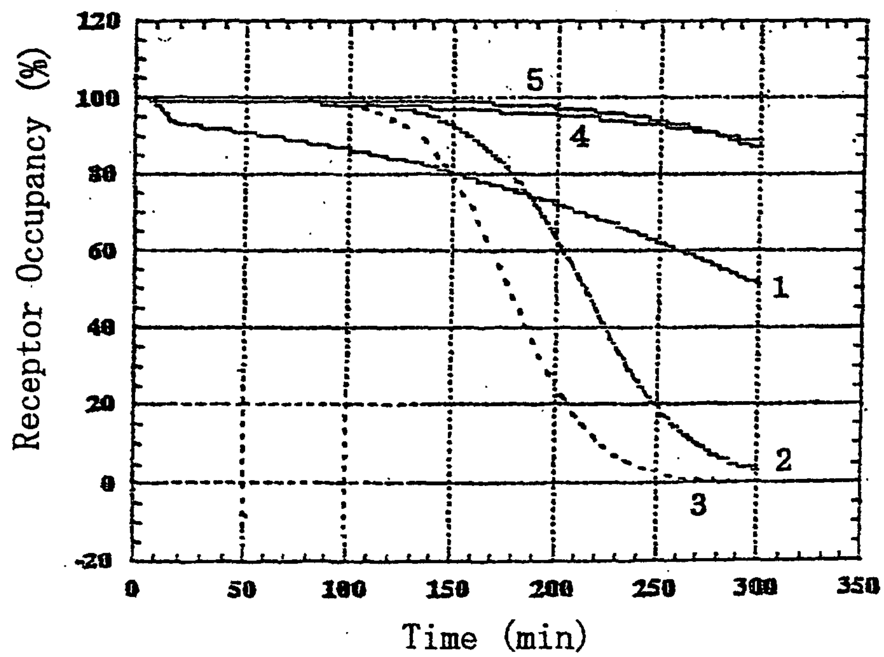
120
100
80
60
40
20
0
-20
Receptor Occupancy (%)
0
50
100
150
200
250
300
350
Time (min)
5
4
1
2
3

(C)

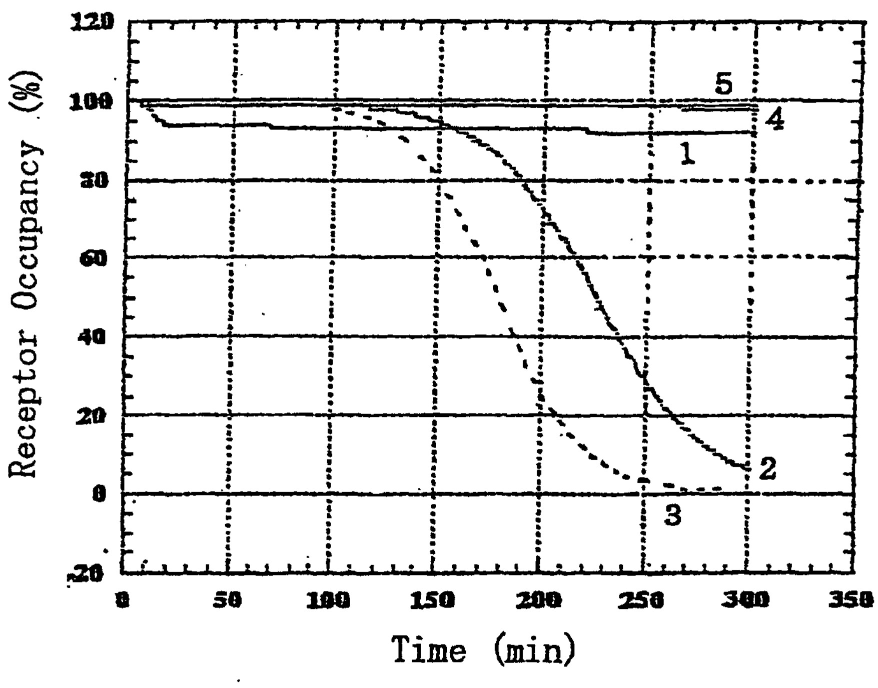
120
100
80
60
40
20
0
-20
0
50
100
150
200
250
300
350
Receptor Occupancy (%)
Time (min)
1
2
3
4
5

Fig. 14

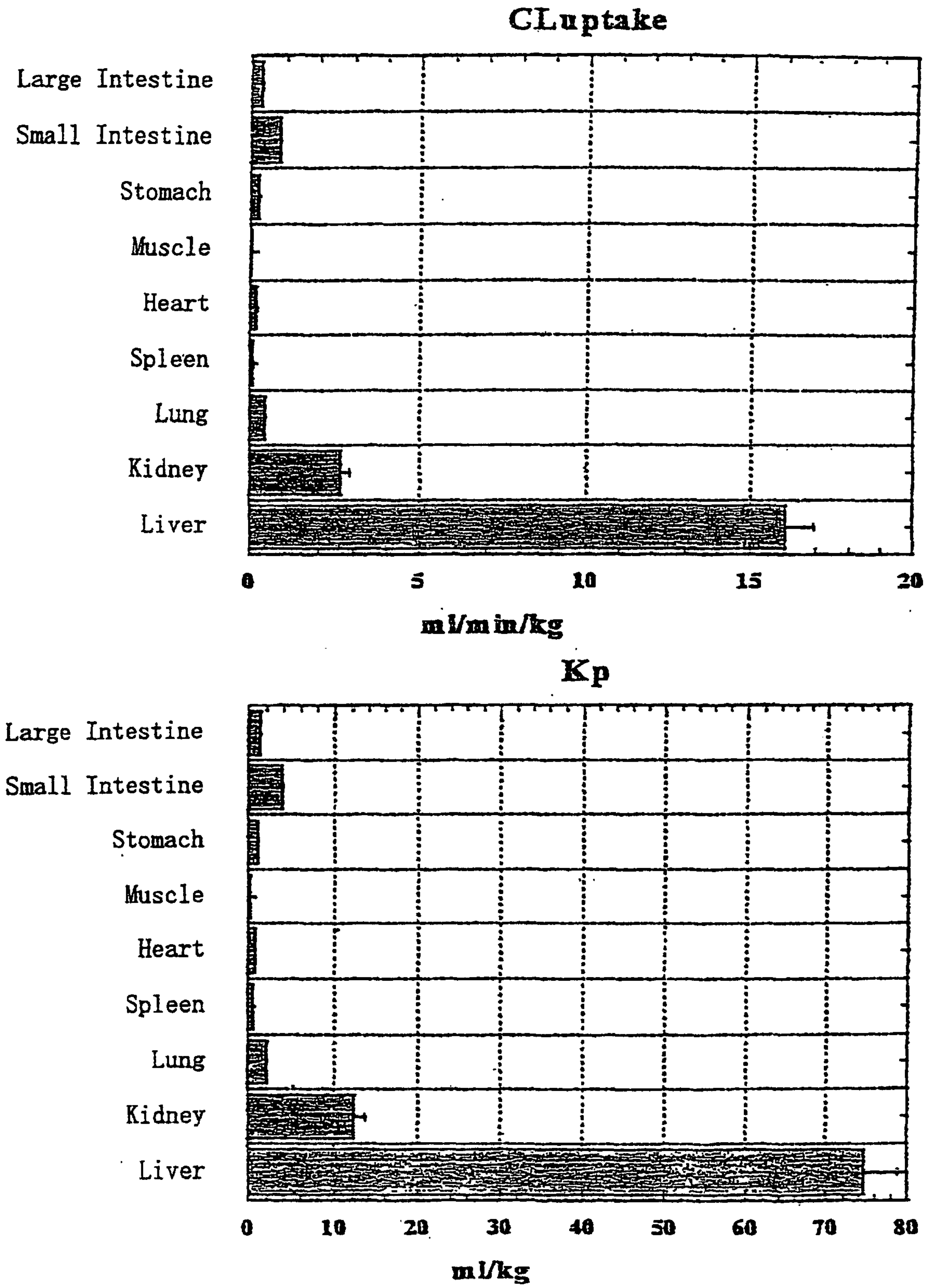
CLuptake
Large Intestine
Small Intestine
Stomach
Muscle
Heart
Spleen
Lung
Kidney
Liver
0
5
10
15
20
ml/min/kg
Kp
Large Intestine
Small Intestine
Stomach
Muscle
Heart
Spleen
Lung
Kidney
Liver
0
10
20
30
40
50
60
70
80
ml/kg

Fig. 15

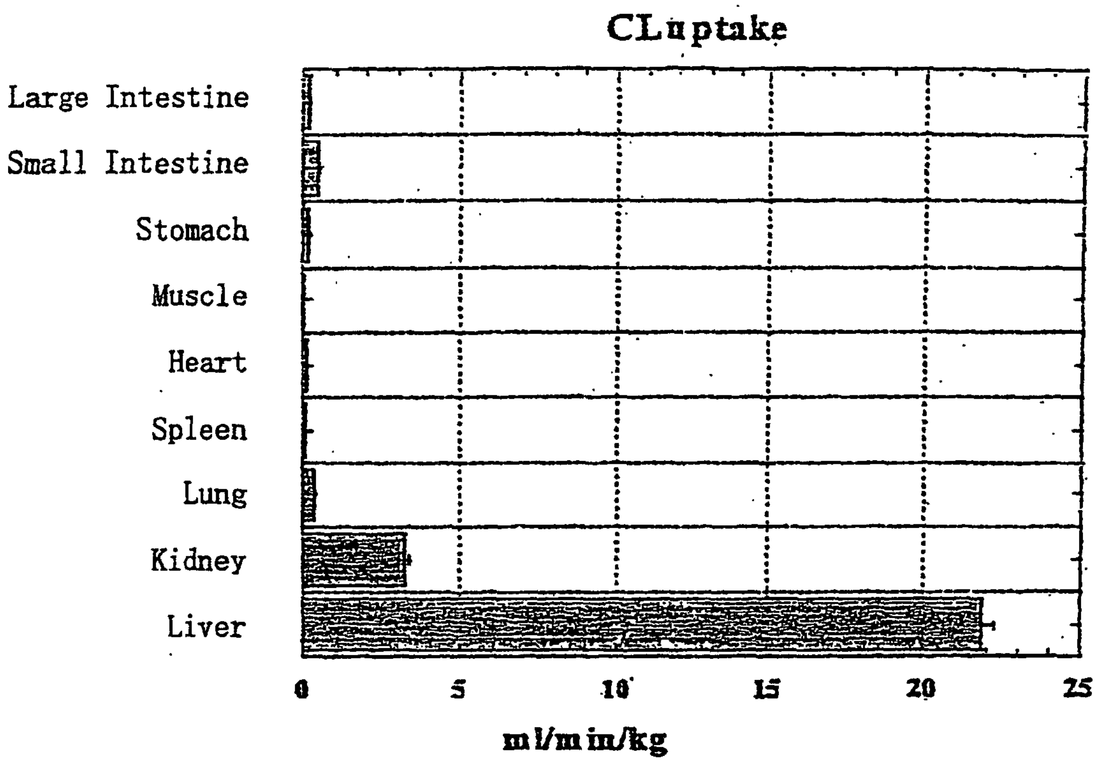
CLuptake
Large Intestine
Small Intestine
Stomach
Muscle
Heart
Spleen
Lung
Kidney
Liver
0
5
10
15
20
25
ml/min/kg

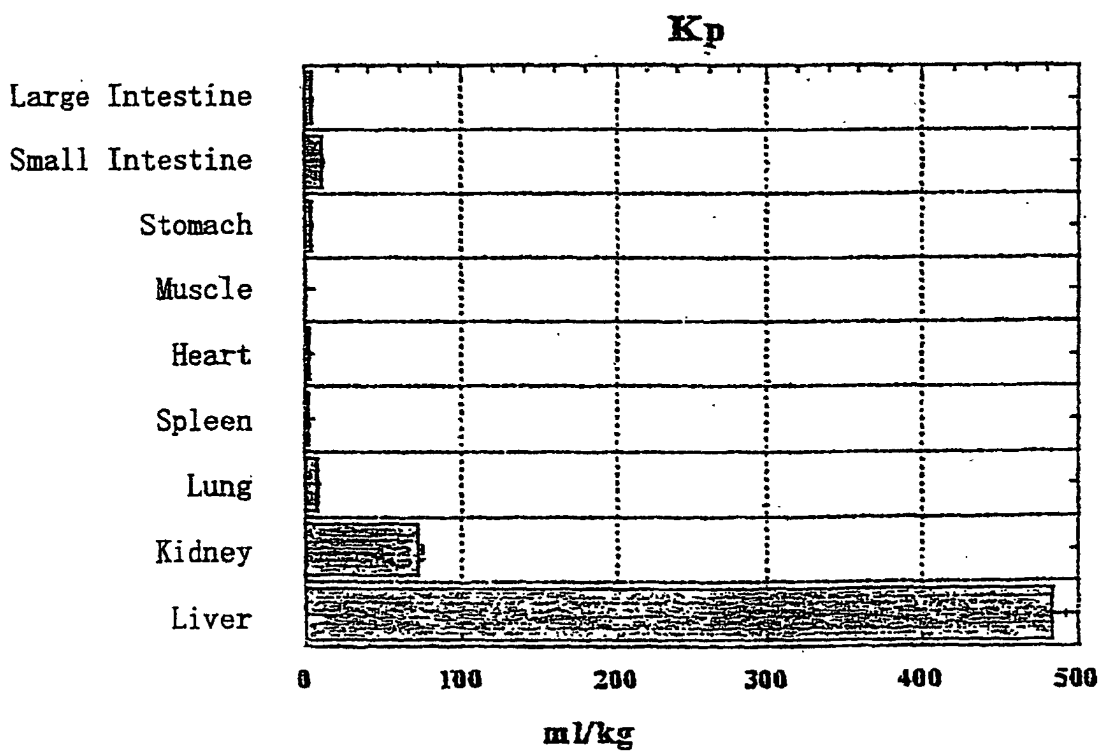
Kp
Large Intestine
Small Intestine
Stomach
Muscle
Heart
Spleen
Lung
Kidney
Liver
0
100
200
300
400
500
ml/kg

## INTERNATIONAL SEARCH REPORT

International application No.
PCT/JP00/04362

A. CLASSIFICATION OF SUBJECT MATTER
Int.Cl$^7$ G01N33/15, G01N33/566, G01N33/50, A61K47/48, A61K38/20, A61K38/21, A61K31/7028, C07H15/04

According to International Patent Classification (IPC) or to both national classification and IPC

B. FIELDS SEARCHED

Minimum documentation searched (classification system followed by classification symbols)
Int.Cl$^7$ G01N33/15, G01N33/566, G01N33/50, A61K47/48, A61K38/20, A61K38/21, A61K31/7028, C07H15/04

Documentation searched other than minimum documentation to the extent that such documents are included in the fields searched
Jitsuyo Shinan Koho 1922-1996 Toroku Jitsuyo Shinan Koho 1994-2000
Kokai Jitsuyo Shinan Koho 1971-2000 Jitsuyo Shinan Toroku Koho 1996-2000

Electronic data base consulted during the international search (name of data base and, where practicable, search terms used)
CA(STN), REGSTRY

C. DOCUMENTS CONSIDERED TO BE RELEVANT

| Category* | Citation of document, with indication, where appropriate, of the relevant passages | Relevant to claim No. |
|---|---|---|
| A | Matsuda, M. et al., Interaction between the amino-terminal SH3 domain of CRK and its natural target proteins. Journal of Biological Chemistry, 1996, Vol. 271, No. 24, pp.14468-14472 | 1-33 |
| A | Bider, M. et al., High-affinity ligand binding to subunitH1 of the asialoglycoprotein receptor in the absence of subunit H2. European Journal of Biochemistry, 1995, Vol.230, No.1, pp.207-212 | 1-33 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 August, 2000 (16.08.00) | 29 August, 2000 (29.08.00) |
| Name and mailing address of the ISA/ Japanese Patent Office | Authorized officer |
| Facsimile No. | Telephone No. |

Form PCT/ISA/210 (second sheet) (July 1992)